# EUROPÄISCHE PATENTANMELDUNG

(11) **EP 4 491 699 A1**
(43) Veröffentlichungstag der Anmeldung: **15.01.2025**
(21) Anmeldenummer: 23185184.1
(22) Anmeldetag: 13.07.2023
(51) Int. Cl.: C10M 105/70, C10N 20/00, C10N 20/02, C10N 30/02, C10N 30/10, C10N 30/00, C10N 10/04

(54) **PHTHALIMIDVERBINDUNGEN ALS BASISÖL FÜR SCHMIERSTOFFZUSAMMENSETZUNGEN**

(71) Anmelder: Klueber Lubrication München GmbH & Co. KG, 81379 München (DE)
(72) Erfinder: Schmidt-Amelunxen, Martin, 85244 Röhrmoos/Arzbach (DE); Bieser, Arno, 69469 Weinheim (DE); Kilthau, Thomas, 82538 Geretsried (DE); Ma, Ling, 81825 München (DE); Schweigkofler, Martin, 86316 Friedberg (DE); Grundei, Stefan, 86415 Mering (DE); Hertz, Georg, 87616 Marktoberdorf (DE); Maier, Gerhard, 80807 München (DE)
(74) Vertreter: Kuhn, Daniela

(57) **Zusammenfassung**

Die vorliegende Erfindung betrifft eine Schmierstoffzusammensetzung, die wenigstens eine Phthalimidverbindung als Basisöl enthält sowie die Verwendung dieser Phthalimidverbindungen, insbesondere als Komponente einer Schmierstoffzusammensetzung zum teilweisen oder vollständigen Ersatz einer fluorhaltigen Schmierstoffkomponente.

## Beschreibung

Die vorliegende Erfindung betrifft eine Schmierstoffzusammensetzung, die wenigstens eine Phthalimidverbindung als Basisöl enthält sowie die Verwendung dieser Phthalimidverbindungen.

### HINTERGRUND DER ERFINDUNG

Schmierstoffe sind wesentliche Bestandteile vieler industrieller Prozesse, bei denen sich zwei oder mehr Oberflächen in engem Kontakt bewegen. Das Anwendungsspektrum für Schmieröle ist sehr breit und umfasst unter anderem Kfz-Schmiermittel, Schmiermittel für Zweitakt- und Viertakt-Benzinmotoren, Schmiermittel für Dieselmotoren, Gasmotorenöle, Gasturbinenöle, Automatikgetriebeflüssigkeiten, Getriebeöle usw.

Schmierstoffe können als Schmieröle und Schmierfette ausgebildet sein. Industrielle Schmieröle umfassen unter anderem industrielle Getriebeöle, pneumatische Werkzeugschmierungsmittel, Hochtemperaturöle, Luft- und Gaskompressoröle für alle Arten von Kompressoren, Werkzeugmaschinenöle, Textilöle, Dampfturbinenöle, Hydraulikflüssigkeiten, Papiermaschinenöle, Lebensmittelmaschinenöle, Dampfzylinderöle, Metallbearbeitungsflüssigkeiten zum Metallschneiden, Metallwalzen, Metallziehen, Metallschmieden und Metallprägen. Schmierfette umfassen zusätzlich zum Schmieröl noch einen oder mehrere Verdicker.

Spezielle Anforderungen werden an Schmierstoffzusammensetzung für den Einsatz bei hohen Temperaturen (Hochtemperaturschmierstoffe) gestellt. Herkömmliche Schmierstoffe sind für den Einsatz bei hohen Temperaturen, speziell im Dauerbetrieb, nicht geeignet, da sie beispielsweise über Oxidations- und/oder thermische Zersetzungsreaktionen und (De)polymerisation zerstört werden und ihre schmierenden Eigenschaften verlieren. Geeignete Basisöle mit hoher Temperaturbeständigkeit sind bekannt und umfassen z.B. alkylierte Aromaten, Alkylester von Carbonsäuren oder Säuren des Phosphors, höhermolekulare Esterverbindungen, wie Estolide, Polyphenylether, Siliconöle, Fluorkohlenwasserstoffe, etc.

Die WO 2016/096074 beschreibt Hochtemperaturschmierstoffe, die ein Basisöl enthalten, das ausgewählt ist unter Alkylaromaten, Estoliden und Trimellitsäureestern.

Die US 2015/0166447 A1 beschreibt alkylierte Diphenylether und deren Verwendung als Basisöl für verschiedene Schmiermittel, wie Lageröle, Getriebeöle, Motoröle, Gasturbinenöle, Automatikgetriebeöle, Vakuumpumpenöle, sowie für Fette.

Die US 2014/0274836 A1 beschreibt multiaromatische Basisöle, die aus über Linker verknüpfte Naphthalingruppen bestehen. Als Linker werden unter Anderem Alkylengruppen sowie Linker mit Ether-, Acyl-, Ester-, Anhydrid-, Amino-, Amido-, Urethan-, Schwefelgruppen und Kombinationen davon genannt. In den Ausführungsbeispielen werden jedoch nur über Alkylengruppen verknüpfte Naphthaline eingesetzt.

Derzeit werden in Schmierstoffen für Hochtemperaturanwendungen vielfach noch fluorhaltige Verbindungen, wie voll- oder teilfluorierte Alkylverbindungen, eingesetzt. So verfügen speziell Perfluorpolyether (PFPE) über gute Schmierstoffeigenschaften in einem sehr weiten Temperaturbereiche von etwa -70°C bis über 300°C. Aufgrund ihrer langkettigen Polymerstruktur sind PFPEs zudem hochgradig inert und eignen sich auch für Einsatzbereiche, die den Kontakt mit reaktiven Chemikalien erfordern. Die fluorhaltigen Schmierstoffe besitzen zudem in der Regel eine gute Kompatibilität mit einer Vielzahl von Polymermaterialien, sind nicht entflammbar und weisen eine geringe Toxizität auf. Der Einsatz von fluorierten Alkylverbindungen und Fluorpolymeren wird aufgrund ihrer geringen Abbaubarkeit und der resultierenden Umweltfolgen inzwischen kritisch gesehen und EU-weit wird eine Beschränkung von per- und polyfluorierten Alkylverbindungen (PFAS) angestrebt. Es besteht daher ein Bedarf, den Anteil an diesen Verbindungen auch im Bereich der Schmierstoffe zu reduzieren oder zukünftig ganz auf diese zu verzichten.

Der Erfindung liegt die Aufgabe zugrunde, ein Basisöl für eine Schmierstoffzusammensetzung bereitzustellen, das sich als Ersatzstoff für den zumindest teilweisen Ersatz von voll- oder teilfluorierten Schmierstoffen und speziell voll- oder teilfluorierten Alkylverbindungen, wie die Perfluorpolyetheröle, eignet. Trotz des (teilweisen) Verzichts auf fluorierte Komponenten soll die resultierende Schmierstoffzusammensetzung über gute anwendungstechnische Eigenschaften, wie eine hohe Temperaturstabilität, gute rheologische Eigenschaften und eine gute Oxidationsstabilität verfügen.

Überraschenderweise wurde nun gefunden, dass diese Aufgabe durch den Einsatz spezieller Phthalimidverbindungen als Basisöl für Schmierstoffzusammensetzungen gelöst wird.

### ZUSAMMENFASSUNG DER ERFINDUNG

Ein erster Gegenstand der Erfindung ist eine Schmierstoffzusammensetzung, enthaltend
a) wenigstens eine Phthalimidverbindung der allgemeinen Formel (I) 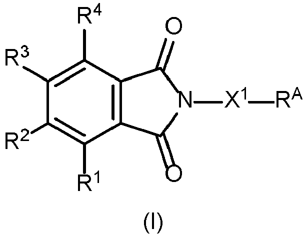 worin
   - R¹, R², R³ und R⁴: unabhängig voneinander ausgewählt sind unter Wasserstoff, geradkettigem oder verzweigtem Alkyl, geradkettigem oder verzweigtem Alkoxy, geradkettigem oder verzweigtem Alkanoyl, geradkettigem oder verzweigtem Alkyloxycarbonyl,
   unsubstituiertem oder substituiertem Aryl, unsubstituiertem oder substituiertem Arylalkylen, unsubstituiertem oder substituiertem Aryloxy, unsubstituiertem oder substituiertem Arylalkylenoxy, unsubstituiertem oder substituiertem Arylthio, unsubstituiertem oder substituiertem Arylalkylenthio, unsubstituiertem oder substituiertem Aroyl, unsubstituiertem oder substituiertem Aryloxycarbonyl, unsubstituiertem oder substituiertem Arylcarbonyloxy,
   Monoalkylaminocarbonyl, Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Dialkylaminocarbonyl, Diarylaminocarbonyl, Di(arylalkylen)aminocarbonyl, Monoalkanoylamino, Monoaroylamino, Monoalkanoylmonoaroylamino, Dialkanoylamino, Diaroylamino, wobei die Arylreste von Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Diarylaminocarbonyl, Di(arylalkylen)aminocarbonyl, Monoaroylamino, Monoalkanoylmonoaroylamino und Diaroylamino jeweils unsubstituiert oder substituiert sind,
   unsubstituierten oder substituierten aromatischen Kohlenwasserstoffen mit 2, 3, 4 oder mehr aromatischen Ringen, wobei jeweils 2 der Ringe durch eine Einfachbindung oder eine divalente Gruppe, ausgewählt unter
   -CH₂-, -O-, -S- oder -N(H)- miteinander verbunden sind, und wobei die unsubstituierten oder substituierten aromatischen Kohlenwasserstoffe mit 2, 3, 4 oder mehr aromatischen Ringen über eine Einfachbindung oder eine divalente Gruppe, ausgewählt unter -CH₂-, -O-, -S-, -N(R^{a})-, -O-C(=O)- oder -N(R^{b})-C(=O)an den Benzolring der Phthalimidgruppe gebunden sind, wobei R^{a} und R^{b} unabhängig voneinander ausgewählt sind unter Wasserstoff und C₁-C₄-Alkyl,
   Carboxyl, Hydroxy, Nitro, NH₂, Alkylamino und Dialkylamino,
   wobei die Reste R¹ und R² oder R² und R³ oder R³ und R⁴ auch gemeinsam für eine Gruppe der Formel (1.1) stehen können 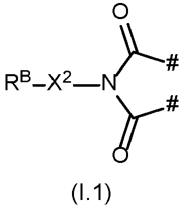 wobei
   - #: jeweils die Bindungsstelle zum Rest des Moleküls bezeichnet,
   wobei einer der Reste R¹, R², R³ oder R⁴ auch gemeinsam mit einem weiteren Rest R¹, R², R³ oder R⁴, der an eine weitere Gruppe der Formel (I) gebunden ist, für eine zweiwertige verbrückende Gruppe Y stehen kann, die die zwei Gruppen der Formel (I) miteinander verbindet,
   - X¹ und, falls vorhanden, X²: unabhängig voneinander für geradkettiges oder verzweigtes C₁-C₃₀-Alkylen steht, wobei die Alkylenkette durch 1 bis 15 nicht benachbarte Heteroatome, ausgewählt unter O und S, unterbrochen sein kann, oder eine Gruppe der Formel #-(CR⁵R⁶)ₙ-SiR⁷R⁸(O-SiR⁷R⁸)ₘ-(CR⁹R¹⁰)ₚ-# steht, worin
   # jeweils die Bindungsstelle zum Rest des Moleküls bezeichnet,
   R⁵, R⁶, R⁹ und R¹⁰ unabhängig voneinander für Wasserstoff oder C₁-C₄-Alkyl stehen,
   R⁷ und R⁸ unabhängig voneinander für geradkettiges oder verzweigtes Alkyl oder für unsubstituiertes oder jeweils mit 1, 2 oder 3 C₁-C₄-Alkylgruppen substituiertes Phenyl stehen,
   n für eine ganze Zahl von 1 bis 30 steht,
   m für eine ganze Zahl von 1 bis 30 steht,
   p für eine ganze Zahl von 1 bis 30 steht, oder eine Gruppe der Formel #-(CR¹¹R¹²)_{q}-NR¹³-(CR¹⁴R¹⁵)ᵣ-# steht, worin
   # jeweils die Bindungsstelle zum Rest des Moleküls bezeichnet,
   R¹¹, R¹², R¹⁴ und R¹⁵ unabhängig voneinander für Wasserstoff oder C₁-C₄-Alkyl stehen,
   R¹³ für Wasserstoff oder C₁-C₄-Alkyl steht,
   q für eine ganze Zahl von 1 bis 30 steht,
   r für eine ganze Zahl von 1 bis 30 steht, oder eine Gruppe der Formel (1.2) 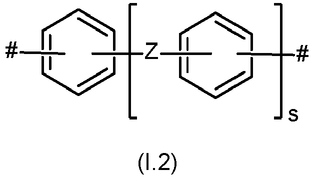 steht, worin
   # jeweils die Bindungsstelle zum Rest des Moleküls bezeichnet,
   Z für O oder S steht,
   s für eine ganze Zahl von 1 bis 30 steht,
   - Y: für eine Gruppe der Formel
   -(O)_{0,1}-(C₁-C₃₀-Alkylen)-(O)_{0,1}- oder -(S)_{0,1}-(C₁-C₃₀-Alkylen)-(S)_{0,1}- oder -(C=O)-(O)_{0,1}-(C₁-C₃₀-Alkylen)-(O)_{0,1}-(C=O)- steht,
   wobei Alkylen geradkettig oder verzweigt ist und durch 1 bis 15 nicht benachbarte Heteroatome, ausgewählt unter O und S, unterbrochen sein kann,
   oder Y für eine Gruppe der Formel
   #-(O)_{0,1}-(CR⁵R⁶)ₙ-SiR⁷R⁸(O-SiR⁷R⁸)ₘ-(CR⁹R¹⁰)ₚ-(O)_{0,1}-# oder
   #-(O)_{0,1}-(CR⁵R⁶)ₙ-SiR⁷R⁸(O-SiR⁷R⁸)ₘ-(CR⁹R¹⁰)ₚ-(O)_{0,1}-#
   steht, worin
   - #: jeweils die Bindungsstelle zum Rest des Moleküls bezeichnet,
   - R⁵, R⁶, R⁹ und R¹⁰: unabhängig voneinander für Wasserstoff oder C₁-C₄-Alkyl stehen,
   - R⁷ und R⁸: unabhängig voneinander für geradkettiges oder verzweigtes Alkyl oder für unsubstituiertes oder jeweils mit 1, 2 oder 3 C₁-C₄-Alkylgruppen substituiertes Phenyl stehen,
   - n: für eine ganze Zahl von 1 bis 30 steht,
   - m: für eine ganze Zahl von 1 bis 30 steht,
   - p: für eine ganze Zahl von 1 bis 30 steht,
   oder Y für eine Gruppe der Formel
   #-(O)_{0,1}-(CR¹¹R¹²)_{q}-NR¹³-(CR¹⁴R¹⁵)ᵣ-(O)_{0,1}-# oder
   #-(O)_{0,1}-(CR¹¹R¹²)_{q}-NR¹³-(CR¹⁴R¹⁵)ᵣ-(S)_{0,1}-#
   steht, worin
   - #: jeweils die Bindungsstelle zum Rest des Moleküls bezeichnet,
   - R¹¹, R¹², R¹⁴ und R¹⁵: unabhängig voneinander für Wasserstoff oder C₁-C₄-Alkyl stehen,
   - R¹³: für Wasserstoff oder C₁-C₄-Alkyl steht,
   - q: für eine ganze Zahl von 1 bis 30 steht,
   - r: für eine ganze Zahl von 1 bis 30 steht,
   oder Y für eine Gruppe der Formel (I.3) 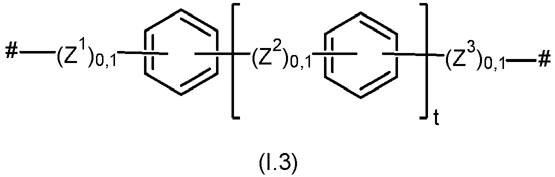 steht, worin
   - #: jeweils die Bindungsstelle zum Rest des Moleküls bezeichnet,
   - Z¹, Z² und Z³: unabhängig voneinander für O oder S stehen,
   - t: für 0 oder eine ganze Zahl von 1 bis 30 steht,

   - R^{A} und, falls vorhanden, R^{B}: unabhängig voneinander für Wasserstoff, unsubstituiertes oder substituiertes Aryl, unsubstituiertes oder substituiertes Aroyl, Monoalkylaminocarbonyl, Monoarylaminocarbonyl, Mono(arylalkylen)aminocarbonyl, Monoalkylmonoarylaminocarbonyl, Monoalkyl(monoarylalkylen)aminocarbonyl, Monoarylmono(arylalkylen)aminocarbonyl, Dialkylaminocarbonyl, Diarylaminocarbonyl, Di(arylalkylen)aminocarbonyl, Monoalkanoylamino, Monoaroylamino, Monoalkanoylmonoaroylamino, Dialkanoylamino, Diaroylamino, wobei die Arylreste von Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Diarylaminocarbonyl, Di(arylalkylen)aminocarbonyl, Monoaroylamino, Monoalkanoylmonoaroylamino und Diaroylamino jeweils unsubstituiert oder substituiert sind, oder eine Phthalimidgruppe der Formel (1.4) 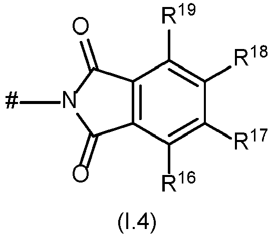
   steht, worin
   - R¹⁶, R¹⁷, R¹⁸ und R¹⁹: unabhängig voneinander die zuvor für R¹, R², R³ und R⁴ angegebene Bedeutung haben,
   und
b) wenigstens ein davon verschiedenes Additiv.

Eine spezielle Ausführungsform der Erfindung ist eine Schmierstoffzusammensetzung in Form eines Schmieröls.

Bevorzugt enthält ein erfindungsgemäßes Schmieröl:
a) 80 bis 99,9 Gew.-%, bevorzugt 90 bis 99,8 Gew.-%, bezogen auf das Gesamtgewicht der Schmierstoffzusammensetzung wenigstens eine Verbindung der allgemeinen Formel (I),
b) 0,1 bis 20 Gew.-%, bevorzugt 0,2 bis 10 Gew.-%, bezogen auf das Gesamtgewicht der Schmierstoffzusammensetzung, wenigstens ein Additiv.

Eine weitere spezielle Ausführungsform der Erfindung ist eine Schmierstoffzusammensetzung in Form eines Schmierfetts. Schmierfette enthalten wenigstens einen Verdicker als Additiv.

Bevorzugt enthält ein erfindungsgemäßes Schmierfett:
a1) wenigstens eine Verbindung der allgemeinen Formel (I) als Basisöl,
a2) gegebenenfalls wenigstens ein von a1) verschiedenes weiteres Basisöl,
b1) wenigstens einen Verdicker,
b2) gegebenenfalls wenigstens ein weiteres Additiv.

Ein weiterer Gegenstand der Erfindung ist die Verwendung von Phthalimidverbindungen der allgemeinen Formel (I), wie zuvor und im Folgenden definiert, als Basisöl einer Schmierstoffzusammensetzung zum Schmieren von tribologischen Systemen.

Ein weiterer Gegenstand der Erfindung ist die Verwendung von Phthalimidverbindungen der allgemeinen Formel (I), wie zuvor und im Folgenden definiert, als Basisöl eines Hochtemperaturschmierfetts zur Schmierung von Gleitlagern, zur Schmierung von Wälzlagern und/oder zum Antrieb von Produktionsanlagen in der chemischen Industrie, wobei die Gleitlager, die Wälzlagern und/oder als Schmierfett zum Einsatz in Produktionsanlagen in der Chemischen Industrie.

Ein weiterer Gegenstand der Erfindung ist die Verwendung von Phthalimidverbindungen der allgemeinen Formel (I), wie zuvor und im Folgenden definiert, als Basisöl eines Hochtemperaturschmierfetts
- zur Schmierung von Gleitlagern, insbesondere Gasarmaturen, Aktuatoren, Linearführungen, Regel- und Steuerklappen im Ansaugkrümmer, Kupplungen, Schrauben, Bolzen, Fittings, Ketten für die Lebensmittelindustrie, insbesondere in Brot- und Waffelbackautomaten;
- zur Schmierung von Wälzlagern, insbesondere Wälzlagern für Holzpress- oder Wellpappanlagen und/oder
- zum Antrieb von Produktionsanlagen in der Chemischen Industrie.

Ein weiterer Gegenstand der Erfindung ist die Verwendung einer Phthalimidverbindung der Formel (I), wie zuvor und im Folgenden definiert, als Komponente einer Schmierstoffzusammensetzung zum teilweisen oder vollständigen Ersatz einer fluorhaltigen Schmierstoffkomponente, insbesondere eines linearen oder verzweigten Perfluoropolyetheröls (PFPE-Öls).

### BESCHREIBUNG DER ERFINDUNG

Ein tribologisches System ist ganz allgemein ein technisches System, welches Bewegung durch Kontakte ermöglicht, beeinflusst oder verhindert und das als wesentliche Komponenten die an einer tribologischen Beanspruchung beteiligten Wirkflächenpaare und das auf diese einwirkende Beanspruchungskollektiv umfasst. Typischerweise umfasst ein tribologisches System wenigstens einen Grundkörper, der sich in Kontakt und Relativbewegung mit wenigstens einem Gegenkörper befindet. Das Beanspruchungskollektiv umfasst die auf die Körper aufgebrachte Belastung sowie die Bewegungsverhältnisse, den Reibungszustand und die Temperatur. Zwischen den beiden Körpern befindet sich häufig ein Zwischenstoff, wobei es sich speziell um gezielt zur Verschleißminderung eingebrachte Schmierstoffe handeln kann.

Im Rahmen der Erfindung bezeichnet ein tribologisches System speziell ein System, bei dem Reibung infolge der Relativbewegung von aufeinander einwirkenden Oberflächen entsteht, und einer Schmierung, um Verschleiß durch die Beanspruchung der Oberflächen infolge der Reibung zu minimieren. Schmierstoffe reduzieren den Verschleiß und sichern die Funktion von Teilen, auf die Reibungskräfte einwirken. Typische tribologische Systeme für den Einsatz der erfindungsgemäßen Schmierstoffzusammensetzungen sind Lager, Ketten, Kompressoren, Getriebe, etc.

Die erfindungsgemäßen Schmierstoffzusammensetzungen haben folgende Vorteile:
- Die erfindungsgemäßen Schmierstoffzusammensetzungen und die darin enthaltenen Phthalimidverbindungen zeichnen sich insbesondere durch eine sehr gute Oxidationsstabilität aus.
- Die Schmierstoffzusammensetzungen und die Phthalimidverbindungen weisen ein Eigenschaftsprofil bezüglich des Viskositäts- und Temperaturverhaltens auf, dass sie für den Einsatz als Hochleistungsschmierstoffe befähigt.
- Die Phthalimidverbindungen der Formel (I), eignen sich vorteilhaft als Komponente einer Schmierstoffzusammensetzung zum teilweisen oder vollständigen Ersatz einer fluorhaltigen Schmierstoffkomponente, insbesondere eines linearen oder verzweigten Perfluoropolyetheröls (PFPE-Öls).
- Die Phthalimidverbindungen der Formel (I) zeichnen sich durch eine hohe Kompatibilität, speziell Mischbarkeit, mit einer Vielzahl weiterer Basisöle aus.
- Die Phthalimidverbindungen der Formel (I) zeichnen sich weiterhin durch eine hohe Kompatibilität mit einer Vielzahl von Schmierstoffadditiven aus.
- Die erfindungsgemäßen Schmierstoffzusammensetzungen verfügen über sehr gute tribologische Eigenschaften. Insbesondere zeigen sie bei hoher Temperatur über einen langen Zeitraum eine gute Schmierwirkung. Sie weisen eine gute hydrolytische Stabilität auf und sind korrosions- und verschleißresistent.
- Die erfindungsgemäßen Schmierstoffzusammensetzungen verfügen auch über ein gutes Tieftemperaturverhalten, speziell einen niedrigen pour point.

### Phthalimidverbindunaen der Formel (I)

In den abgebildeten Formelzeichnungen kann eine Methylgruppe als eine durchgezogene Linie dargestellt sein. So sind zum Beispiel die folgenden Formeln zwei Alternativen zur Darstellung derselben Verbindung:

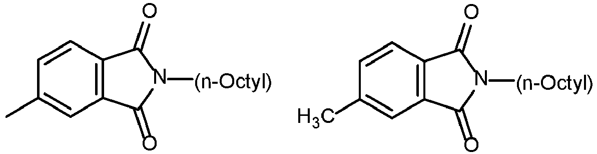

Es wird auch darauf hingewiesen, dass in den abgebildeten Formeln Wasserstoffatome im Allgemeinen nicht dargestellt werden, wie es der üblichen Praxis entspricht. Entsprechend bedeutet die Definition, dass ein aromatischer Ring, z. B. ein Benzolring, durch 0 bis x Substituenten substituiert sein kann, dass Ringatome, die substituiert werden können, aber keinen Substituenten tragen, stattdessen Wasserstoffatome tragen.

Die in den Formeln angegebenen Definitionen der Variablen verwenden Sammelbegriffe, die im Allgemeinen für die jeweiligen Substituenten repräsentativ sind. Die Definition Cₙ-Cₘ gibt die Anzahl der jeweils möglichen Kohlenstoffatome im jeweiligen Substituenten oder Substituententeil an.

Der hierin verwendete Begriff "unverzweigt" wird auch als linear oder geradkettig bezeichnet.

Der Begriff "Cₙ-Cₘ-Alkyl", wie er hier verwendet wird, bezieht sich auf eine verzweigte oder unverzweigte gesättigte Kohlenwasserstoffgruppe mit n bis m Kohlenstoffatomen, z.B. 1 bis 4 ("C₁-C₄-Alkyl") oder 1 bis 20 ("C₁-C₂₀-Alkyl"). Dazu zählen beispielsweise C₁-C₄-Alkyl, wie Methyl, Ethyl, Propyl, Isopropyl, n-Butyl, Isobutyl, sec.-Butyl und tert.-Butyl. Geeignete höherkettige C₅-C₂₀-Alkyle sind z.B. n-Pentyl, 2-Pentyl, 2-Methylbutyl, 3-Methylbutyl, 1,2- Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1 ,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Nonanyl und dessen verzweigte Isomere, n-Decanyl und dessen verzweigte Isomere, n-Undecanyl und dessen verzweigte Isomere, n-Dodecanyl und dessen verzweigte Isomere, n-Tridecanyl und dessen verzweigte Isomere, n-Tetradecanyl und dessen verzweigte Isomere, n-Pentadecanyl und dessen verzweigte Isomere, n-Hexedecanyl und dessen verzweigte Isomere, n-Heptadecanyl und dessen verzweigte Isomere, n-Octadecanyl und dessen verzweigte Isomere, n-Nonadecanyl und dessen verzweigte Isomere, n-Eicosanyl und dessen verzweigte Isomere und dergleichen. Das gleiche gilt für die Alkylreste in Alkoxy, Alkanoyl, Alkyloxycarbonyl, Monoalkylaminocarbonyl, Monoalkylmonoarylaminocarbonyl, Monoalkyl(monoarylalkenyl)-aminocarbonyl, Dialkylaminocarbonyl, Monoalkanoylamino, Monoalkanoylmonoaroylamino, Dialkanoylamino, etc.

Der Ausdruck Aryl bezeichnet monocyclische, bicyclische, tricyclische, tetracyclische oder höhere aromatische Kohlenwasserstoffreste mit vorzugsweise 6 bis 18 Ringkohlenstoffatomen, bei denen die Ringe alle kondensiert sind oder zwei der aromatischen Ringe auch durch eine chemische Bindung und einen zweiwertigen Rest, ausgewählt aus -CH₂-, -O-, -S- oder N(H)-, miteinander verbunden sein können. Beispiele sind Phenyl, Naphthyl, Anthracenyl, Phenanthrenyl, Fluorenyl, Dibenzofuranyl, Dibenzothienyl, Carbazolyl, 11H-Benzo[b]fluorenyl, Naphtho[2,3-b]benzofuryl, Naphtho[2,3-b]benzothienyl und 5H-Benzo[b]carbazolyl. Aryl kann an einer, zwei, drei, vier, mehr als vier oder an allen substituierbaren Stellen substituiert sein. Geeignete Substituenten sind im Allgemeinen C₁-C₂₀-Alkyl, C₁-C₄-Alkoxy, Acyl, Dialkylamino, Arylalkylenamino, Diarylamino, C₅-C₈-Cycloalkyl, etc. Das gleiche gilt für die Arylreste in Aryloxy, Arylalkylenoxy, Arylthio, Arylalkylenthio, Aroyl, Aryloxycarbonyl, Arylcarbonyloxy, Monoarylaminocarbonyl, Mono(arylalkylen)-aminocarbonyl, Monoalkylmonoaryl¬aminocarbonyl, Monoalkyl-mono(arylalkylen)-aminocarbonyl, Monoarylmono(arylalkylen)aminocarbonyl, Diaryl-aminocarbonyl, Di(arylalkylen)aminocarbonyl, Monoaroylamino, Monoalkanoylmonoaroylamino, Diaroylamino, etc.

Speziell sind die Arylreste in Aryloxy, Arylalkylenoxy, Arylthio, Arylalkylenthio, Aroyl, Aryloxycarbonyl, Arylcarbonyloxy, Monoarylaminocarbonyl, Mono(arylalkylen)-aminocarbonyl, Monoalkylmonoaryl¬aminocarbonyl, Monoalkyl-mono(arylalkylen)¬aminocarbonyl, Monoarylmono(arylalkylen)aminocarbonyl, Diarylaminocarbonyl, Di(arylalkylen)aminocarbonyl, Monoaroylamino, Monoalkanoylmonoaroylamino und Diaroylamino unsubstituiert oder weisen 1, 2, 3 oder 4 Substituenten auf, vorzugsweise unabhängig voneinander ausgewählt unter C₁-C₂₀-Alkyl und C₁-C₄-Alkoxy. Speziell sind die Substituenten der Arylreste ausgewählt unter C₁-C₄-Alkyl und C₁-C₄-Alkoxy, insbesondere Methyl, Ethyl, n-Propyl, Isopropyl, n-Butyl, sec.-Butyl, tert.-Butyl, Methoxy und Ethoxy.

Im Rahmen der vorliegenden Erfindung bezieht sich der Ausdruck "Alkylen" (Alkandiyl) auf zweiwertige Kohlenwasserstoffreste mit vorzugsweis 1 bis 30, besonders bevorzugt 2 bis 20 Kohlenstoffatomen. Die zweiwertigen Kohlenwasserstoffreste können unverzweigt oder verzweigt sein. Dazu zählen beispielsweise C₁-C₃₀-Alkylengruppen, wie Methylen, Ethylen, 1,2-Propylen, 1,3-Propylen, 1,4-Butylen, 1,5-Pentylen, 1,6- Hexylen, 2-Methyl-1,5-pentylen, 3-Methyl-1,5-pentylen, 1,7-Heptylen, 2-Methyl-1,6-hexylen, 3-Methyl-1 ,6-hexylene, 1 8-Octylen, 2-Methyl-1,7-heptylen, 2-Ethyl-1,6-hexylen, 1,9-Nonylen, 2-Methyl-1,8-Octylen, 3-Methyl-1,8-Octylen, 4-Methyl-1,8-Octylen, 1,10-Decylen, 1,11-Undecylen, 1,12-Dodecylen, 1,13-Tridecylen, 1,14-Tetradecylen, 1,15-Pentadecylen, 1,16-Hexadecylen 1,18-Octadecylen, 1,20-Eicosanylen, 1,25-Pentacosanylen, 1,30-Triacontanylen und dergleichen.

Bevorzugt steht in den Verbindungen der Formel (I) wenigstens einer der Reste R¹, R², R³ und R⁴ und/oder, falls vorhanden, wenigstens einer der Reste R¹⁶, R¹⁷, R¹⁸ und R¹⁹ für eine Gruppe, ausgewählt unter den Gruppen (AR-I) bis (AR-XXIV) 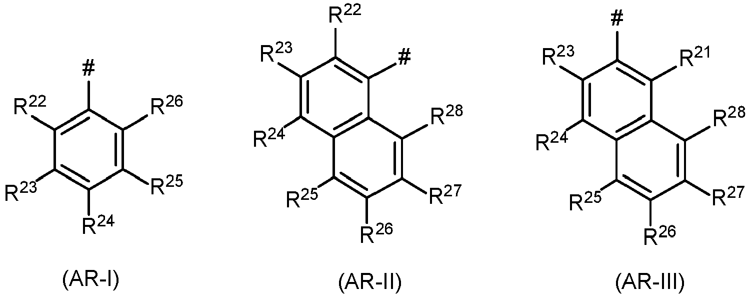 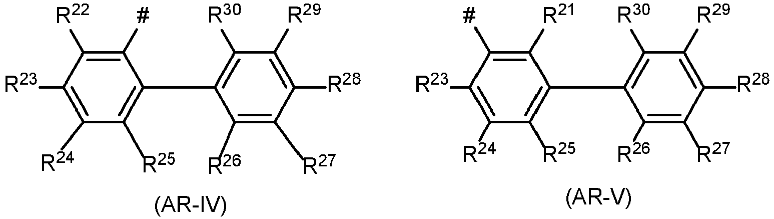 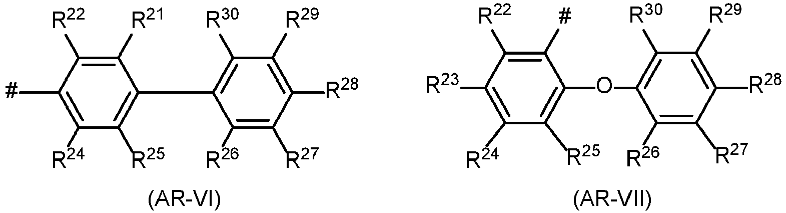 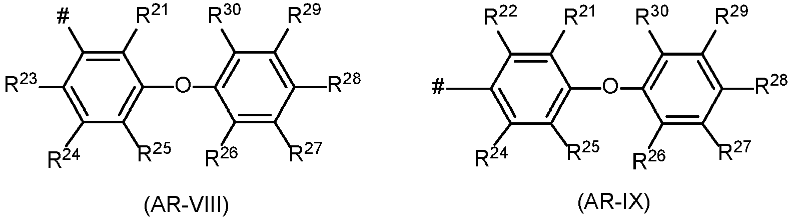 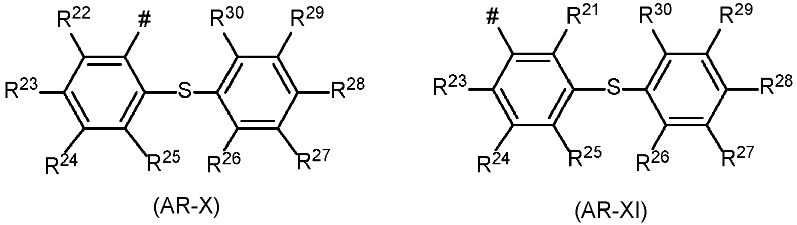 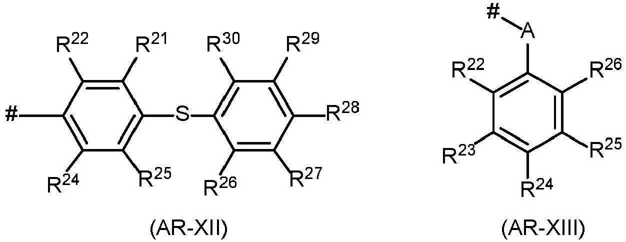 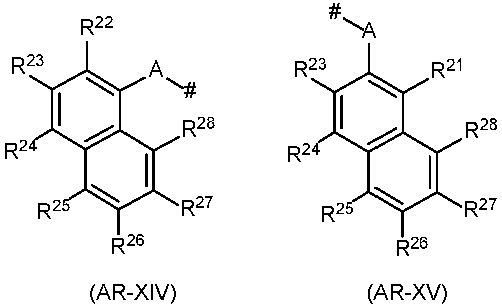 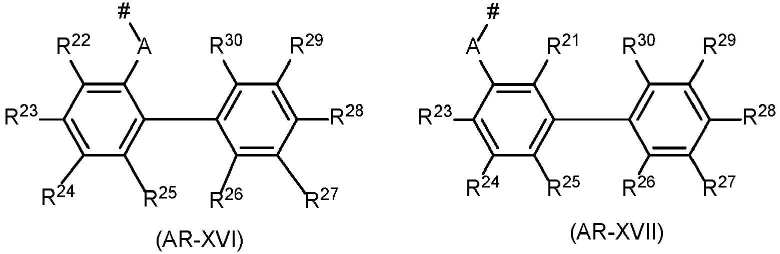 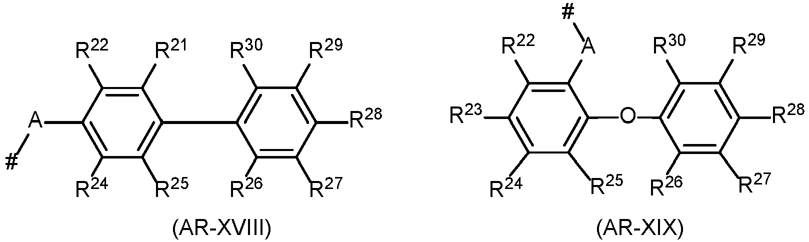 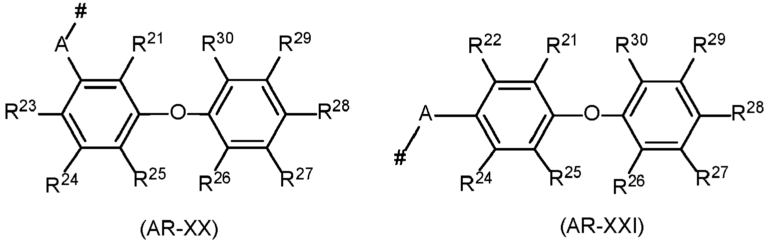 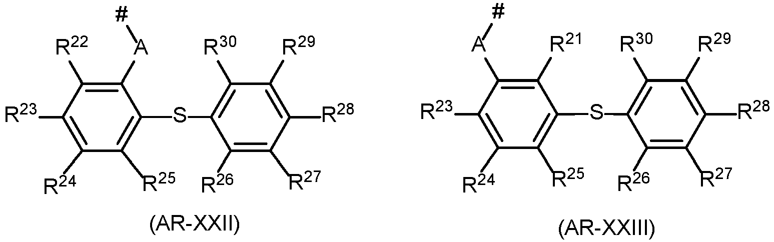 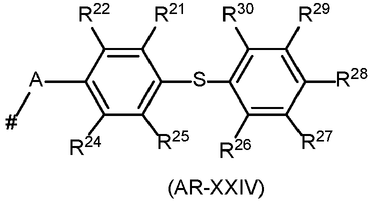 worin
- #: jeweils die Bindungsstelle zum Rest des Moleküls bezeichnet,
- A: ausgewählt ist unter den Gruppen 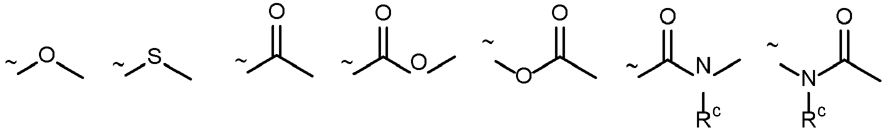
worin
~ jeweils die Bindungsstelle der Gruppe A zum Benzolring bezeichnet,
R^{c} für Wasserstoff oder C₁-C₄-Alkyl steht,
R²¹, R²², R²³, R²⁴, R²⁵, R²⁶, R²⁷, R²⁸, R²⁹ und R³⁰, falls vorhanden, unabhängig voneinander ausgewählt sind unter Wasserstoff, geradkettigem oder verzweigtem C₁-C₂₀-Alkyl und geradkettigem oder verzweigtem C₁-C₄-Alkoxy.

In einer bevorzugten Ausführungsform steht in den Verbindungen der Formel (I) wenigstens einer der Reste R¹, R², R³ und R⁴ für Wasserstoff.

In einer weiteren bevorzugten Ausführungsform stehen in den Verbindungen der Formel (I) wenigstens zwei der Reste R¹, R², R³ und R⁴ für Wasserstoff.

In einer weiteren bevorzugten Ausführungsform stehen in den Verbindungen der Formel (I) wenigstens drei der Reste R¹, R², R³ und R⁴ für Wasserstoff.

Insbesondere stehen in den Verbindungen der Formel (I) die Reste R¹, R³ und R⁴ für Wasserstoff oder die Reste R², R³ und R⁴ für Wasserstoff oder die Reste R¹, R², R³ und R⁴ für Wasserstoff.

In einer bevorzugten Ausführungsform weisen die Verbindungen der Formel (I) eine Phthalimidgruppe der Formel (I.4) auf und steht wenigstens einer der Reste R¹⁶, R¹⁷, R¹⁸ und R¹⁹ für Wasserstoff.

In einer weiteren bevorzugten Ausführungsform weisen die Verbindungen der Formel (I) eine Phthalimidgruppe der Formel (I.4) auf und stehen wenigstens zwei der Reste R¹⁶, R¹⁷, R¹⁸ und R¹⁹ für Wasserstoff stehen.

In einer weiteren bevorzugten Ausführungsform weisen die Verbindungen der Formel (I) eine Phthalimidgruppe der Formel (I.4) auf und stehen wenigstens drei der Reste R¹⁶, R¹⁷, R¹⁸ und R¹⁹ für Wasserstoff stehen.

Insbesondere weisen die Verbindungen der Formel (I) eine Phthalimidgruppe der Formel (I.4) auf und stehen die Reste R¹⁶, R¹⁸ und R¹⁹ für Wasserstoff oder die Reste R¹⁷, R¹⁸ und R¹⁹ für Wasserstoff oder die Reste R¹⁶, R¹⁷, R¹⁸ und R¹⁹ für Wasserstoff.

Bevorzugt stehen in den Verbindungen der Formel (I) X¹ und, falls vorhanden, X² unabhängig voneinander für
- geradkettiges oder verzweigtes C₁-C₂₀-Alkylen, oder
- eine Gruppe der Formel #-(CH₂CH₂O)₁₋₁₀-(CH₂CH₂)-#, oder
- eine Gruppe der Formel #-(CH₂)₁₋₅-Si(CH₃)₂-(O-(Si(CH₃)₂))₁₋₅-(CH₂)₁₋₅-#, oder
- eine Gruppe der Formel #-(CH₂)₁₋₅-NR¹³-(CH₂)₁₋₅-#, worin R¹³ für Wasserstoff oder C₁-C₄-Alkyl steht, oder
- oder eine Gruppe der Formel (I.2a) 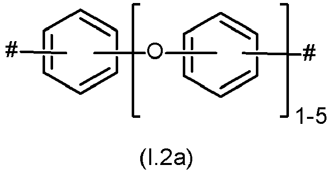
worin
- #: jeweils die Bindungsstelle zum Rest des Moleküls bezeichnet.

Bevorzugt steht in den Verbindungen der Formel (I) die Gruppe Y für
- -(O)_{0,1}-(C₁-C₂₀-Alkylen)-(O)_{0,1}- oder -(S)_{0,1}-(C₁-C₂₀-Alkylen)-(S)_{0,1}- oder -(C=O)-(O)_{0,1}-(C₁-C₃₀-Alkylen)-(O)_{0,1}-(C=O)-, wobei Alkylen geradkettig oder verzweigt ist, oder
- eine Gruppe der Formel #-(O)_{0,1}-(CH₂CH₂O)₁₋₁₀-(CH₂CH₂)-(O)_{0,1}-#, oder
- eine Gruppe der Formel #-(O)_{0,1}-(CH₂)₁₋₅-Si(CH₃)₂-(O-(Si(CH₃)₂))₁₋₅-(CH₂)₁₋₅-(O)_{0,1}-#, oder
- eine Gruppe der Formel #-(O)_{0,1}-(CH₂)₁₋₅-NR¹³-(CH₂)₁₋₅-(O)_{0,1}-#, worin R¹³ für Wasserstoff oder C₁-C₄-Alkyl steht, oder
- oder eine Gruppe der Formel (I.3a) 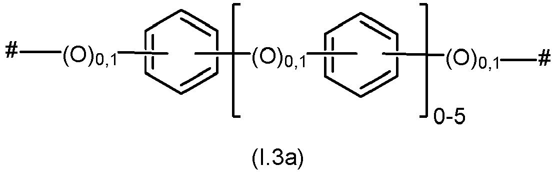 worin
   - #: jeweils die Bindungsstelle zum Rest des Moleküls bezeichnet.

In einer speziellen Ausführungsform steht in den Verbindungen der Formel (I) wenigstens einer der Reste R^{A} und, falls vorhanden, R^{B} für eine Gruppe (AR-I) bis (AR-XXIV), wie zuvor definiert.

In einer speziellen Ausführungsform steht in den Verbindungen der Formel (I) wenigstens einer der Reste R^{A} und, falls vorhanden, R^{B} für eine Phthalimidgruppe der Formel (I.4).

Bevorzugt ist die Verbindung der Formel (I), ausgewählt unter den folgenden Verbindungen (1) bis (116) und den durch Reduktion der nicht-amidischen Carbonylgruppe(n) der Verbindungen (1) - (9), (22) - (27) und (71) - (90) erhältlichen Verbindungen. Des Weiteren bevorzugt umfasst die erfindungsgemäße Schmierstoffzusammensetzung wenigstens eine Verbindung der Formel (I), ausgewählt unter Verbindungen (1) bis (116) und den durch Reduktion der nicht-amidischen Carbonylgruppe(n) der Verbindungen (1) - (9), (22) - (27) und (71) - (90) erhältlichen Verbindungen 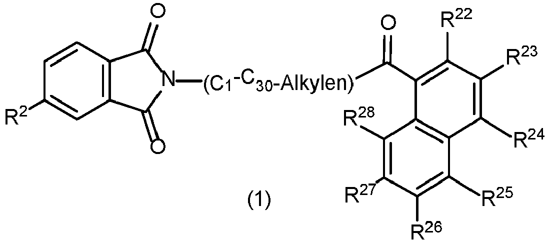 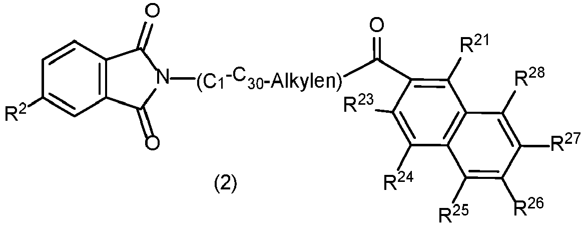 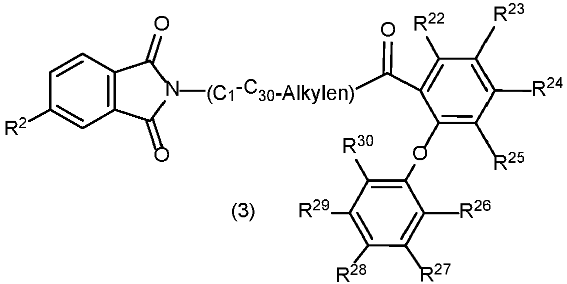 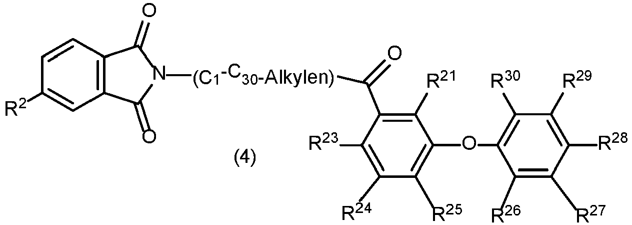 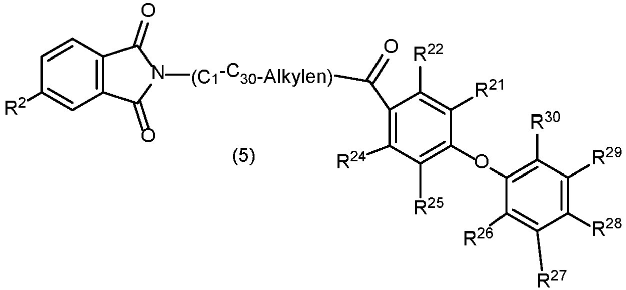 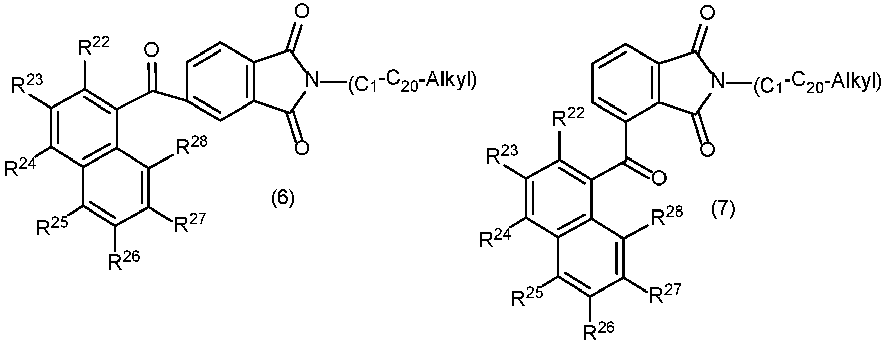 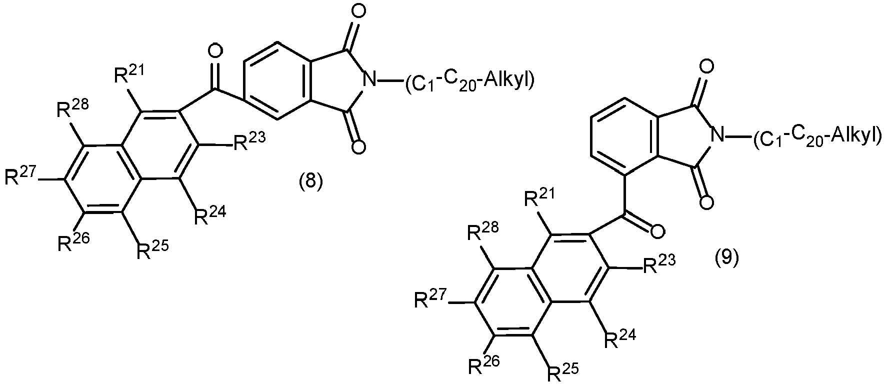 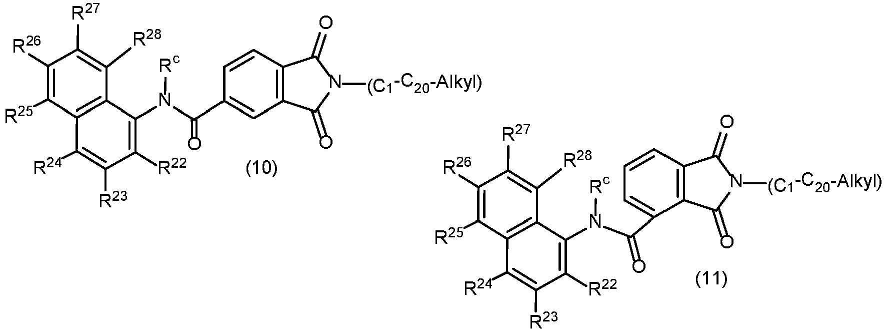 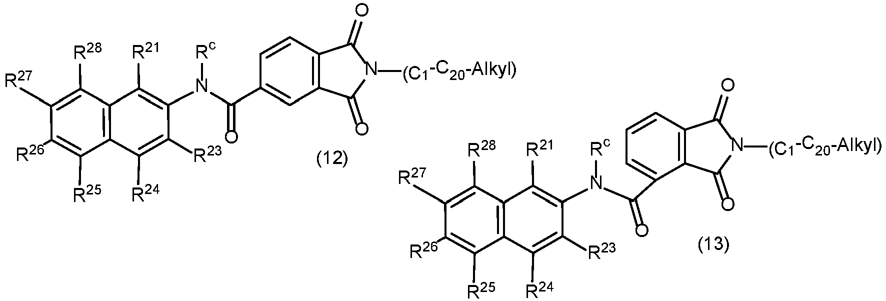 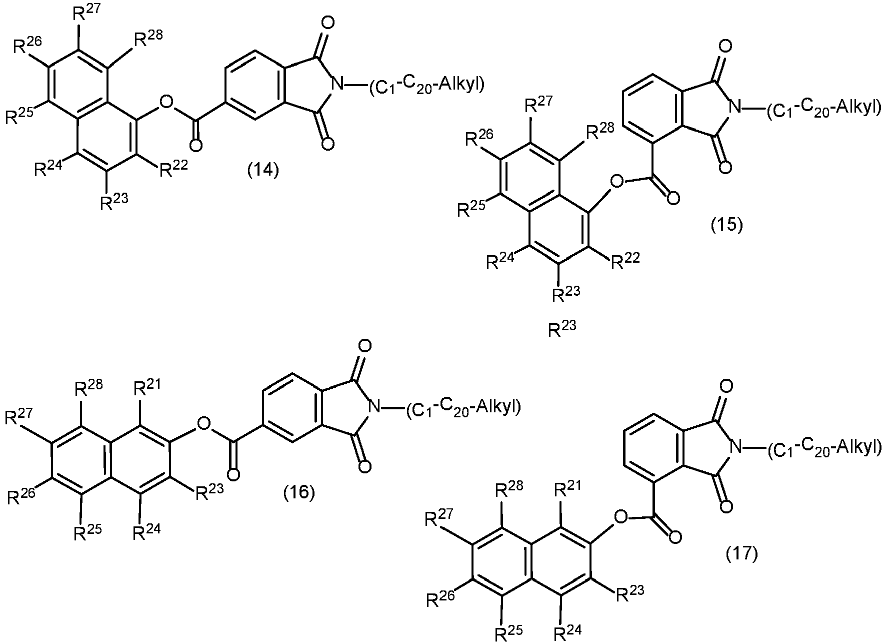 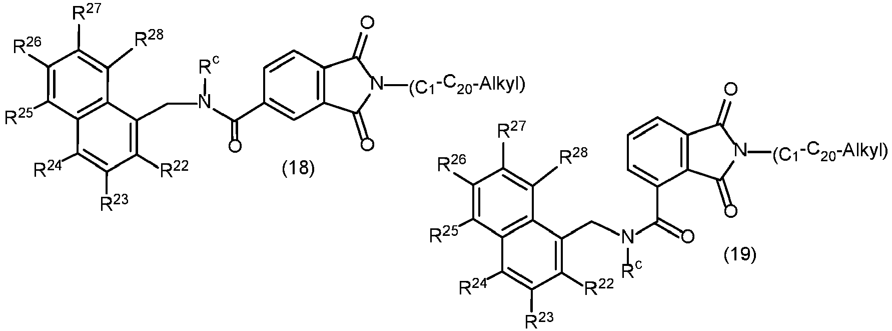 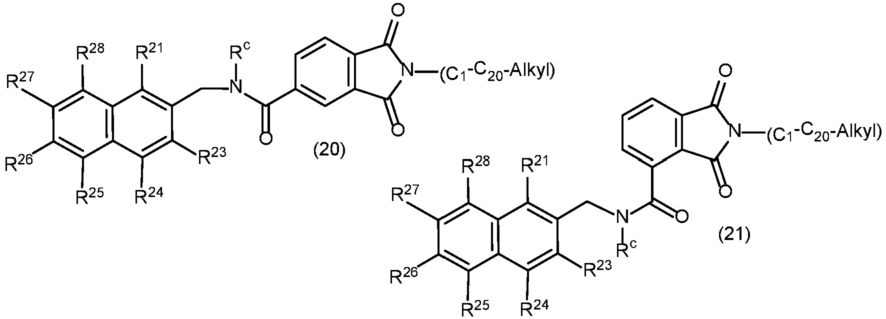 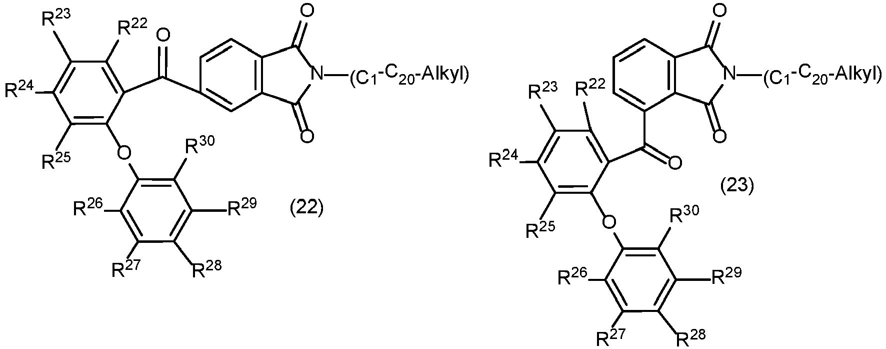 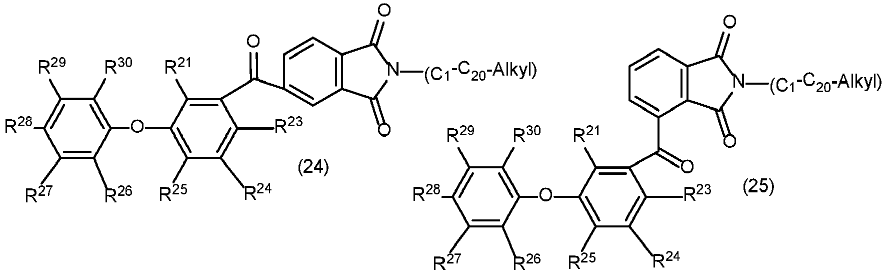 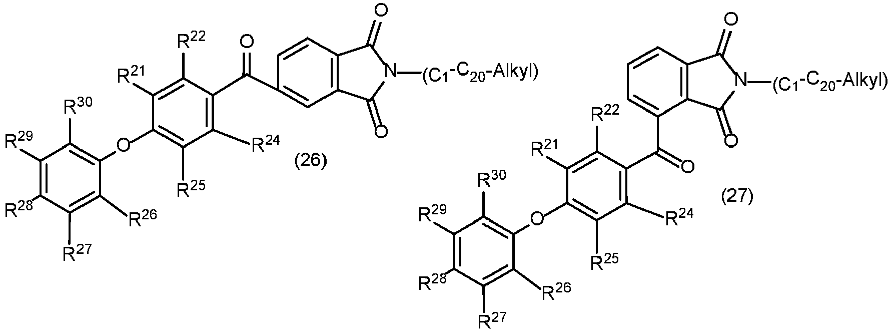 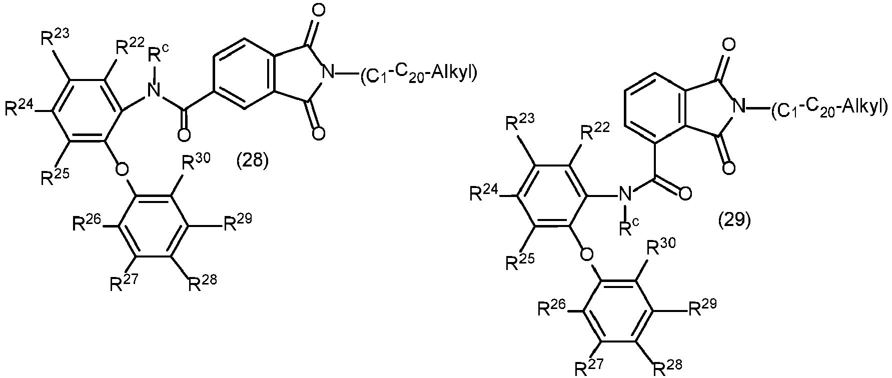 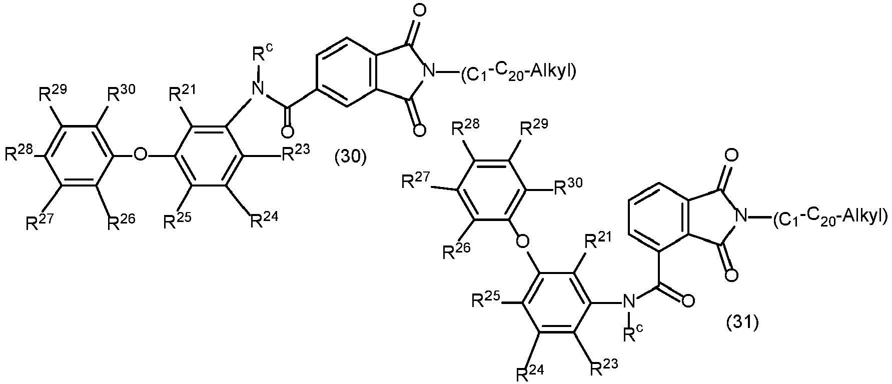 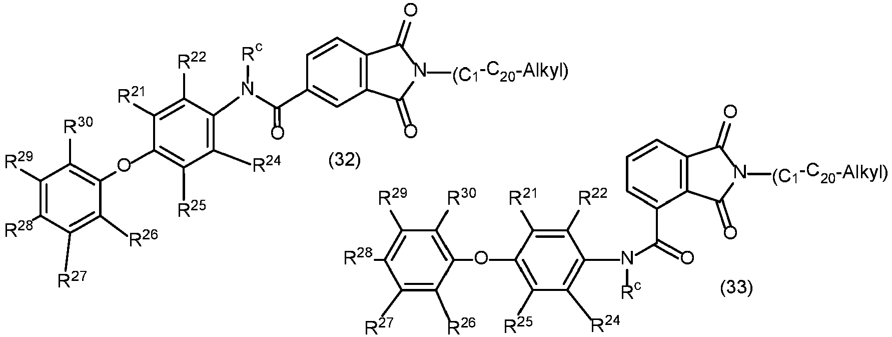 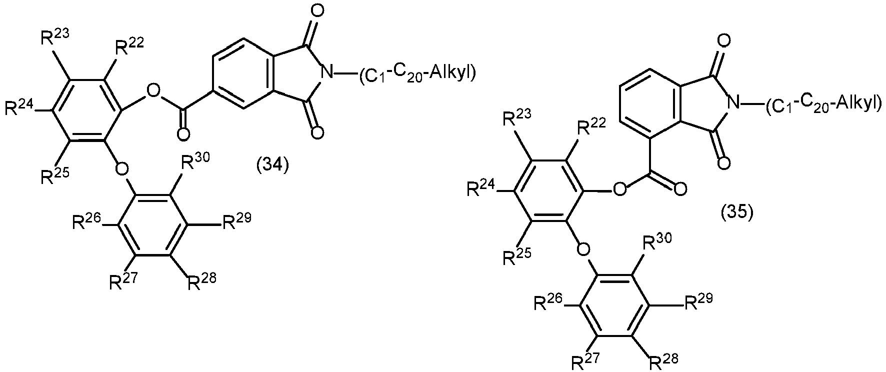 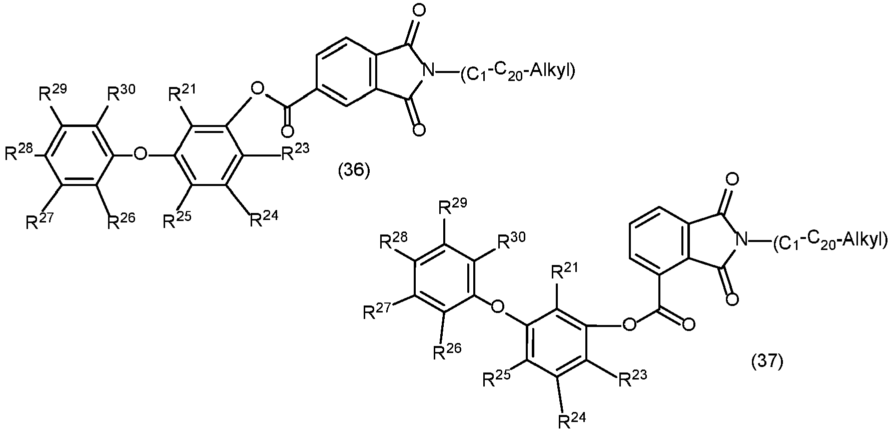 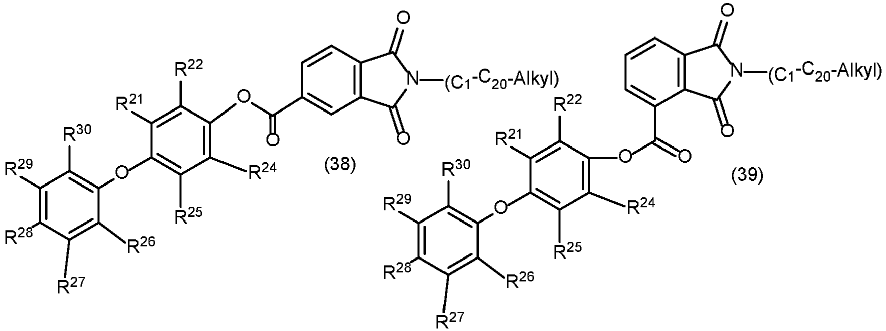 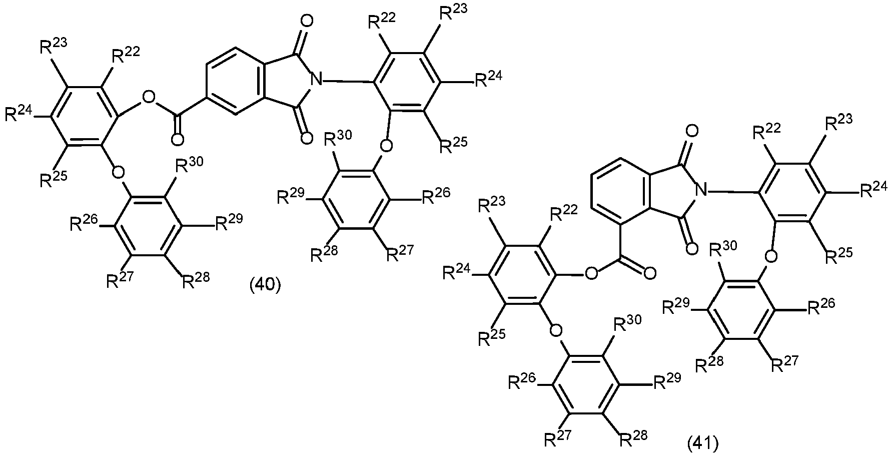 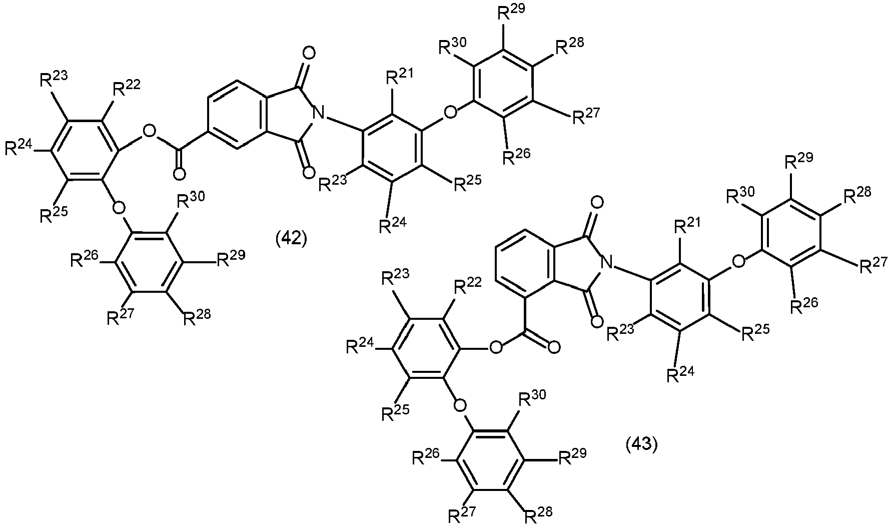 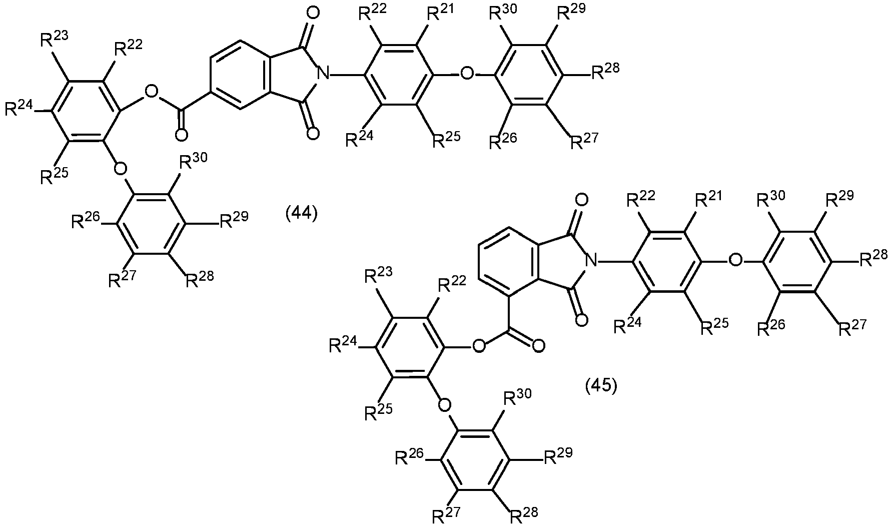 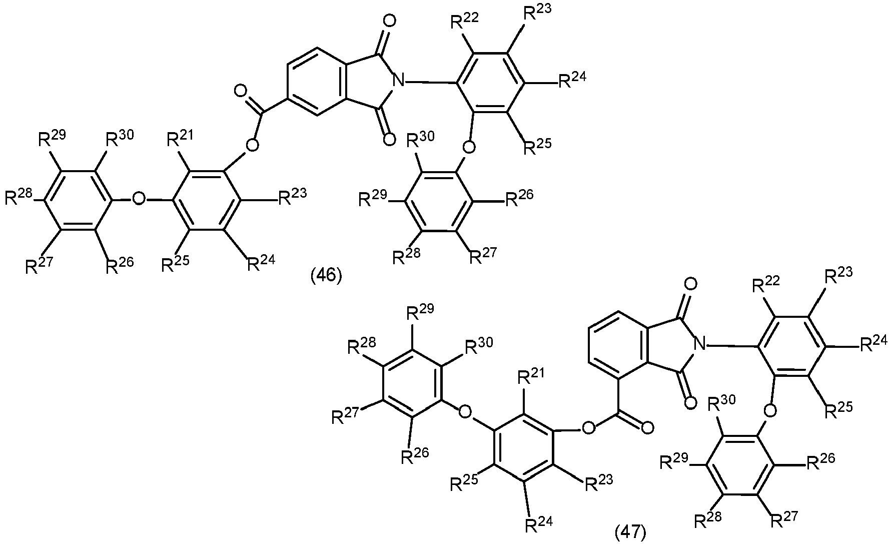 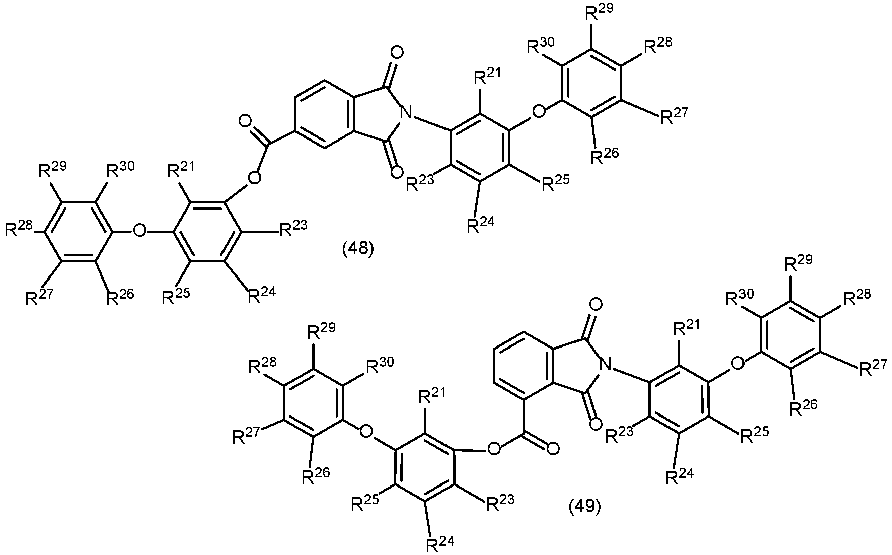 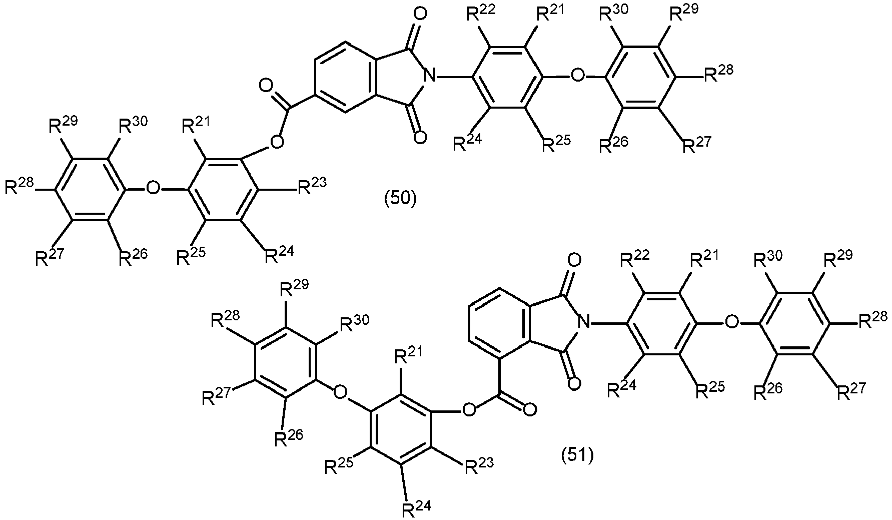 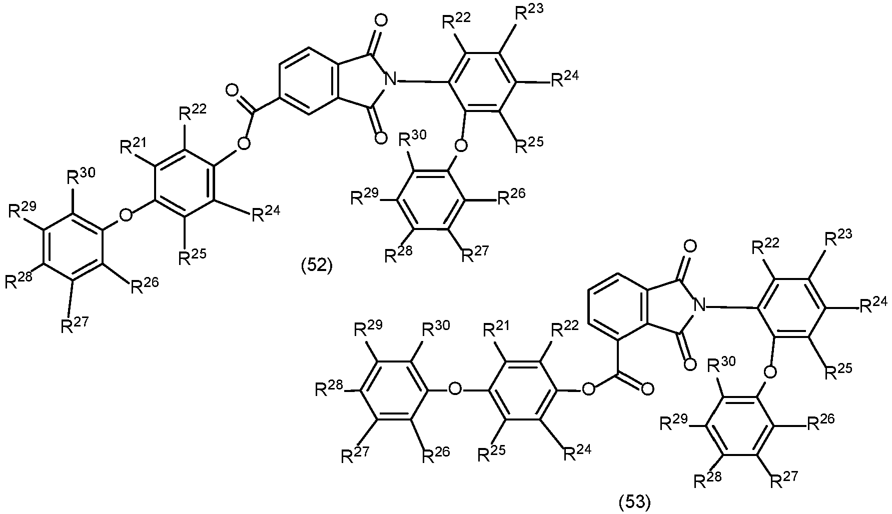 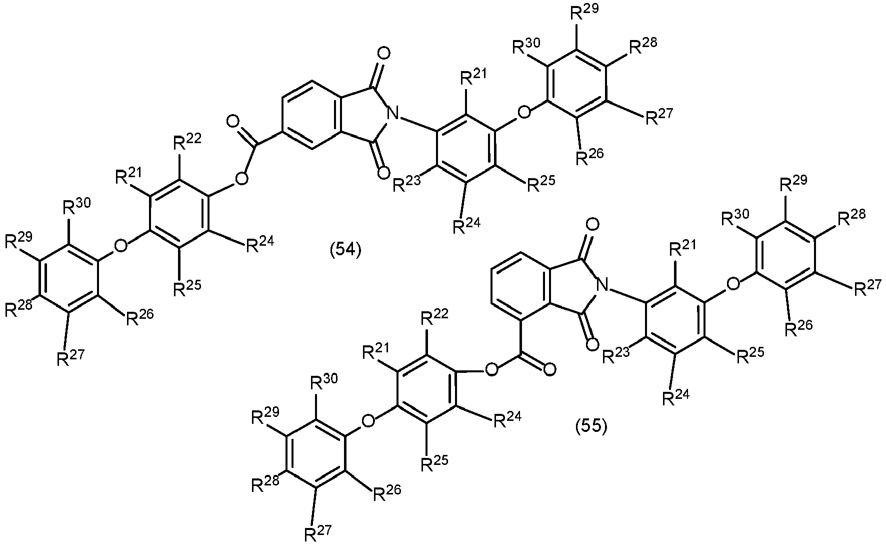 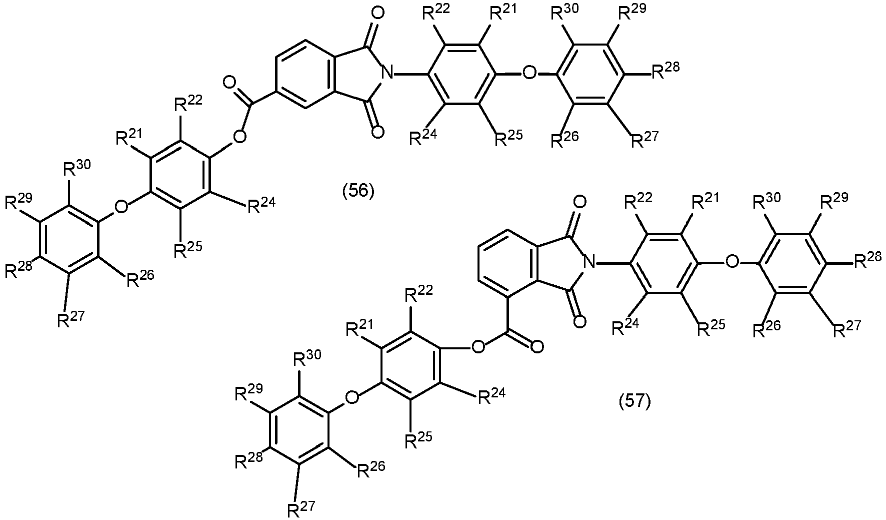 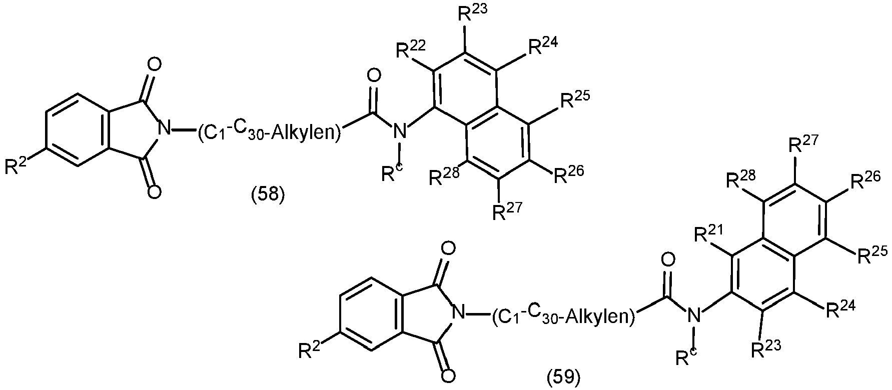 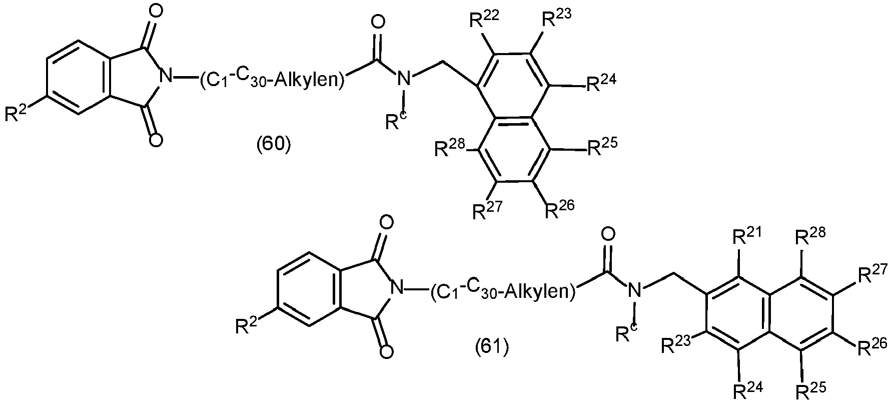 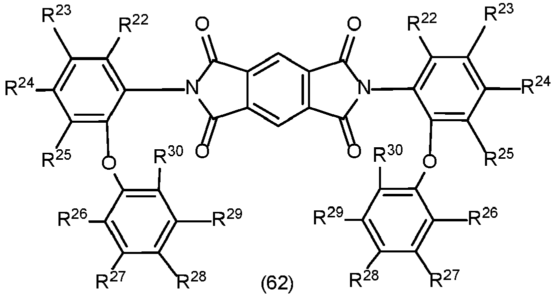 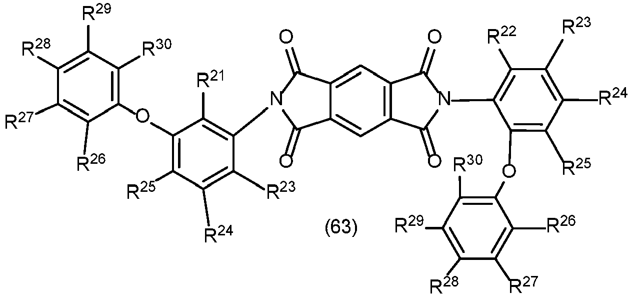 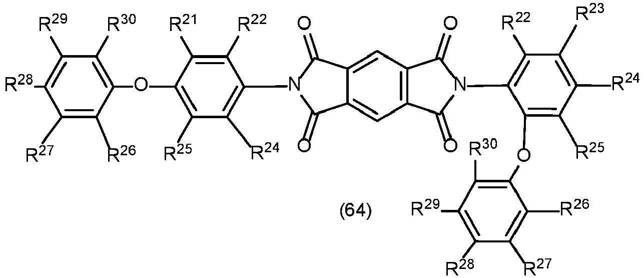 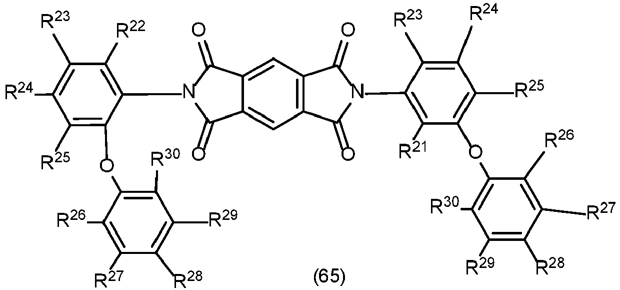 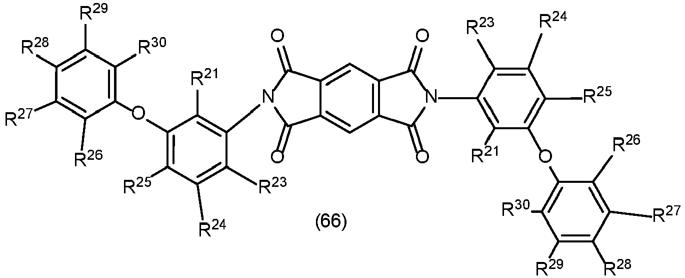 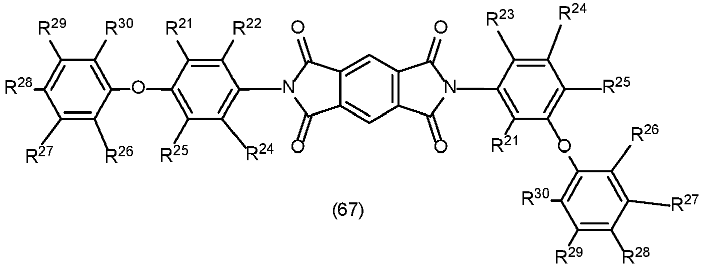 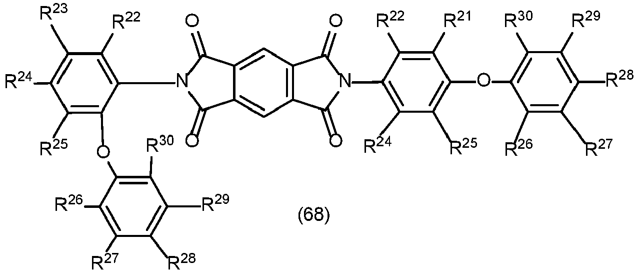 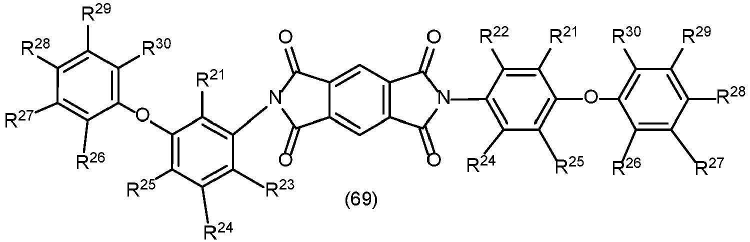 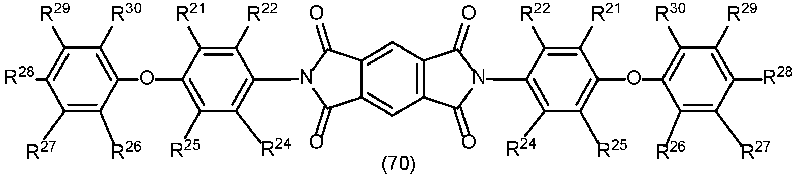 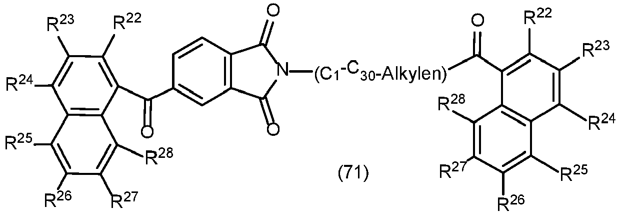 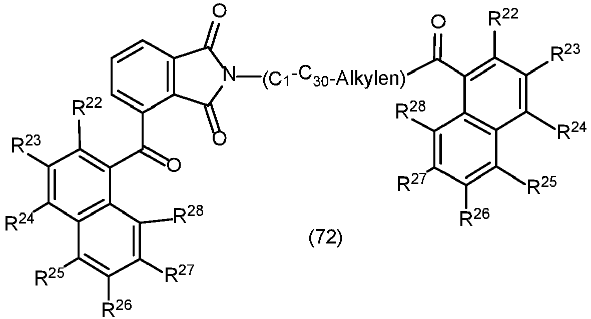 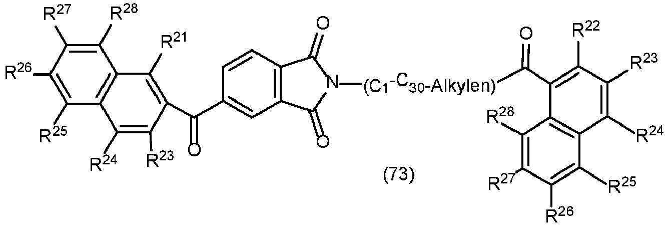 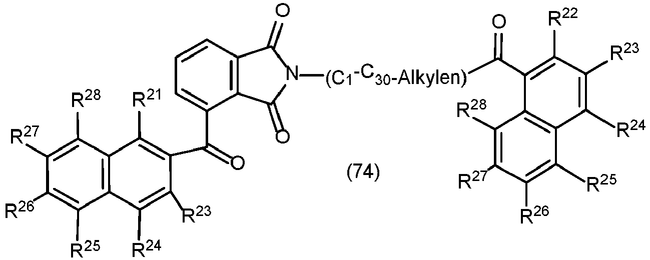 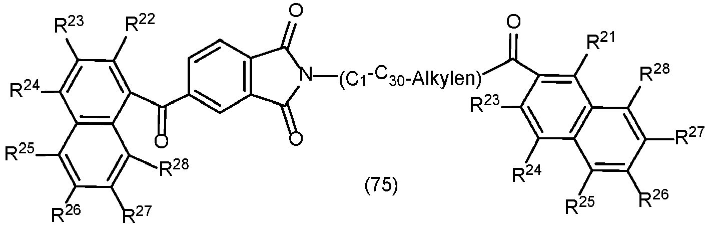 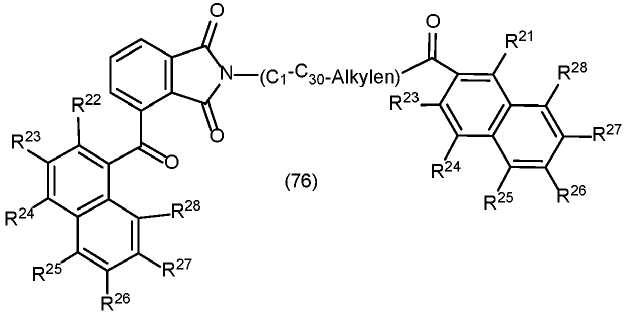 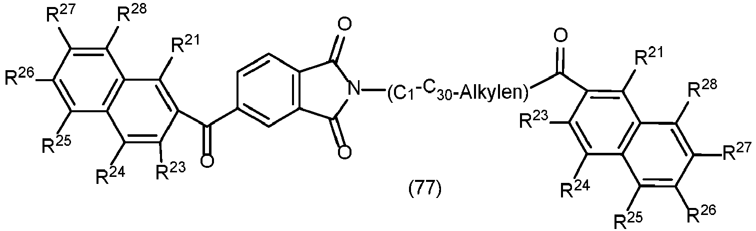 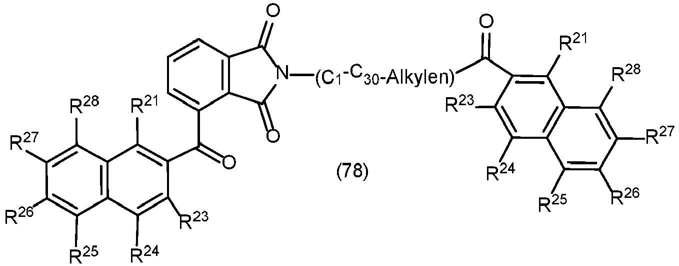 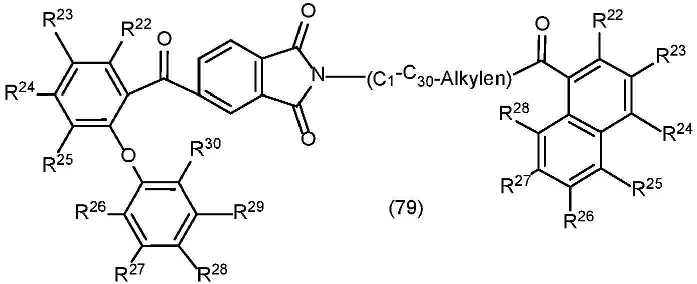 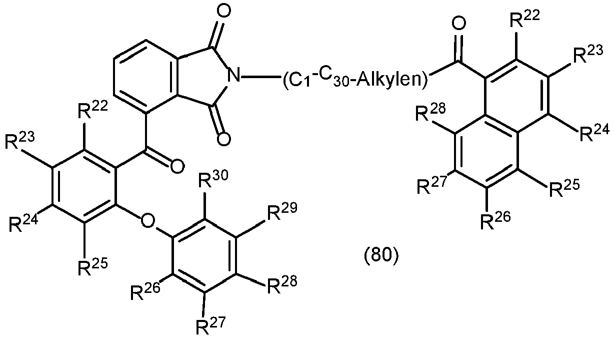 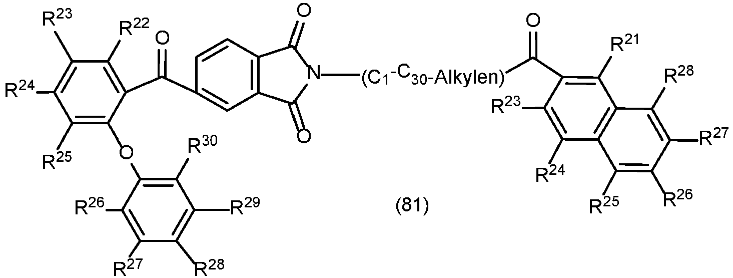 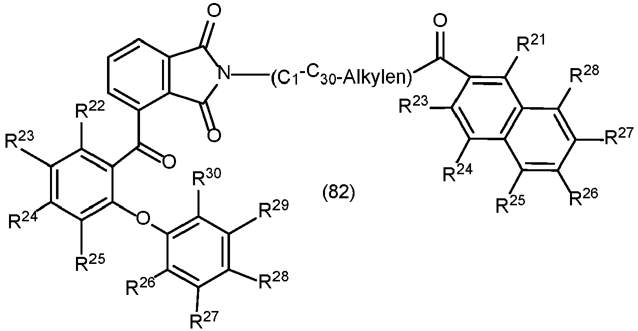 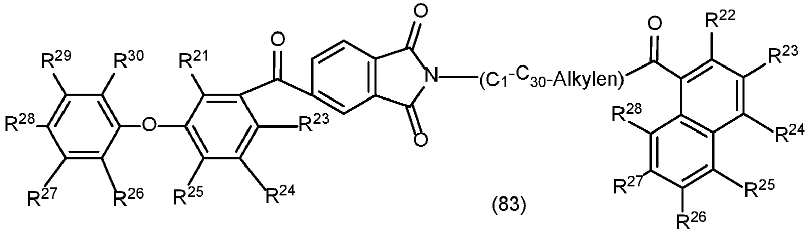 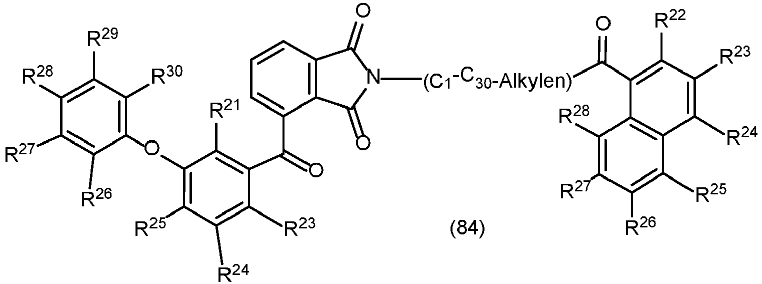 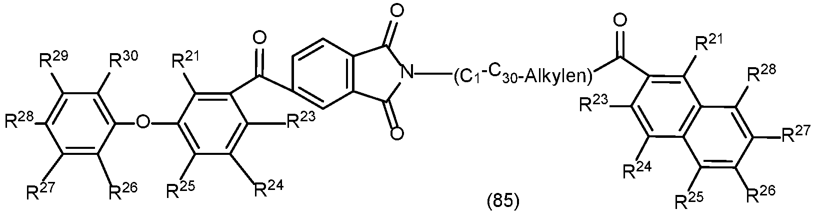 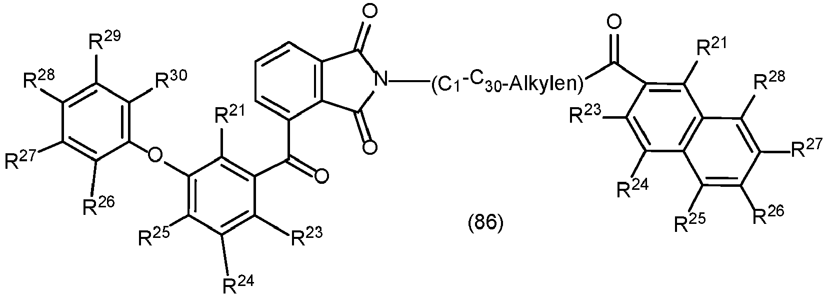 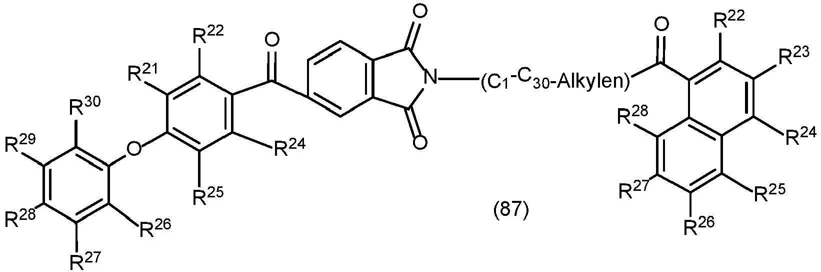 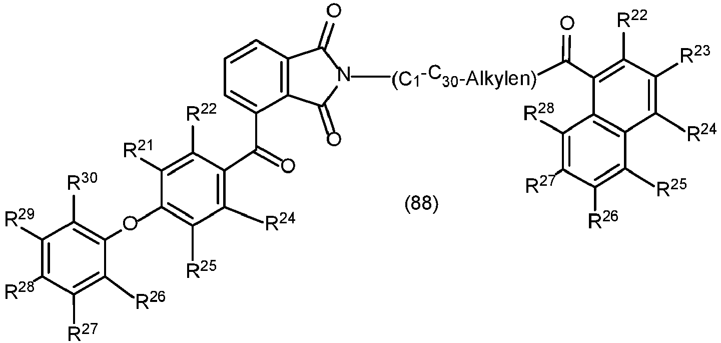 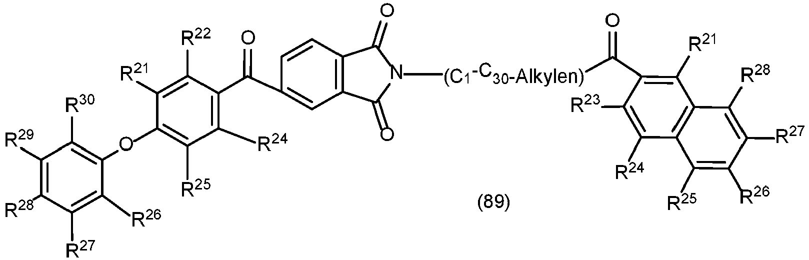 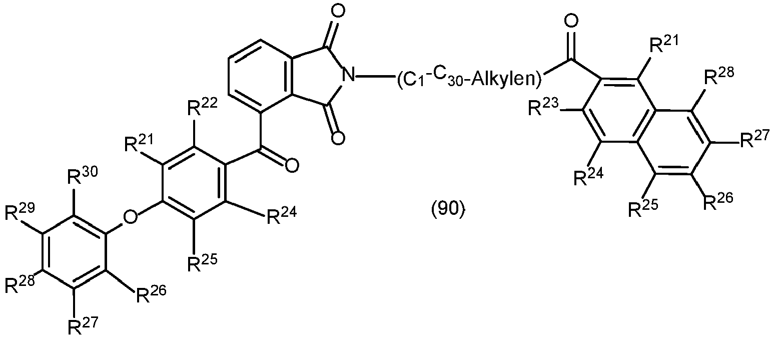 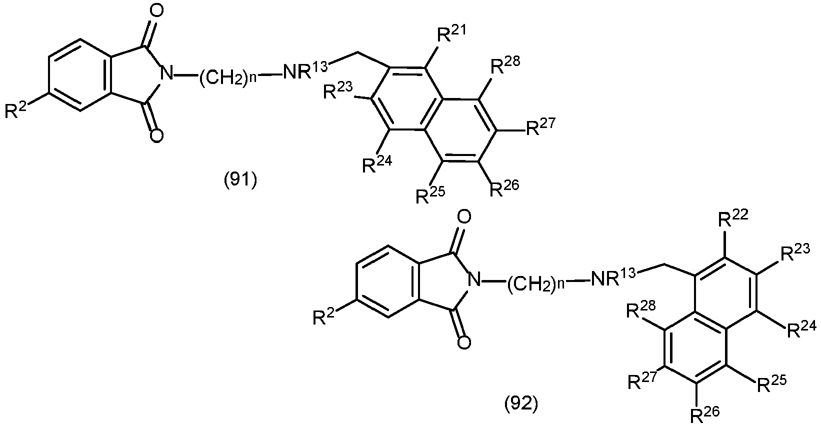 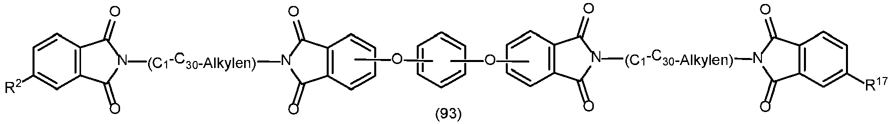 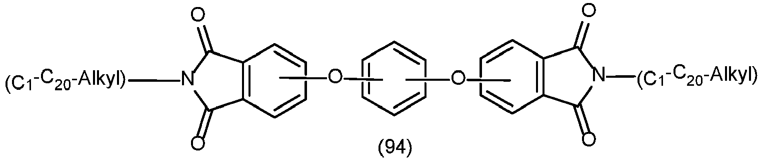 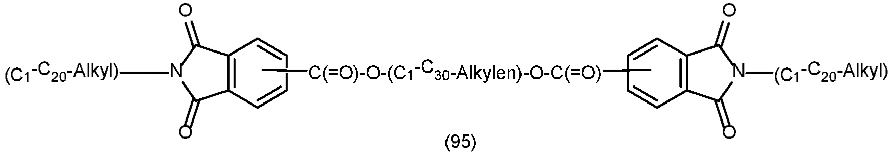 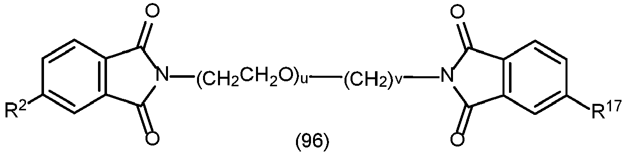 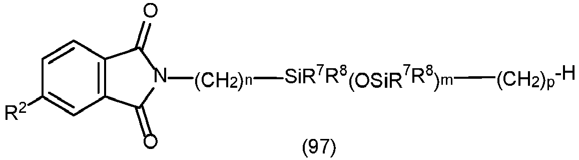 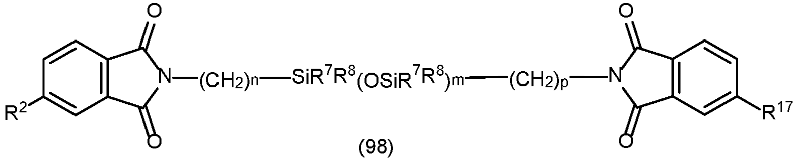 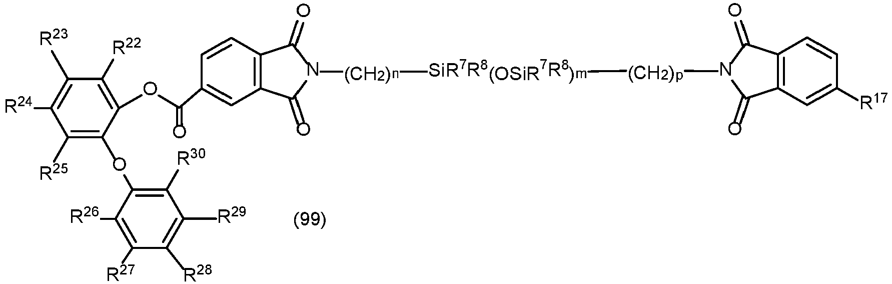 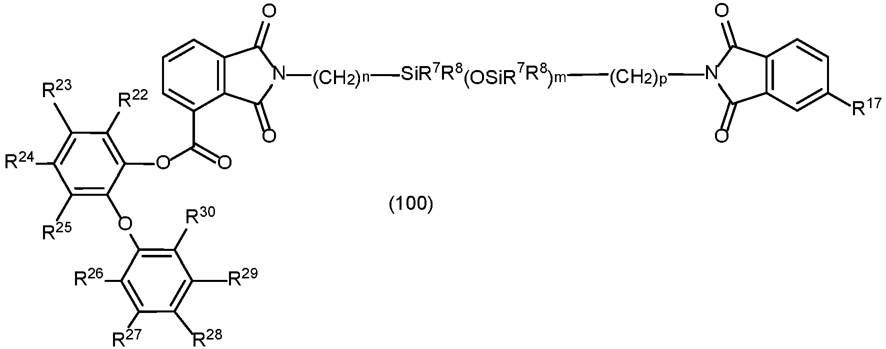 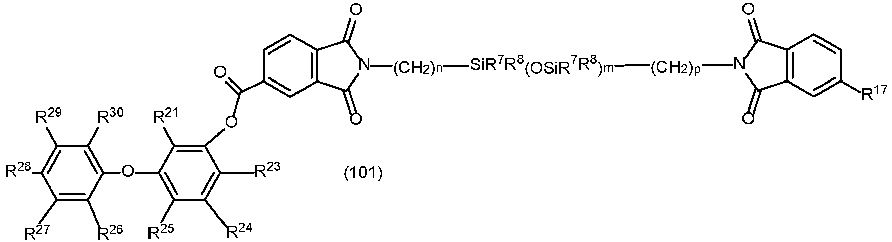 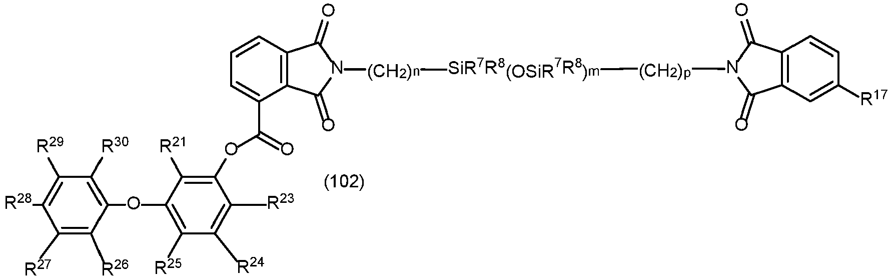 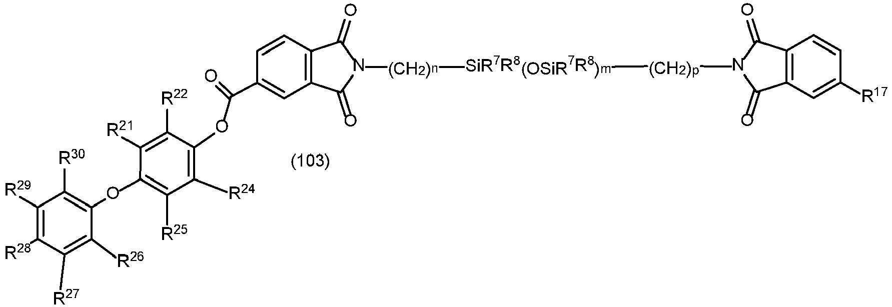 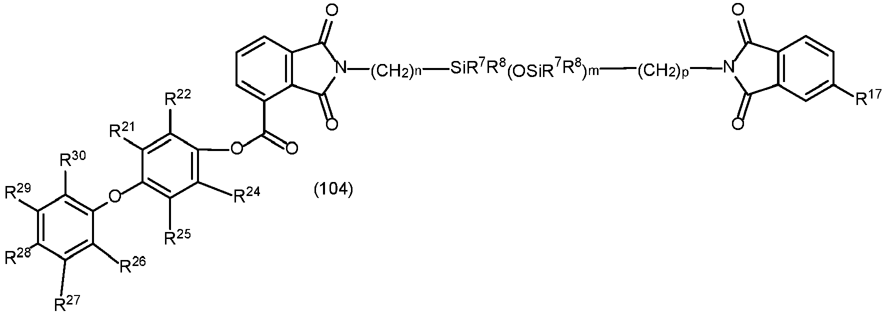 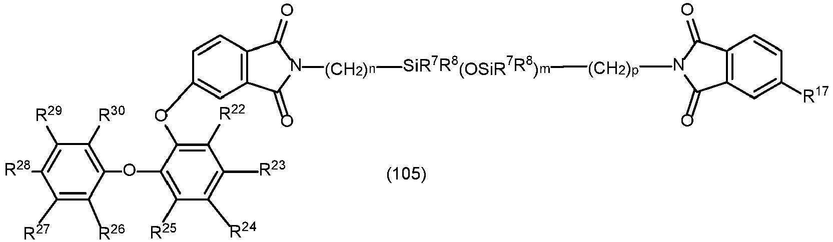 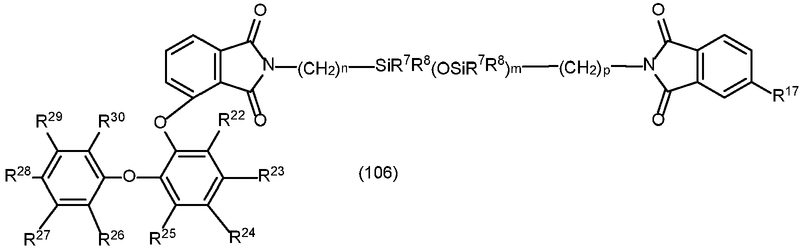 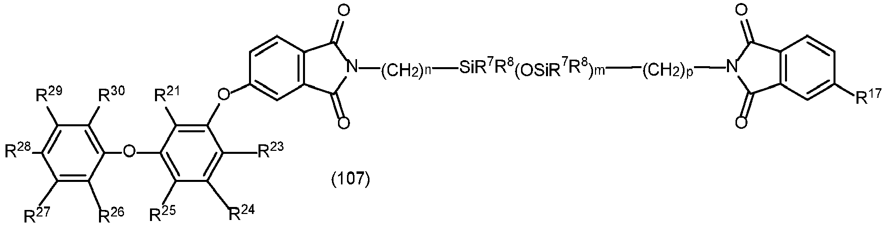 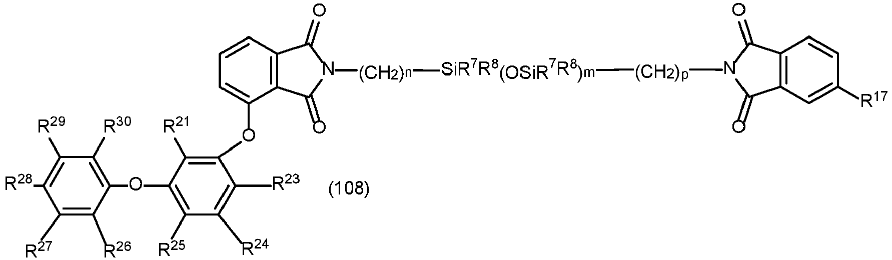 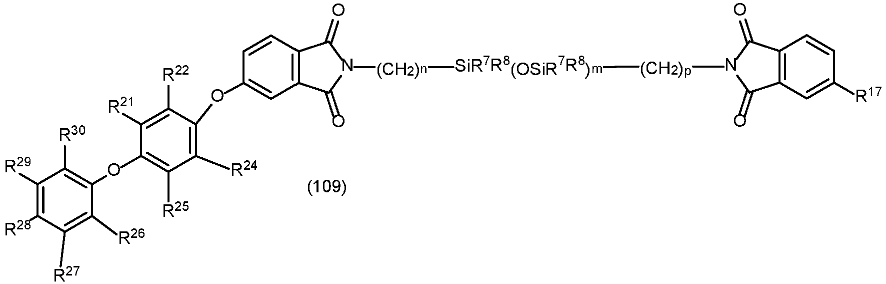 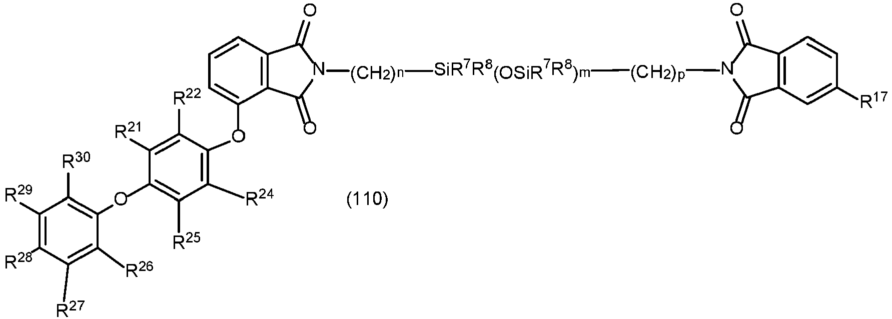 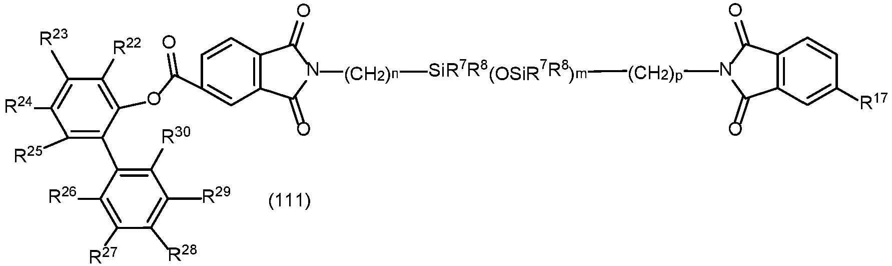 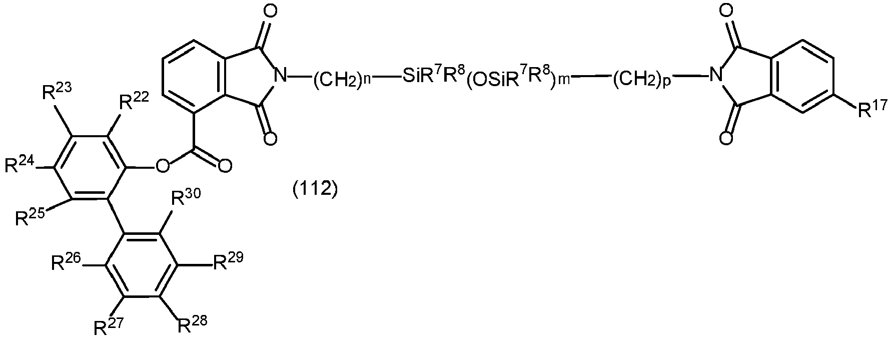 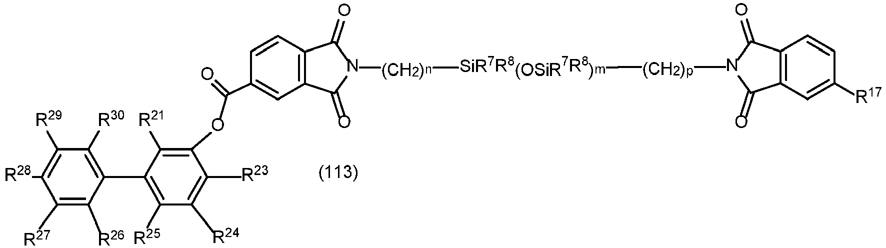 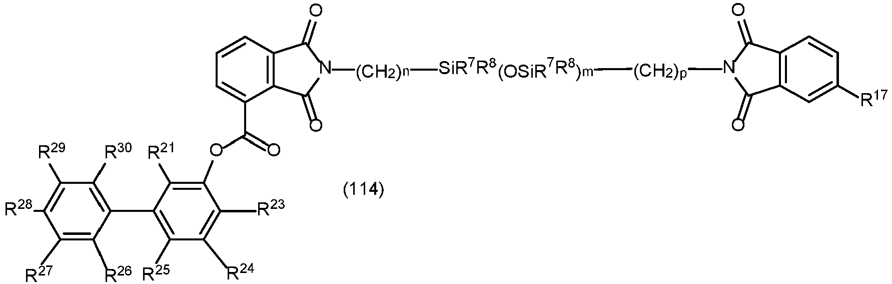 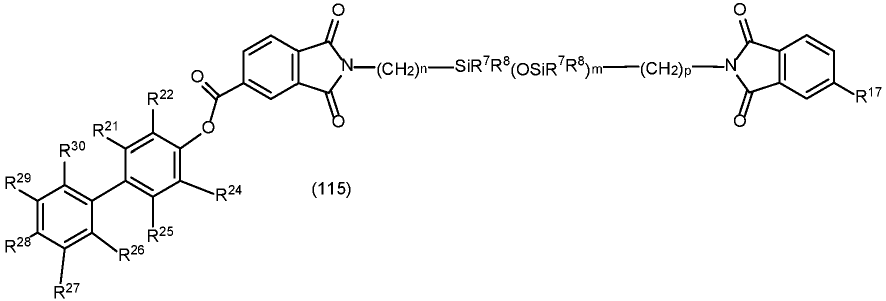 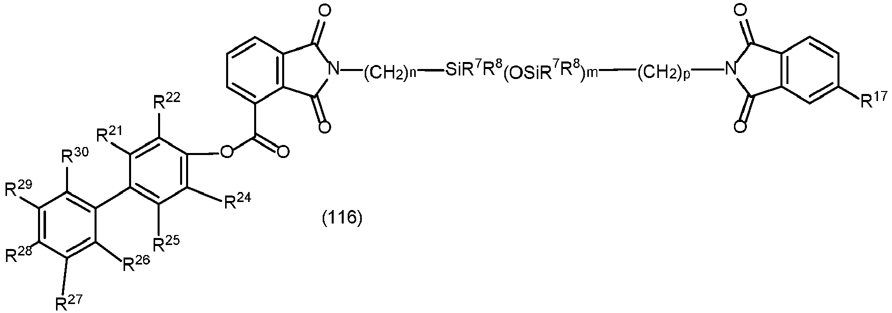 worin
n für eine ganze Zahl von 1 bis 30 steht,
m für eine ganze Zahl von 1 bis 30 steht,
p für eine ganze Zahl von 1 bis 30 steht,
u für eine ganze Zahl von 1 bis 15 steht,
v für 1 oder 2 steht,
R² und R¹⁷ unabhängig voneinander ausgewählt sind unter Wasserstoff, geradkettigem oder verzweigtem Alkyl, geradkettigem oder verzweigtem Alkoxy, geradkettigem oder verzweigtem Alkanoyl, geradkettigem oder verzweigtem Alkyloxycarbonyl, unsubstituiertem oder substituiertem Aryl, unsubstituiertem oder substituiertem Aryloxy, unsubstituiertem oder substituiertem Arylalkylenoxy, unsubstituiertem oder substituiertem Arylthio, unsubstituiertem oder substituiertem Arylalkylenthio, unsubstituiertem oder substituiertem Aroyl, unsubstituiertem oder substituiertem Aryloxycarbonyl, unsubstituiertem oder substituiertem Arylcarbonyloxy, Monoalkylaminocarbonyl, Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Dialkylaminocarbonyl, Diarylaminocarbonyl, Di(arylalkylen)aminocarbonyl, Monoalkanoylamino, Monoaroylamino, Monoalkanoylmonoaroylamino, Dialkanoylamino, Diaroylamino, wobei die Arylreste von Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Diarylaminocarbonyl, Di(arylalkylen)aminocarbonyl, Monoaroylamino, Monoalkanoylmonoaroylamino und Diaroylamino jeweils unsubstituiert oder substituiert sind,
R⁷ und R⁸ unabhängig voneinander für geradkettiges oder verzweigtes C₁-C₄-Alkyl stehen,
R¹³ für Wasserstoff oder C₁-C₄-Alkyl steht,
R^{c} für Wasserstoff oder C₁-C₄-Alkyl steht,
R²¹, R²², R²³, R²⁴, R²⁵, R²⁶, R²⁷, R²⁸, R²⁹ und R³⁰, falls vorhanden, unabhängig voneinander ausgewählt sind unter Wasserstoff, geradkettigem oder verzweigtem C₁-C₂₀-Alkyl und geradkettigem oder verzweigtem C₁-C₄-Alkoxy.

### Herstellung der Phthalimidverbindungen

Die Verbindungen der Formel (I) können prinzipiell nach den im Folgenden beschriebenen Schemata und nach dem Fachmann bekannten Syntheseverfahren der organischen Chemie hergestellt werden. Substituenten, Variablen und Indizes entsprechen den zuvor für die Verbindungen (I) angegebenen Definitionen, sofern im Einzelfall nichts anderes angegeben ist. Konkrete Bedingungen für die Reaktionen können den Ausführungsbeispielen entnommen werden. Konkrete Verbindungen der Formel (I) können nach den dargestellten Schemata durch geeignete Auswahl von Reagenzien mit geeigneter Substitution hergestellt werden. Geeignete Lösungsmittel, Temperaturen, Drücke und andere Reaktionsbedingungen können von einem Fachmann ohne weiteres ausgewählt werden. Die Ausgangsstoffe sind kommerziell erhältlich oder können von einem Fachmann ohne weiteres hergestellt werden.

Schema 1 beschreibt ganz allgemein die Herstellung von Verbindungen (I) durch Kondensation von Phthalsäureanhydrid und Derivaten davon mit primären Aminen. Als Beispiel wird die Umsetzung von Phthalsäureanhydrid und seinen Derivaten mit einem Alkylamin (X¹ = C₁-C₃₀-Alkylen, R^{A} = H) gezeigt. 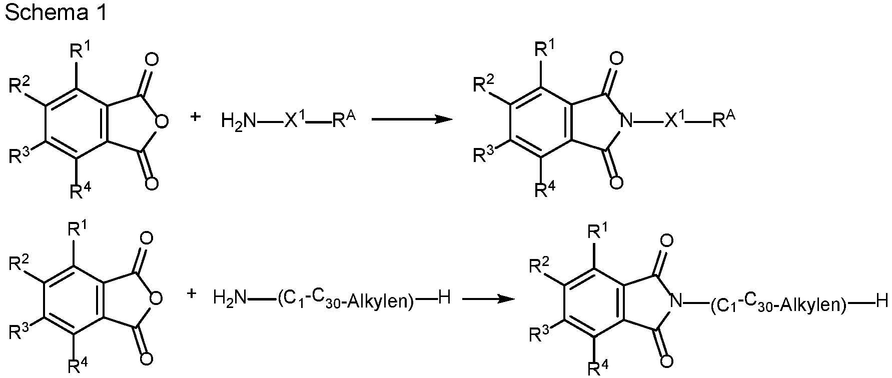 worin R¹, R², R³, R⁴, X¹ und R^{A} die zuvor angegebenen Bedeutungen besitzen.

Mit Diaminen werden in Abhängigkeit von den Molverhältnissen der Produkte Monoaminogruppen-haltige Zwischenprodukte oder Kondensate mit zwei identischen terminalen Phthalimidgruppen erhalten. Monoaminogruppen-haltige Zwischenprodukte können beispielsweise zu Kondensaten mit zwei unterschiedlichen terminalen Phthalimidgruppen umgesetzt werden. Schema 2 zeigt diese Synthesen am Beispiel eines Diaminoalkyl-terminierten Polysiloxans. 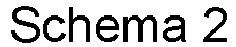 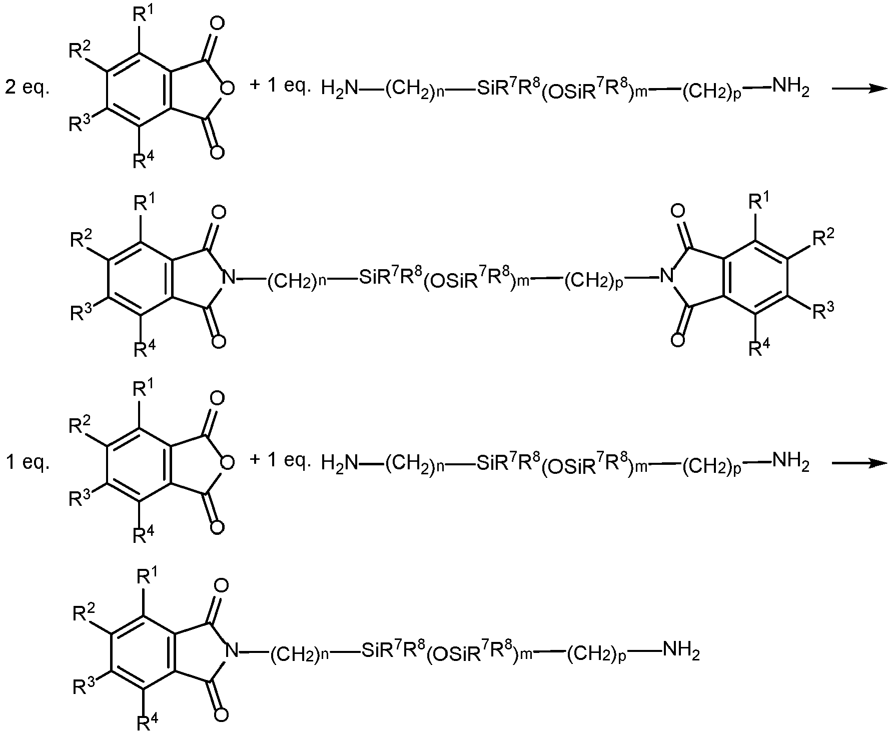 worin R¹, R², R³, R⁴, R⁷, R⁸, X¹, n, m und p die zuvor angegebenen Bedeutungen besitzen.

Alternativ gelingt die Bildung von N-substituierten Phthalimiden durch Reaktion von Kaliumphthalimiden mit entsprechenden Halogenverbindungen, wie im einfachsten Fall mit Alkylhalogeniden, wie in Schema 3 abgebildet. 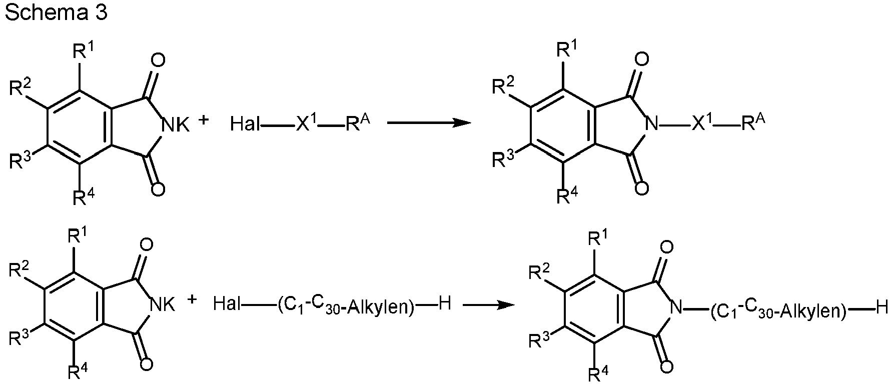 worin Hal für CI, Br oder I steht und R¹, R², R³, R⁴, X¹ und R^{A} die zuvor angegebenen Bedeutungen besitzen.

Die Synthese von Verbindungen der allgemeinen Formel (I) mit bestimmten Substituenten R¹, R², R³ und R⁴ kann ausgehend von entsprechend substituierten Phthalsäureanhydrid-Derivaten erfolgen. Eine Vielzahl dieser Verbindungen ist kommerziell erhältlich. So lassen sich z.B. Verbindungen der Formel (I), worin R² für Methyl steht, ausgehend von 4-Methylphthalsäureanhydrid (CAS-Nummer: 19438-61-0) synthetisieren. Alternativ können entsprechende Phthalsäureanhydrid-Derivate oder entsprechende Phthalimid-Derivate nach gängigen Syntheseverfahren hergestellt werden. Schema 4 zeigt beispielhaft die Synthese von Verbindungen der Formel (I) mit Amidgruppen R³ ausgehend von Trimellitsäureanhydrid (CAS-Nummer: 552-30-7) oder Trimellitsäureanhydrid-chlorid (CAS-Nummer: 1204-28-0). 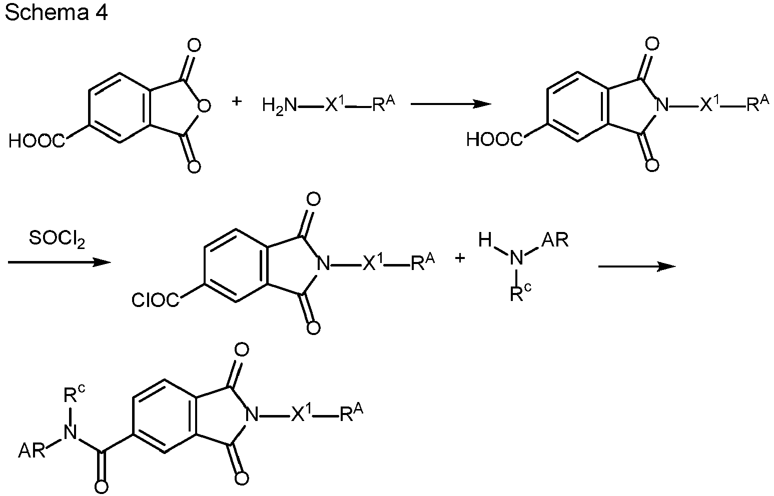 worin X¹ und R^{A} die zuvor angegebenen Bedeutungen besitzen, R^{c} für C₁-C₄-Alkyl steht und AR für einen Arylgruppen-haltigen Rest steht, vorzugsweise ausgewählt unter Gruppen (AR-I) bis (AR-XII), wie zuvor definiert.

Die Synthese von Verbindungen der allgemeinen Formel (I) mit bestimmten Substituenten R^{A} kann ausgehend von entsprechend substituierten Phthalimid-Derivaten erfolgen. Schema 5 zeigt beispielhaft die Synthese von Verbindungen der Formel (I) mit Amidgruppen R^{A} ausgehend von einer Phthalimidverbindung als Zwischenstufe, worin X¹ für C₁-C₃₀-Alkylen steht. 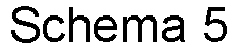 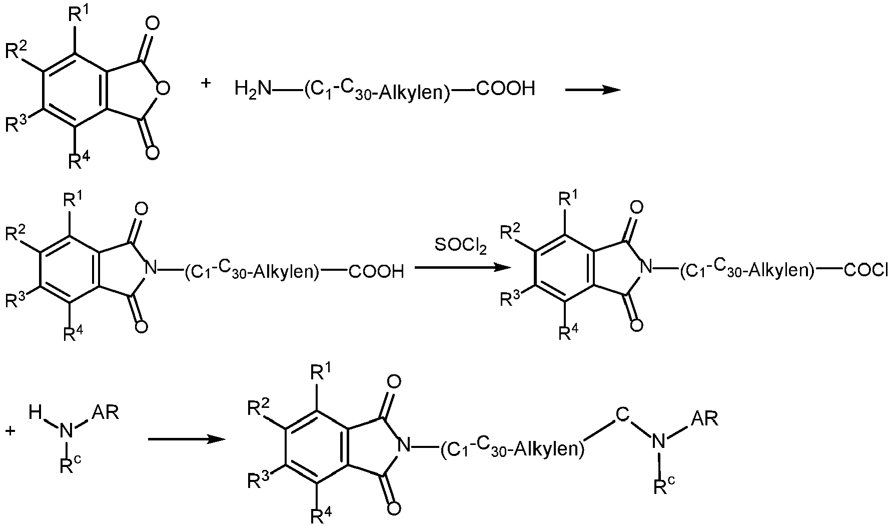 worin R¹, R², R³ und R⁴ die zuvor angegebenen Bedeutungen besitzen, R^{c} für C₁-C₄-Alkyl steht und AR für einen Arylgruppen-haltigen Rest steht, vorzugsweise ausgewählt unter Gruppen (AR-I) bis (AR-XII), wie zuvor definiert.

Zur Hydrierung der nicht-amidischen Carbonylgruppen der Verbindungen (1) - (9), (22) - (27) und (71) - (90) können Hydrosilane, wie Triethylsilan, eingesetzt werden. Die Umsetzung erfolgt bevorzugt in Gegenwart einer starken Säure, wie Trifluoressigsäure. Derartige Umsetzungen sind in A. Winter et al, J. Biol. Chem. (2010), 285, 28883 sowie in WO 2010/107909 und WO 2008/036755 beschrieben. Nicht-amidische Carbonylgruppen liegen nicht in Form einer Amidgruppe (-C(=O)-NH-) oder einer Imidgruppe (-NH-C(=O)-NH-) vor.

### Schmierstoffzusammensetzungen

Ein weiterer Gegenstand der Erfindung ist eine Schmierstoffzusammensetzung, die
a) wenigstens eine Phthalimidverbindung der allgemeinen Formel (I) als Basisöl und
b) wenigstens ein Schmierstoffadditiv
enthält.

Eine spezielle Ausführungsform ist eine Schmierstoffzusammensetzung, enthaltend
a1) wenigstens eine Verbindung der allgemeinen Formel (I) als Basisöl,
a2) gegebenenfalls wenigstens ein von a1) verschiedenes weiteres Basisöl,
b) wenigstens ein Additiv, vorzugsweise ausgewählt unter Verdickern, Korrosionsinhibitoren, Antioxidantien, Metalldeaktivatoren, anorganischen oder organischen Festschmierstoffen, Viskositätsindex-Verbesserern, Verschleißschutzadditiven, Radikalfängern, UV-Stabilisatoren, Pourpoint-Verbesserern, Reibwertveränderern, Additiven zur Verbesserung der Hochdruckeigenschaften, Haftfähigkeitsverbesserern, weiteren oberflächenaktiven Verbindungen und Mischungen davon.

Bevorzugt enthält die erfindungsgemäße Schmierstoffzusammensetzung wenigstens eine Phthalimidverbindung der allgemeinen Formel (I) in einer Menge von 10,0 Gew.-% bis 99,9 Gew.-%, besonders bevorzugt von 20,0 Gew.-% bis 99,0 Gew.-%, noch bevorzugter von 25,0 Gew.-% bis 95,0 Gew.-% und insbesondere von 30,0 Gew.-% bis 90,0 Gew.-%, jeweils bezogen auf das Gesamtgewicht der Schmierstoffzusammensetzung.

Die Schmierstoffzusammensetzung kann zusätzlich zu wenigstens einer Phthalimidverbindung der allgemeinen Formel (I) wenigstens ein weiteres, davon verschiedenes Basisöl enthalten (= Komponente a2).

Wenn die Schmierstoffzusammensetzung wenigstens ein weiteres Basisöl a2) enthält, so ist dieses vorzugsweise ausgewählt unter Estern, Ethern, linearen oder verzweigten Perfluoropolyetherölen (PFPE-Ölen), synthetischen Kohlenwasserstoffen, Mineralölen, unbehandelten und chemisch modifizierten pflanzlichen Öle, Gruppe III Ölen, Gas to Liquid (GTL) Ölen, Reraffinaten und Recyclaten, Polyalphaolefinen (PAO), Silikonölen, Polyisobutenen und Mischungen davon.

Bevorzugt ist das weitere Basisöl a2) ausgewählt unter Estern einer aliphatischen oder aromatischen Di-, Tri- oder Tetracarbonsäure mit einem oder mehreren in Mischung vorliegenden C₇-bis C₂₂-Alkoholen, Estern von Trimethylolpropan, Pentaerythrit oder Dipentaerythrit mit aliphatischen C₇-bis C₂₂-Carbonsäuren, C₁₈-Dimersäureester mit C₇-bis C₂₂-Alkoholen, sowie Komplexestern und Estoliden.

In einer speziellen Ausführungsform enthält die erfindungsgemäße Schmierstoffzusammensetzung als weiteres Basisöl a2) wenigstens einen Ester, ausgewählt unter Pentaerytritestern, Dipentaerythritestern, Trimellitsäureestern, Hemimellitsäureestern, Pyromellitsäureestern, Estoliden, Dimersäureester, Trimersäureester, Trimethylolpropanestern (TMP-Estern), Neopentylglycolestern, Dicarbonsäureestern und Mischungen davon.

Bevorzugt ist das weitere Basisöl a2) ausgewählt unter Ethern, bevorzugt Polyphenylether, Diarylether, Triarylether, Polyglycolen, bevorzugt Homo- und/oder Copolymere von Ethylenoxid, Propylenoxid, 1,2-Butylenoxid und/oder Tetrahydrofuran(THF), vorzugsweise gestartet mit Monoalkoholen, Dialkoholen, Trialkoholen und höherwertigen Alkoholen mit 4, 5 oder mehr alkoholischen OH-Gruppen. Geeignete höherwertige Alkohole sind z.B. Pentaerytritol und Dipentaerythritol.

In einer speziellen Ausführungsform enthält die erfindungsgemäße Schmierstoffzusammensetzung als weiteres Basisöl a2) wenigstens einen Ether, ausgewählt unter Polyalkylenglycolen (PAG). Geeignete Poly(alkylen)glykole a2) (PAG) sind Homopolymere, Copolymere und Mischungen (Blends) davon. Im Sinne der Erfindung bezeichnet der Begriff Copolymer auch Polymere, die aus drei, vier oder mehr verschiedenen Monomeren aufgebaut sind (Terpolymere, Quaterpolymere usw.). Geeignete Poly(alkylen)glykole a2) sind Polyethylenglykole, Polypropylenglykole, Polybutylenglykole, Polytetrahydrofurane und Copolymere aus zwei oder mehreren verschiedenen Alkylenoxid-Copolymeren. Geeignete Alkylenoxide zur Herstellung von Poly(alkylen)glykolen a2) sind z. B. Ethylenoxid, Propylenoxid, Epichlorhydrin, 1,2- und 2,3-Butylenoxid. Geeignete Beispiele für Copolymere sind Copolymere von Ethylenoxid und Propylenoxid, Copolymere von Ethylenoxid und Butylenoxid und Copolymere von Ethylenoxid, Propylenoxid und mindestens einem Butylenoxid. Die Alkylenoxid-Copolymere können die copolymerisierten Alkylenoxid-Einheiten in zufällig verteilter Form oder in Form von Blöcken enthalten.

Als weitere Basisöle a2) eignen sich weiterhin vorteilhaft lineare oder verzweigte Perfluoropolyetherölen (PFPE-Ölen). Geeignet sind z.B. Perfluorpolyether (PFPE) der Formel:

R^{A}-(O-CF₂)ᵥ-(O-C₂F₄)_{w}-(O-C₃F₆)ₓ-(O-CFCF₃)_{y}-(O-CF₂CF(CF₃))_{z}-O-R^{B}

wobei R^{A} und R^{B} identisch oder verschieden sind und ausgewählt sind unter -CF₃, -C₂F₅ und -C₃F₇, und v, w, x, y, z ganze Zahlen von ≥ 0 bis 500 sind. PFPE-Öle werden beispielsweise unter dem Markennamen Aflunox^{®}, Krytox^{®}, Fomblin^{®} und Demnum^{®} vertrieben.

Als weitere Basisöle a2) eignen sich weiterhin vorteilhaft synthetischen Kohlenwasserstoffen, Mineralöle und Mischungen davon.

Als weitere Basisöle a2) eignen sich weiterhin vorteilhaft Gruppe III Öle. Gruppe III Öle (hydrogecrackte synthetische Öle) sind synthetische Kohlenwasserstoffe aus Petroleum-Basisölen, die vollständig durch Hydrocracken, Hydroisomerisierung und Hydrodesulfurierung hergestellt werden. Sie weisen mindestens 90 Prozent gesättigte Verbindungen und maximal 0,03 Prozent Schwefel sowie einen Viskositätsindex mindestens 120 auf.

Als weitere Basisöle a2) eignen sich weiterhin vorteilhaft GTL Öle (Gas to liquids Öle). GTL Öle basieren auf Synthesegas aus Wasserstoff und Kohlenmonoxid, aus dem mittels Fischer-Tropsch-Synthese längerkettige Kohlenwasserstoffe hergestellt werden.

Als weitere Basisöle a2) eignen sich weiterhin vorteilhaft Polyalphaolefine (PAO). Polyalphaolefine werden auch als Gruppe IV Öle bezeichnet und haben in der Regel einen Viskositätsindex im Bereich von 125 bis 200. Polyalphaolefine können nach bekannten Verfahren katalytisch aus Ethylen hergestellt werden, wobei als Zwischenprodukt zunächst Alphaolefine mit größerer Kettenlänge resultieren. Hieraus werden die Polyalphaolefine im Wesentlichen durch Oligomerisierung synthetisiert, wobei in der Regel Isoparaffine mit einer unterschiedlichen Anzahl von gleichlangen Seitenketten entstehen. Die Synthese kann durch säurekatalysierte (konventionelle) oder Metallocen-katalysierte Olefinpolymerisation (mPAO) erfolgen. Konventionelle PAO weisen einen hohen Isomerisierungsgrad auf, wohingegen bei der Metallocen-Oligomerisierung Produkte resultieren, die im Wesentlichen frei von Isomerisierung sind. Geeignete Polyalphaolefine sind z.B. die Oligomere, vorzugsweise die Dimere, Trimere, Tetramere, Pentamere und höheren Oligomere mit mehr als 5 Wiederholungseinheiten von Alphaolefinen, und Gemische dieser Oligomere. Die zur Herstellung der Polyalphaolefine eingesetzten Alphaolefine sind vorzugsweise ausgewählt unter C₈-C₁₄-Alphaolefinen, insbesondere 1-Octen, 1-Decen, 1-Dodecen und Mischungen davon.

Als weitere Basisöle a2) eignen sich weiterhin vorteilhaft Silikonöle. Bevorzugt werden als weitere Basisöle a2) Dimethylsilikonöle eingesetzt.

In einer speziellen Ausführungsform der Erfindung ist das weitere Basisöl a2) ein fluorfreies Basisöl, vorzugsweise ausgewählt unter Pentaerythritestern, Dipentaerythritestern, Trimellitsäureestern, Hemimellitsäureestern, Pyromellitsäureestern, Estoliden, Dimersäureestern, Trimersäureestern, Dicarbonsäureestern Diarylethern, Polyglycolen, synthetischen Kohlenwasserstoffen, konventionellen Polyalphaolefinen (PAO), Metallocen-katalysierten Polyalphaolefinen (mPAO); Mineralölen, unbehandelte und chemisch modifizierte pflanzliche Öle, Gruppe III Ölen, Dimethylsilikonölen und Mischungen. Bevorzugte Ether sind Homo- und/oder Copolymere von Ethylenoxid, Propylenoxid, 1,2-Butylenoxid und/oder Tetrahydrofuran(THF), vorzugsweise gestartet mit Monoalkoholen, Dialkoholen und Trialkoholen. Bevorzugt synthetische Kohlenwasserstoffe sind alkylierte Naphthaline.

In einer weiteren bevorzugten Ausführungsform weist das Basisöl oder Basisölgemisch (d.h. Phthalimidverbindungen (I) und, falls vorhanden, wenigstens ein weiteres Basisöl a2) eine kinematische Viskosität, bestimmt nach ASTM-D-7042, Ausgabe September 2014, bei 40°C von 30 mm²/s bis 6000 mm²/s, noch bevorzugter bei 40°C von 50 mm²/s bis 1200 mm²/s, insbesondere bei 40°C von 50 mm²/s bis 800 mm²/s auf.

In einer weiteren bevorzugten Ausführungsform der Erfindung ist das Basisöl ausgewählt aus der Gruppe bestehend aus Estern, bevorzugt Dipentaerythritester, Trimellithsäureester, Hemimellithsäureester, Pyromellitsäureester, Estoliden, Pentaerytritester, Dimersäureester, Trimersäureester, TMP-Ester, Dicarbonsäureester, jeweils mit einer kinematischen Viskosität bei 40°C bestimmt nach ASTM-D-7042, Ausgabe September 2014 von 100 mm²/s bis 1200 mm²/s; Ethern, bevorzugt Polyphenylether, Diarylether, Triarylether, linearen oder verzweigten Perfluorpolyetherölen, jeweils mit einer kinematischen Viskosität bei 40°C bestimmt nach ASTM-D-7042-Ausgabe September 2014 von 20 mm²/s bis 1200 mm²/s; Polyglycolen, bevorzugt Homo- und/oder Copolymere von Ethylenoxid, Propylenoxid, 1,2-Butylenoxid und/oder Tetrahydrofuran(THF), vorzugsweise gestartet mit Monoalkoholen, Dialkoholen und Trialkoholen, jeweils mit einer kinematischen Viskosität bei 40°C bestimmt nach ASTM-D-7042-Ausgabe September 2014 von 20 mm²/s bis 46000 mm²/s; synthetischen Kohlenwasserstoffen, bevorzugt alkylierte Naphthaline, Polyalphaolefine (PAOs), metallocene Polyalphaolefine (mPAOs), jeweils mit einer kinematischen Viskosität bei 40°C bestimmt nach ASTM-D-7042-Ausgabe September 2014 von 10 mm²/s bis 20000 mm²/s; Gruppe III Ölen mit einer kinematischen Viskosität bei 40°C bestimmt nach ASTM-D-7042-Ausgabe September 2014 von 10 mm²/s bis 100 mm²/s, Dimethylsiliconölen, arylierten Silikonölen bevorzugt Alkylarylsilikonöle, insbesondere Methyl/Aryl-Silikonöle und vollständig arylierte Silikonöle, jeweils mit einer kinematischen Viskosität bei 40°C bestimmt nach ASTM-D-7042-Ausgabe September 2014 von 10 mm²/s bis 1200 mm²/s und/oder jeweils mit einer kinematischen Viskosität bei 25°C bestimmt nach DIN 53019, Ausgabe 2008.09 von 20 bis 2000000 mm²/s, die einzeln oder in Kombination verwendet werden können.

### Schmieröle und Schmierfette

Die erfindungsgemäßen Schmierstoffzusammensetzungen können sowohl als Schmieröl als auch als Schmierfett vorliegen. Schmierfette enthalten im Gegensatz zu Schmierölen wenigstens einen Verdicker als Additiv. Verdicker sind feste, in den Grundölen nahezu oder völlig unlösliche Substanzen (unlösliche Feststoffe), die verdickend wirken. Im Sinne dieser Erfindung werden alle im Basisöl unlöslichen Feststoffe als Verdicker angesehen, unabhängig davon, ob Sie einen Einfluss auf Reibung und Verschleiß haben. Eine spezielle Ausführungsform sind anorganische und organische Festschmierstoffe.

Schmierfette sind Zusammensetzungen, die eine (bei Raumtemperatur) flüssige Phase und Feststoffe enthalten. Die Konsistenz von Schmierfetten lässt sich anhand eines standardisierten Messverfahrens in Form der sogenannten Walkpenetration nach DIN ISO 2137 ermitteln. Die Walkpenetration wird nach dem Walken des Schmierfetts in einem Fettkneter bzw. Schmierfettwalker mit Hilfe eines Penetrometers gemessen als die die Eindringtiefe eines Standardkonus unter definierten Bedingungen. Die Zuordnung der gemessenen Konuspenetration zu einer definierten NLGI-Klasse (NLGI = National Lubricating Grease Institute) erfolgt dann nach DIN 51818. Erfindungsgemäße Schmierfette weisen bei der Messung nach DIN 51818 speziell eine Walkpenetration von 85 - 475 Konuspenetration in 0,1 mm auf.

Weist eine in den erfindungsgemäßen Schmierstoffzusammensetzungen als Additiv eingesetzte Komponente mehrere Wirkungen auf, z.B. als Verdicker und Reibwertveränderer und wird sie in dieser Anmeldung entsprechend auch als Additiv für mehrere Zwecke genannt, so wird sie mengenmäßig jeder dieser Additivkomponenten voll zugerechnet.

Eine erste spezielle Ausführungsform der Erfindung ist eine Schmierstoffzusammensetzung in Form eines Schmieröls, enthaltend
a) 80 bis 99,9 Gew.-%, bezogen auf das Gesamtgewicht der Schmierstoffzusammensetzung wenigstens eine Verbindung der allgemeinen Formel (I), wie zuvor definiert,
b) 0,1 bis 20 Gew.-%, bezogen auf das Gesamtgewicht der Schmierstoffzusammensetzung, wenigstens ein Additiv.

Bevorzugt enthält die Schmierstoffzusammensetzung in Form eines Schmieröls 90 bis 99,8 Gew.-% wenigstens einer Verbindung der allgemeinen Formel (I) (Komponente a).

Bevorzugt enthält die Schmierstoffzusammensetzung in Form eines Schmieröls 0,2 bis 10 Gew.-% wenigstens eines Additivs (Komponente b).

In einer speziellen Ausführungsform enthält die Schmierstoffzusammensetzung in Form eines Schmieröls wenigstens ein Basisöl a2) in einer Menge von bis zu 50 Gew.-%, bevorzugt von bis zu 30 Gew.-%, insbesondere von bis zu 15 Gew.-%, bezogen auf das Gesamtgewicht der Schmierstoffzusammensetzung.

Eine zweite spezielle Ausführungsform der Erfindung ist eine Schmierstoffzusammensetzung in Form eines Schmierfetts, enthaltend
a1) wenigstens eine Verbindung der allgemeinen Formel (I) als Basisöl,
a2) gegebenenfalls wenigstens ein von a1) verschiedenes weiteres Basisöl,
b1) wenigstens einen Verdicker,
b2) gegebenenfalls wenigstens ein weiteres Additiv.

In einer speziellen Ausführungsform enthält die Schmierstoffzusammensetzung in Form eines Schmierfetts wenigstens ein Basisöl, ausgewählt unter Basisölen a1) und a2), in einer Menge von 40,0 bis 95,0 Gew.-%, bevorzugt von 50,0 bis 90 Gew.-%, bezogen auf das Gesamtgewicht der Schmierstoffzusammensetzung.

### Verdicker b1)

Bevorzugt enthält die Schmierstoffzusammensetzung in Form eines Schmierfetts wenigstens einen Verdicker b1) in einer Menge von 0,2 bis bis 60,0 Gew.-%, besonders bevorzugt von 3,0 bis 55 Gew.-%, insbesondere von 5,0 bis 50 Gew.-%, bezogen auf das Gesamtgewicht der Schmierstoffzusammensetzung.

Vorzugsweise ist der Verdicker b1) ausgewählt unter Metall-Einfachseifen, Metall-Komplexseifen Harnstoffverdickern, Metallsulfonatverdickern, Wachsen, Bornitrid, Ruß, Graphit, Metallchalcogeniden, hochfluorierten polymeren Substanzen und fluorfreien Substanzen. Dabei sind die fluorfreien Substanzen vorzugsweise ausgewählt unter fluorfreien Polymeren, die aromatische, heteroaromatische und/oder heterocyclische Gruppen aufweisen und einen Schmelz- oder Zersetzungspunkt, gemessen nach DIN EN ISO 11357-1, Ausgabe 2008.04, von höher als 200°C aufweisen, fluorfreien Phthalocyaninen, Silikonharzen, Ligninen, anorganischen Schichtsilikaten, die mit organischen Gruppen funktionalisiert sein können, Phosphorverbindungen, Melaminderivaten und Mischungen von zwei oder mehreren dieser Verdicker.

Als Verdicker b1) geeignete Metall-Einfachseifen sind vorzugsweise Metall-Einfachseifen der Elemente der ersten und zweiten Hauptgruppe des Periodensystems, insbesondere Lithiumseifen. Bevorzugt leiten sich die Metall-Einfachseifen von mindestens einer gesättigten oder ungesättigten C₈-₂₂-, vorzugsweise mindestens einer C₁₄-₂₀-Fettsäure oder einem Derivat davon ab. Dazu zählt z.B. hydriertes Rizinusöl, d.h. das Glycerid der 12-Hydroxystearinsäure. In einer bevorzugten Ausführungsform umfasst der Verdicker b1) wenigstens eine Lithiumseife. Besonders bevorzugte Lithiumseifen sind Lithiumsalze von Stearinsäure.

Als Verdicker b1) geeignete Metall-Komplexseifen sind vorzugsweise Metall-Komplexseifen der Elemente der ersten und zweiten Hauptgruppe des Periodensystems, wie Lithiumkomplexseifen, Natriumkomplexseifen, Calciumkomplexseifen und Aluminiumkomplexseifen. Bevorzugt sind Lithiumkomplexseifen.

Geeignete Metallkomplexseifen können erhalten werden durch Reaktion einer Metallbase (wie ein Hydroxid, Oxid oder Carbonat) mit
b11) mindestens einer Carbonsäurekomponente mit 10 bis 32 Kohlenstoffatomen, vorzugsweise ausgewählt aus ungesättigten oder gesättigten C₁₀-C₃₂-Monocarbonsäuren oder ungesättigten oder gesättigten C₁₀-C₃₂-Hydroxy-Monocarbonsäuren und Derivaten der genannten C₁₀-C₃₂-Monocarbonsäuren und C₁₀-C₃₂-HydroxyMonocarbonsäuren und
b12) mindestens ein Komplexierungsmittel.

Die Komponente b11) ist vorzugsweise ausgewählt aus gesättigten oder ungesättigten C₁₂-C₂₂-Monocarbonsäuren, gesättigten oder ungesättigten C₁₂-C₂₂-Hydroxymonocarbonsäuren und deren Ester und Mischungen. Insbesondere sind die Monocarbonsäuren und Hydroxymonocarbonsäuren b11) ausgewählt aus Laurinsäure, Myristinsäure, Palmitinsäure, Stearinsäure, Arachidinsäure, Myristoleinsäure, Palmitoleinsäure, Ölsäure, Linolsäure, Arachidonsäure, Behensäure, 12-Hydroxystearinsäure, 17-Hydroxystearinsäure, 2-Hydroxytetradecansäure, 3-Hydroxytetradecansäure, 2-Hydroxyhexadecansäure, 3-Hydroxyhexadecansäure und deren Mischungen.

Das Komplexierungsmittel b12) ist vorzugsweise ausgewählt aus gesättigten oder ungesättigten C₂-C₁₆-Dicarbonsäuren, gesättigten oder ungesättigten C₂-C₈-Hydroxy-Monocarbonsäuren, Derivaten und Gemischen davon. Als Dicarbonsäuren b2) eignen sich insbesondere Adipinsäure, Sebacinsäure, Azelainsäure, 3-tert.-Butyladipinsäure sowie deren Ester und Gemische. Besonders geeignet als Komponente b2) sind auch Hydroxybenzoesäuren, z. B. Salicylsäure, 3-Hydroxybenzoesäure, 4-Hydroxybenzoesäure, 2-Hydroxy-4-hexylbenzoesäure, 2,5-Dihydroxybenzoesäure, 2,6-Dihydroxybenzoesäure, 4-Hydroxy-4-methoxybenzoesäure und deren Mischungen.

Ganz besonders bevorzugt weist die Lithiumkomplexseife Lithiumsalze von 12-Hydroxystearinsäure kombiniert mit Azelainsäure oder Sebacinsäure, insbesondere 12-Hydroxystearinsäure kombiniert mit Azelainsäure auf.

In einer bevorzugten Ausführungsform ist der Verdicker b1) ein Harnstoffverdicker, insbesondere ein alkylierter und/oder arylierter (Oligo)Harnstoff. Bevorzugte Harnstoffverdicker sind die Reaktionsprodukte aus wenigstens einem Diisocyanat mit wenigstens einen Amin, ausgewählt unter Monoaminen, Polyaminen und Mischungen davon. Bevorzugt ist das Diisocyanat ausgewählt unter 2,4-Diisocyanatotoluol, 2,6-Diisocyanatotoluol, 4,4'-Diisocyanatodiphenylmethan, 2,4'-Diisocyanatophenylmethan, 4,4'-Diisocyanatodiphenyl, 4,4'-Diisocyanato-3-3'-dimethylphenyl, 4,4'-Diisocyanato-3,3'-dimethylphenylmethan und Mischungen davon.

Vorzugsweise ist das Amin ausgewählt aus Monoaminen der Formel R^{a}₂N-R^{b}, Diaminen der Formel R^{a}₂N-R^{c}-NR^{a}₂ und Mischungen davon, wobei
- R^{a}: unabhängig ausgewählt ist aus Wasserstoff, linearem oder verzweigtem C₁-C₂₂-Alkyl und C₆-C₁₄-Aryl,
- R^{b}: unabhängig ausgewählt ist aus linearen oder verzweigten C₁-C₂₂-Alkyl- und C₆-C₁₄-Arylresten,
- R^{c}: eine zweiwertige verbrückende Gruppe ist, die vorzugsweise aus C₁-C₂₂-Alkylen und C₆-C₁₄-Arylen ausgewählt ist.

Als Verdicker b1) geeignete Metallsulfonatverdicker sind insbesondere Calciumsulfonatverdickern. Calciumsulfonatverdicker enthalten speziell kristallines Calciumcarbonat in Form von Calcit und Calciumsalze von Säuren, insbesondere aromatischen Sulfonsäuren, ganz besonders bevorzugt Alkylbenzolsulfonsäuren, Carbonsäuren insbesondere Stearinsäure, 12-Hydroxystearinsäure, Essigsäure, Borsäure und deren Gemische.

Als Verdicker b1) geeignete Wachse sind insbesondere Polyethylen (PE)-, Polypropylen (PP)- und Polyamid (PA)-Wachs. Vorzugsweise weist das Wachs einen Schmelz- oder Zersetzungspunkt, gemessen nach DIN EN ISO 11357-1 Ausgabe 2008.04 von höher als 100°C aufweist.

Als Verdicker b1) geeignete Metallchalcogeniden sind insbesondere Molybdändisulfid, Wolframdisulfid und Metallselenide.

Als Verdicker b1) geeignete hochfluorierten polymeren Substanzen sind insbesondere PTFE. Dies ist jedoch keine bevorzugte Ausführungsform der Erfindung

Als Verdicker b1) eignen sich weiterhin fluorfreie Komponenten, die sich von den zuvor genannten Verdickern b1) unterscheiden. Bevorzugt weisen die fluorfreien Komponenten eine mittlere Partikelgröße (D50) von unter 80 µm, beispielsweise 1 µm bis 80 µm, noch bevorzugter von 1 µm bis 50 µm, noch bevorzugter von 1 µm bis 20 µm, insbesondere von 1 µm bis 15 µm, jeweils gemessen nach ISO 13320-1, Ausgabe 2020-01 auf. Bevorzugte weitere fluorfreie Komponenten sind Bornitrid, Ruß, Graphit, Graphen, Zinn (IV) sulfid, Zinc (II) sulfid, Wolframsulfid. Bevorzugte weitere fluorfreie Komponenten sind Talkum, Bentonit, Glimmer und pyrogenes Siliziumdioxid, das mit organischen Gruppen funktionalisiert sein kann. Eine spezielle Ausführung der Bentonite sind Montmorillonite, deren Natriumionen teilweise oder vollständig gegen Ammoniumionen ausgetauscht sein können. Als weitere fluorfreie Verdicker b1) eignen sich Aluminosilicate, Aluminiumoxid und Kieselsäure (z.B. Aerosil). Als weitere fluorfreie Verdicker b1) eignen sich nanopartikuläres Siliziumdioxid, das mit organischen Gruppen funktionalisiert ist, Zinkpyrophosphat, Calcium(pyro)phosphat und Zirkonhydrogenphosphat. Als weitere fluorfreie Verdicker b1) eignen sich Melaminderivate, ausgewählt unter Melamincyanurat, Melaminphosphat und 1,3,5-Triazin-2,4,6 (1H,3H,5H)-trithion.

In einer weiteren bevorzugten Ausführungsform ist der Verdicker b1) eine Kombination aus zwei oder mehr der vorgenannten weiteren Verdicker.

### Weitere Additive:

Die erfindungsgemäße Schmierstoffzusammensetzung kann wenigstens ein von den Verdickern b1) verschiedenes Additiv b2) aufweisen. Die Additive sind vorzugsweise ausgewählt unter Korrosionsinhibitoren, Antioxidantien, Metalldeaktivatoren, anorganischen oder organischen Festschmierstoffen, Viskositätsindex-Verbesserern, Verschleißschutzadditiven, Radikalfängern, UV-Stabilisatoren, Pourpoint-Verbesserern, Reibwertveränderern, Additiven zur Verbesserung der Hochdruckeigenschaften, Haftfähigkeitsverbesserern, weiteren oberflächenaktiven Verbindungen und Mischungen davon.

Falls vorhanden, enthält die Schmierstoffzusammensetzung jedes der Additive bevorzugt in einer Menge von 0,1 bis 20 Gew.-%, besonders bevorzugt 0,5 bis 10 Gew.-%, noch bevorzugter von 1 bis 8 Gew.-%, und insbesondere von 1 bis 5 Gew.-%, jeweils bezogen auf das Gesamtgewicht der Schmierstoffzusammensetzung.

### Korrosionsschutzmittel

Die erfindungsgemäße Schmierstoffzusammensetzung kann wenigstens ein Korrosionsschutzmittel als Additiv enthalten.

Korrosionsinhibitoren neutralisieren saure Reaktionsprodukte, z.B. aus der Oxidation des Basisöls oder einem Additivabbau. So wird ein korrosiver Angriff abgeschwächt oder verhindert. Geeignete Korrosionsinhibitoren sind die Salze verschiedener Säuren, wie zum Beispiel Sulfonate, Naphthenate, Carboxylate, Aminphosphate, Bernsteinsäurehalbester oder partielle Polyolester. Der Korrosionsinhibitor ist vorzugsweise ausgewählt unter Calciumsulfonaten, bevorzugt "overbased" Calcium Sulfonaten mit einer Basenzahl (TBN) von 100 bis 500 mg KOH/g, mit Aminen (teil)neutralisierten Phosphaten, alkylierten Ca-Naphthalinsulfonaten, Oxazolin-Derivaten, Imidazol-Derivaten, Bernsteinsäurehalbestern, Benzotriazol, N-alkylierten Benzotriazolen und Mischungen davon.

Bevorzugt enthält die erfindungsgemäße Schmierstoffzusammensetzung wenigstens ein Korrosionsschutzmittel in einer Menge von wenigstens 0,1 Gew.-%, besonders bevorzugt wenigstens 0,5 Gew.-%, insbesondere wenigstens 1,0 Gew.-%, bezogen auf das Gesamtgewicht der Schmierstoffzusammensetzung.

### Antioxidantien

Die erfindungsgemäße Schmierstoffzusammensetzung kann wenigstens ein Antioxidans als Additiv enthalten.

Antioxidantien führen zu einer Steigerung der Widerstandsfähigkeit gegen Alterung durch oxidative und/oder thermische Belastungen an der Schmierstelle. Durch Oxidation können Säuren und ölunlösliche Bestandteile entstehen, die Verunreinigungen bilden, welche sich auf der Schmierstelle absetzen können.

Geeignete Antioxidationsmittel sind aromatische aminische Antioxidantien, wie alkyliertes Phenyl-alpha-Naphthylamin, Dialkyldiphenylamin, aralkylatiertes Diphenylamin, sterisch gehinderte Phenolen, wie Butylhydroxytoluol (BHT), Bis-2,6-di-t-butylphenolderivate, Schwefel enthaltende gehinderte Phenole, Schwefel enthaltendes gehindertes Bisphenol und Mischungen davon.

Bevorzugt enthält die erfindungsgemäße Schmierstoffzusammensetzung wenigstens ein Antioxidationsmittel in einer Menge von wenigstens 0,1 Gew.-%, besonders bevorzugt wenigstens 0,5 Gew.-%, insbesondere wenigstens 1,0 Gew.-%, bezogen auf das Gesamtgewicht der Schmierstoffzusammensetzung.

### Metalldeaktivatoren

Die erfindungsgemäße Schmierstoffzusammensetzung kann wenigstens einen Metalldesaktivator (Additiv zum Schutz gegen Metalleinflüsse) als Additiv enthalten.

Verschiedene Metalle, wie Kupfer und seine Verbindungen sind Katalysatoren für die Bildung von Peroxiden und tragen damit zur Oxidation bei. Geeignete Metalldesaktivatoren sind Chelatbildner, die die Metalloberfläche passivieren. Nicht einschränkende Beispiele für Metalldesaktivatoren sind Triazole oder Thiadiazole, speziell Aryltriazole, wie Benzotriazol und Tolyltriazol, Alkylderivate derartiger Triazole und Benzothiadiazole wie R(C₆H₃)N₂S, wobei R für H oder C₁- bis C₁₀-Alkyl steht.

Bevorzugt enthält die erfindungsgemäße Schmierstoffzusammensetzung wenigstens einen Metalldesaktivator in einer Menge von wenigstens 0,1 Gew.-%, besonders bevorzugt wenigstens 0,5 Gew.-%, insbesondere wenigstens 1,0 Gew.-%, bezogen auf das Gesamtgewicht der Schmierstoffzusammensetzung.

### Verschleißschutzmittel

Die erfindungsgemäße Schmierstoffzusammensetzung kann wenigstens ein Verschleißschutzmittel als Additiv enthalten.

Verschleißschutzmittel dienen dazu Verschleiß durch tribologische Prozesse, wie z. B. Flüssigkeits- und Mischreibung zu verhindern

Das Verschleißschutzmittel ist vorzugsweise ausgewählt unter mit Aminen neutralisierten Phosphaten, alkylierten und nicht alkylierten Triarylphosphaten, alkylierten und nicht alkylierten Triarylthiophosphaten, Zn-, Mo- oder W-dialkyldithiophosphate, Zn-, Mo- oder W-diaryldithiophosphaten, Carbamaten, Thiocarbamaten, Zn-, Mo- oder W-dithiocarbamaten, Dimercaptothiadiazol, Organoborate, Organophosphite und Mischungen davon.

Bevorzugt enthält die erfindungsgemäße Schmierstoffzusammensetzung wenigstens ein Verschleißschutzmittel in einer Menge von wenigstens 0,1 Gew.-%, besonders bevorzugt wenigstens 0,5 Gew.-%, insbesondere wenigstens 1,0 Gew.-%, bezogen auf das Gesamtgewicht der Schmierstoffzusammensetzung.

### Viskositätsindex-Verbesserer

Die erfindungsgemäße Schmierstoffzusammensetzung kann wenigstens einen Viskositätsindex-Verbesserer (VI-Verbesserer) als Additiv enthalten.

Beispiele für VI-Verbesserer sind Olefincopolymere, Polyalkylmethacrylate und dispergierend wirkende Olefincopolymere.

Bevorzugt enthält die erfindungsgemäße Schmierstoffzusammensetzung wenigstens einen VI-Verbesserer in einer Menge von wenigstens 0,1 Gew.-%, besonders bevorzugt wenigstens 0,5 Gew.-%, insbesondere wenigstens 1,0 Gew.-%, bezogen auf das Gesamtgewicht der Schmierstoffzusammensetzung.

### Hochdruckadditive

Die erfindungsgemäße Schmierstoffzusammensetzung kann wenigstens ein Hochdruckaditiv (extreme pressure additive) als Additiv enthalten.

Hochdruckadditive wirken als Deckschichtbildner und/oder oberflächenaktive Substanzen. Bevorzugte Hochdruckadditive sind ausgewählt unter Thiophosphaten, wie Zinkdithiophosphat, Molybdänoxidsulfiddithiophosphat, Molybdänaminverbindungen, Schwefelverbindungen, wie geschwefelten Öle und Fetten, geschwefelte Fettsäuren, geschwefelten Fettsäureestern, alkylierten Polysulfiden und Mischungen davon.

Bevorzugt enthält die erfindungsgemäße Schmierstoffzusammensetzung wenigstens ein Hochdruckadditiv in einer Menge von wenigstens 0,1 Gew.-%, besonders bevorzugt wenigstens 0,5 Gew.-%, insbesondere wenigstens 1,0 Gew.-%, bezogen auf das Gesamtgewicht der Schmierstoffzusammensetzung.

### Pourpoint-Verbesserer

Die erfindungsgemäße Schmierstoffzusammensetzung kann wenigstens einen Poupoint-Verbesserer als Additiv enthalten.

Bevorzugte Additive zur Verbesserung des Pourpoints sind ausgewählt unter linearen oder verzweigten, alkylierten, acrylierten und/oder aliphatischen Polymeren, Copolymeren, die einzeln oder in Kombination verwendet werden können. Ein spezielles Beispiel für einen Pourpoint-Erniedriger ist Polyalkylmethacrylat.

Bevorzugt enthält die erfindungsgemäße Schmierstoffzusammensetzung wenigstens einen Pourpoint-Verbesserer in einer Menge von wenigstens 0,1 Gew.-%, besonders bevorzugt wenigstens 0,5 Gew.-%, insbesondere wenigstens 1,0 Gew.-%, bezogen auf das Gesamtgewicht der Schmierstoffzusammensetzung.

### Haftfähigkeits-Verbesserer

Die erfindungsgemäße Schmierstoffzusammensetzung kann wenigstens einen Haftfähigkeits-Verbesserer als Additiv enthalten.

Bevorzugte Additive zur Verbesserung der Haftfähigkeit sind ausgewählt unter langkettigen polaren Polymeren, die dem Öl oder Fett eine erhöhte Haftfähigkeit auf der zu schmierenden Oberfläche verleihen.

Bevorzugt enthält die erfindungsgemäße Schmierstoffzusammensetzung wenigstens einen Haftfähigkeits-Verbesserer in einer Menge von wenigstens 0,1 Gew.-%, besonders bevorzugt wenigstens 0,5 Gew.-%, insbesondere wenigstens 1,0 Gew.-%, bezogen auf das Gesamtgewicht der Schmierstoffzusammensetzung.

Die folgenden Beispiele dienen der Erläuterung der Erfindung, ohne sie in irgendeiner Weise zu beschränken.

### BEISPIELE

### I.) Synthesebeispiele

### Beispiel 1: Synthese von Verbindung A)

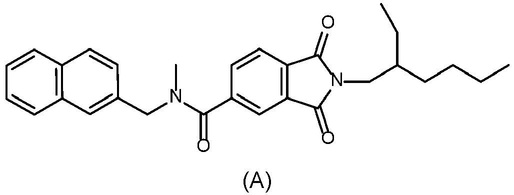

### Stufe 1a: N-(2-Ethylhexyl)-trimellitsäureimid

35,0 g (0,27 mol) 2-Ethylhexylamin und 52,0 g (0,27 mol) Trimellitsäureanhydrid wurden in 131,0 g Essigsäure vorgelegt. Die Mischung wurde für acht Stunden unter Rückfluss gerührt. Während der Synthese wurde ein leichter Stickstoffstrom durch die Apparatur geleitet. Nach dem Abkühlen der Lösung kristallisierte ein weißer Feststoff aus. Der Feststoff wurde abgesaugt, fünfmal mit 0,4 L Wasser gewaschen und anschließend bei 60°C unter Vakuum (0,2 mbar) getrocknet. Es wurde ein weißer Feststoff mit einer Ausbeute von 63,8 g (78%) erhalten.
¹H-NMR (CDCl₃): δ = 0,80-1,00 (m, 6 H), 1,32 (m, 8 H), 1,86 (m, 1 H), 3,63 (d, 2 H), 7,98 (d, 1 H), 8,49 (dd, 1 H), 8,58 (s, 1 H) ppm.

### Stufe 1b: N-(2-Ethylhexyl)-trimellitsäureimid-4-chlorid

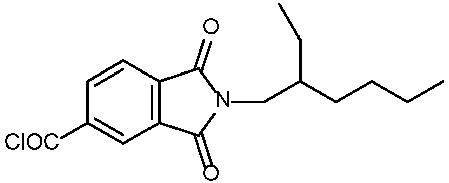

71,3 g (0,24 mol) der Verbindung aus Stufe 1a, 164,2 g (1,38 mol) Thionylchlorid und 4 Tropfen N,N-Dimethylformamid wurden vorgelegt und das Gemisch wurde für 6,3 Stunden unter Stickstoff am Rückfluss gerührt. Die erhaltene Lösung wurde abgekühlt, Thionylchlorid unter Membranpumpenvakuum (150 mbar) abdestilliert und abschließend bei 65°C unter Vakuum (0,3 mbar) getrocknet. Es wurde ein hellgelber Feststoff mit einer Ausbeute von 74,7 g (99%) erhalten.
¹H-NMR (CDCl₃): δ = 0,80-1,00 (m, 6 H), 1,16-1,42 (m, 8 H), 1,85 (m, 1 H), 3,63 (d, 2 H), 7,99 (d, 1 H), 8,47 (dd, 1 H), 8,58 (dd, 1 H) ppm.

### Stufe 1c: Verbindung A)

14,0 g (0,04 mol) der Verbindung aus Stufe 1b wurde in 70,0 g wasserfreiem Toluol vorgelegt. Die Lösung wurde auf 0°C abgekühlt. Zu dieser Lösung wurde 4,2 g (0,05 mol) wasserfreies Pyridin gegeben. Anschließend wurde eine Lösung aus 14,9 g (0,09 mol) N-Methyl-1-naphtylmethylamin in 17,0 g wasserfreiem Toluol über einen Zeitraum von 25 Minuten bei einer maximalen Innentemperatur von 5°C zugetropft. Dabei bildete sich ein gelblicher Feststoff. Nach beendeter Zugabe wurde die Mischung innerhalb von einer Stunde auf Raumtemperatur erwärmt und für weitere 15 Stunden gerührt. Während der Synthese wurde ein leichter Stickstoffstrom durch die Apparatur geleitet. Der gebildete Feststoff wurde abgesaugt und die resultierende Lösung am Rotationsverdampfer getrocknet. Der Rückstand wurde in 50 mL Ethylacetat aufgenommen, dreimal mit 50 ml wässriger HCl (1N) und anschließend viermal mit 50 ml halbgesättigter, wässriger NaCl-Lösung extrahiert. Die org. Phase wurde über Na₂SO₄ getrocknet, abgesaugt, am Rotationsverdampfer das Lösungsmittel entfernt und anschließend bei 60°C unter Vakuum (0,2 mbar) getrocknet. Es wurde ein viskoses, gelb-oranges Öl mit einer Ausbeute von 15,4 g (67%) erhalten.
¹H-NMR (CDCl₃): δ = 0,89 (m, 6 H), 1,29 (m, 8 H), 1,82 (m, 1 H), 2,76 (s, 2 H), 3,19 (s, 1 H), 3,58 (m, 2 H), 4,97 & 5,26 (2 x s, 2 H), 7,40-8,30 (m, 10 H) ppm.
DSC (10 K/min.): T_{g} = 5°C
Dynamische Viskosität (90,6 s⁻¹ @ 100°C): 730 mPas

### Beispiel 2: Synthese von Verbindung B)

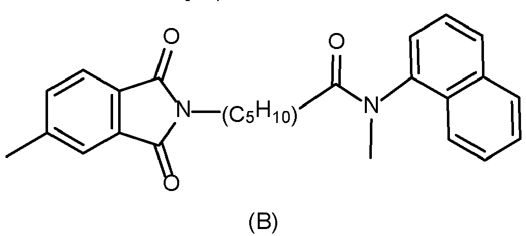

### Stufe 2a: 4-Methyl-N-(5-carboxypentyl)phthalimid

40,0 g (0,31 mol) 6-Aminohexansäure und 49,5 g (0,31 mol) 4-Methylphthalsäureanhydrid wurden in 76,0 g Essigsäure vorgelegt. Die Mischung wurde für acht Stunden unter Rückfluss gerührt. Während der Synthese wurde ein leichter Stickstoffstrom durch die Apparatur geleitet. Nach dem Abkühlen der Lösung kristallisierte ein gelblicher Feststoff aus. Der Feststoff wurde abgesaugt, fünfmal mit 0,4 L Wasser gewaschen und anschließend bei 60°C unter Vakuum (0,2 mbar) getrocknet. Es wurde ein gelblicher Feststoff mit einer Ausbeute von 81,8 g (97%) erhalten.
¹H-NMR (CDCl₃): δ = 1,30-1,50 (m, 2 H), 1,60-1,78 (m, 4 H), 2,35 (t, 2 H), 2,51 (s, 3 H), 2,90 (t, 2 H), 3,67 (t, 3 H), 7,51 (d, 1 H), 7,64 (m, 1 H), 7,72 (d, 1 H) ppm.

### Stufe 2b: Säurechlorid von 4-Methyl-N-(5-carboxypentyl)phthalimid

81,5 g (0,30 mol) der Verbindung aus Stufe 1a, 187,1 g (1,57 mol) Thionylchlorid und 5 Tropfen N,N-Dimethylformamid wurden vorgelegt und das Gemisch unter Stickstoff für 6,6 Stunden unter Rückfluss gerührt. Die erhaltene Lösung wurde abgekühlt, Thionylchlorid unter Membranpumpenvakuum (190 mbar) abdestilliert und abschließend bei 60°C unter Vakuum (0,2 mbar) getrocknet. Es wurde ein hellgelber Feststoff mit einer Ausbeute von 63,5 g (95%) erhalten.
¹H-NMR (CDCl₃): δ = 1,32-1,50 (m, 2 H), 1,62-1,83 (m, 4 H), 2,51 (s, 3 H), 3,63 (d, 2 H), 7,99 (d, 1 H), 8,47 (dd, 1 H), 8,58 (dd, 1 H) ppm.

### Stufe 2c: Verbindung B)

15,0 g (0,05 mol) der Verbindung aus Stufe 2b wurde in 90,0 g wasserfreiem Toluol vorgelegt und die Lösung auf 0°C abgekühlt. Zu dieser Lösung wurde 4,9 g (0,06 mol) wasserfreies Pyridin gegeben. Anschließend wurde eine Lösung aus 17,5 g (0,10 mol) N-Methyl-1-naphtylmethylamin in 22,0 g wasserfreiem Toluol über einen Zeitraum von 20 Minuten bei einer maximalen Innentemperatur von 5°C zugetropft. Dabei bildete sich ein gelblicher Feststoff. Nach beendeter Zugabe wurde die Mischung innerhalb von einer Stunde auf Raumtemperatur erwärmt und für weitere 21 Stunden gerührt. Während der Synthese wurde ein leichter Stickstoffstrom durch die Apparatur geleitet. Der gebildete Feststoff wurde abgesaugt und die resultierende Lösung am Rotationsverdampfer getrocknet. Der Rückstand wurde in 50 mL Ethylacetat aufgenommen und mit 50 ml wässriger HCl (1N) extrahiert. Danach wurde 50 ml Tetrahydrofuran zur organischen Phase gegeben und die Lösung noch zweimal mit 50 ml wässriger HCl (1N) extrahiert. Anschließend wurde die organische Phase viermal mit 50 ml halbgesättigter wässriger NaCl-Lösung extrahiert und abgetrennt. Die organische Phase wurde über Na₂SO₄ getrocknet, abgesaugt, das Lösungsmittel am Rotationsverdampfer entfernt und anschließend bei 60°C unter Vakuum (0,1 mbar) getrocknet. Es wurde ein viskoses, gelb-oranges Öl mit einer Ausbeute von 15,5 g (71%) erhalten.
¹H-NMR (CDCl₃): δ = 1,20-1,50 (m, 2 H), 1,55-1,85 (m, 4 H), 2,25-2,45 (m, 2 H), 2,48 & 2,50 (2 x s, 3 H), 2,83 (s, 2 H), 3,06 (s, 1 H), 3,55-3,75 (m, 2 H), 5,01 & 5,06 (2 x s, 2 H), 7,10-8,15 (m, 10 H) ppm.
DSC (10 K/min.): T_{g} = 10°C
Dynamische Viskosität (90,6 s⁻¹ @ 100°C): 300 mPas

### Beispiel 3: Synthese von Verbindung C)

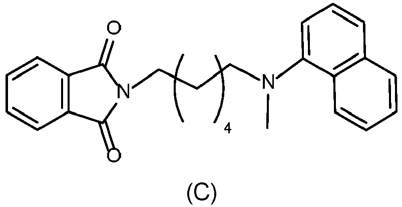

### Stufe 3a: N-(6-Bromhexyl)phthalimid

40,0 g (0,22 mol) Kaliumphthalimid, 211,0 g (0,86 mol) 1,6-Dibromhexan und 3,0 g (0,02 mol) wasserfreies K₂CO₃ wurden unter leichtem Stickstoffstrom in 711,0 g wasserfreiem Acetonitril vorgelegt. Das Reaktionsgemisch wurde für 20 Stunden unter Rückfluss gerührt. Während der Synthese wurde ein leichter Stickstoffstrom durch die Apparatur geleitet. Die Reaktionsmischung wurde abgekühlt und abgesaugt. Acetonitril und überschüssiges 1,6-Dibromhexan wurden von der resultierenden Lösung abdestilliert. Der Rückstand wurde in 100 mL Ethanol aufgenommen und zum Rückfluss des Ethanols erwärmt. Der nach dem Abkühlen gebildete kristalline Feststoff wurde abgesaugt und bei 160°C unter Vakuum (0,5 mbar) getrocknet. Es wurde ein leicht gelblicher Feststoff mit einer Ausbeute von 54,9 g (82%) erhalten.
¹H-NMR (DMSO-d₆): δ = 1,30 (m, 2 H), 1,41 (m, 2 H), 1,60 (q, 2 H), 1,79 (q, 2 H), 3,50 (t, 2 H), 3,57 (t, 2 H), 7,78-7,91 (m, 4 H) ppm.

### Stufe 3b: Verbindung C)

25,0 g (0,08 mol) der Verbindung aus Stufe 3a und 16,7 g (0,12 mol) wasserfreies K₂CO₃ wurden unter leichtem Stickstoffstrom in 195,0 g wasserfreiem Acetonitril vorgelegt. Anschließend wurde portionsweise 23,5 g (0,14 mol) N-Methyl-1-naphtylmethylamin innerhalb von 5 Minuten zugegeben. Der Stickstoffstrom wurde abgestellt und das Reaktionsgemisch wurde für 2 Stunden unter Rückfluss gerührt. Nach dem Abkühlen der Reaktionsmischung wurde der gebildete Feststoff abgesaugt und die resultierende Lösung am Rotationsverdampfer getrocknet. Der Rückstand wurde in 0,15 L Ethylacetat aufgenommen und mit 0,1 L Wasser gewaschen. Die organische Phase wurde abgetrennt und 0,14 L Tetrahydrofuran wurden zur organischen Phase gegeben. Es wurde zweimal mit 80 mL Wasser gewaschen. Die organische Phase wurde über Na₂SO₄ getrocknet, abgesaugt, am Rotationsverdampfer das Lösungsmittel entfernt und dann bei 190°C unter Vakuum (0,3 mbar) getrocknet. Es wurde ein viskoses, orangenes Öl mit einer Ausbeute von 29,0 g (90%) erhalten. Nach ca. fünf Tagen begann das Produkt langsam auszukristallisieren.
¹H-NMR (DMSO-d₆): δ = 1,22 (m, 4 H), 1,35-1,65 (m, 4 H), 2,25-2,45 (m, 2 H), 2,10 (s, 3 H), 2,37 (t, 2 H), 3,51 (t, 2 H), 3,81 (s, 2 H), 7,35-7,55 (m, 4 H), 7,75-7,90 (m, 6 H), 8,26 (dd, 1 H) ppm.
DSC (10 K/min.): T_{g} = -19°C; nach vollständiger Kristallisation weist das Produkt zusätzlich einen Tₘ von 72°C auf
Dynamische Viskosität (90,6 s⁻¹ @ 100°C): 40 mPas

### Beispiel 4: Synthese von Verbindung D)

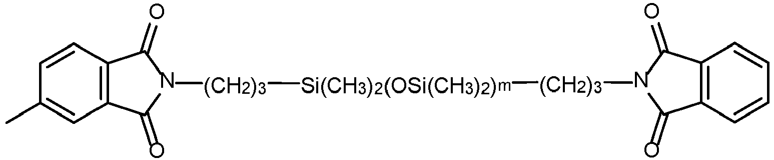

93,1 g (0,57 mol) 4-Methylphthalsäureanhydrid und 85,0 g (0,57 mol) Phthalsäureanhydrid wurden in 1047,0 g Essigsäure vorgelegt. Die Mischung wurde auf 50°C erwärmt. Nach vollständiger Lösung der Anhydride wurde 520,0 g (0,57 mol) Diaminopropyl-terminiertes Polydimethylsiloxan (Viskosität: 10-15 cSt) über einen Zeitraum von 60 Minuten bei einer maximalen Innentemperatur von 60°C zugetropft. Nach beendeter Zugabe wurde die Lösung auf Rückfluss erwärmt und für weitere acht Stunden unter Rückfluss gerührt. Während der Synthese wurde ein leichter Stickstoffstrom durch die Apparatur geleitet. Nach dem Abkühlen auf Raumtemperatur wurde die Reaktionslösung auf 1,5 L Wasser gegossen und 1,8 L Ethylacetat zugegeben. Die wässrige Phase wurde abgetrennt und die organische Phase wurde fünfmal mit 0,5 L halbgesättigter, wässriger NaCl-Lösung, zweimal mit 0,5 L wässriger NaHCOs-Lösung (5%) und einmal mit 0,75 L Wasser gewaschen. Die organische Phase wurde über Na₂SO₄ getrocknet, abgesaugt, das Lösungsmittel am Rotationsverdampfer entfernt und dann bei 100°C unter Vakuum (0,1 mbar) getrocknet. Es wurde eine niedrigviskose, gelborangene Flüssigkeit mit einer Ausbeute von 617,0 g erhalten.
¹H-NMR (CDCl₃): δ = 0,05 (m, 72 H), 0,53 (m, 4 H), 1,65 (m, 4 H), 2,47 (s, 3 H), 3,63 (m, 4 H), 7,45 (2, 1 H), 7,61 (s, 1 H), 7,63-7,73 (m, 3 H), 7,76-7,86 (m, 2 H) ppm.
DSC (10 K/min.): T_{g} = -81°C
Dynamische Viskosität (90,6 s⁻¹ @ 100°C): 14 mPas
Dynamische Viskosität (90,6 s⁻¹ @ 40°C): 81 mPas

### Beispiel 5: Synthese von Verbindung E)

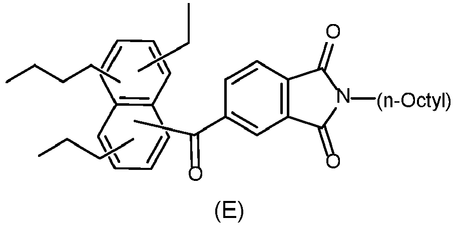

### Stufe 5a: N-(n-Octyl)-trimellitsäureimid

178,4 g (0,93 mol) Trimellitsäureanhydrid wurde in 254,0 g Essigsäure vorgelegt. 120,0 g (0,93 mol) 1-Octylamin wurde über einen Zeitraum von 14 Minuten zugegeben. Die Mischung wurde für acht Stunden unter Rückfluss gerührt. Während der Synthese wurde ein leichter Stickstoffstrom durch die Apparatur geleitet. Das abgekühlte Reaktionsgemisch wurde in 2,0 L Eiswasser gefällt, der Feststoff wurde sechsmal mit 1,8 L Wasser gewaschen und anschließend bei 60°C unter Vakuum (0,4 mbar) getrocknet. Es wurde ein weißer Feststoff mit einer Ausbeute von 273,6 g (97%) erhalten.
¹H-NMR (CDCl₃): δ = 0,87 (t, 3 H), 1,15-1,49 (m, 10 H), 1,69 (q, 2 H), 3,72 (t, 2 H), 7,98 (d, 1 H), 8,50 (dd, 1 H), 8,58 (s, 1 H) ppm.

### Stufe 5b: N-(n-Octyl)-trimellitsäureimid-4-chlorid

240,3 g (0,79 mol) der Verbindung aus Beispiel 5a, 440,5 g (3,70 mol) Thionylchlorid und 10 Tropfen N,N-Dimethylformamid wurden vorgelegt und das Gemisch unter Stickstoff für 6,5 Stunden unter Rückfluss gerührt. Die erhaltene Lösung wurde abgekühlt, Thionylchlorid unter Membranpumpenvakuum (170 mbar) abdestilliert und abschließend bei 60°C unter Vakuum (0,2 mbar) getrocknet. Es wurde ein hellgelber Feststoff mit einer Ausbeute von 246,6 g (97%) erhalten.
¹H-NMR (CDCl₃): δ = 0,87 (t, 3 H), 1,17-1,42 (m, 10 H), 1,69 (q, 2 H), 3,73 (t, 2 H), 8,00 (d, 1 H), 8,48 (dd, 1 H), 8,58 (d, 1 H) ppm.

### Stufe 5c: Verbindung E)

258,6 g (1,94 mol) wasserfreies Aluminiumchlorid wurde in 1662,0 g wasserfreiem Dichlormethan vorgelegt. Die Innentemperatur der Mischung wurde auf 5°C erniedrigt. Anschließend wurde eine Lösung aus 195,0 g (0,61 mol) der Verbindung aus Stufe 5b) in 324,0 g wasserfreiem Dichlormethan über einen Zeitraum von 16 Minuten bei einer maximalen Innentemperatur von 11°C zugegeben. Nach weiteren fünf Minuten wurde 531,0 g einer Mischung aus alkylierten Naphthalinen (NA-LUBE KR-015 von King Industries, kinematische Viscosität von 114 cSt at 40°C und von 13.5 cSt bei 100°C) in 400,0 g wasserfreiem Dichlormethan über einen Zeitraum von 24 Minuten bei einer maximalen Innentemperatur von 11°C zugegeben. Das Gemisch wurde für 110 Minuten bei 5°C gerührt, innerhalb von 50 Minuten auf Raumtemperatur erwärmt und dann für 21 Stunden refluxiert. Während der Synthese wurde ein leichter Stickstoffstrom durch die Apparatur geleitet. Die abgekühlte Reaktionsmischung wurde auf 3,0 L Eiswasser gegossen und für eine Stunde gerührt. Die organische Phase wurde abgetrennt und viermal mit 0,8 L wässriger Na₂CO₃-Lösung (5%) gewaschen. Beim ersten Waschschritt wurde zur orgaischen Phase 0,6 L Tetrahydrofuran, nach dem ersten Waschschritt 0,4 L Tetrahydrofuran und beim letzten Waschschritt 0,4 L einer Ethylacetat/Tetrahydrofuran-Mischung im Verhältnis 1:1 gegeben. Zum Abschluss wurde die organische Phase zweimal mit 0,7 L Wasser gewaschen, über Na₂SO₄ getrocknet, abgesaugt, am Rotationsverdampfer vom Lösungsmittel befreit und dann bei 120°C unter Vakuum (0,3 mbar) getrocknet. Es wurde ein viskoses, braunes Öl mit einer Ausbeute von 475,3 g erhalten.
DSC (10 K/min.): T_{g} = -40°C
Dynamische Viskosität (90,6 s⁻¹ @ 100°C): 90 mPas

### Beispiel 6: Synthese von Verbindung F)

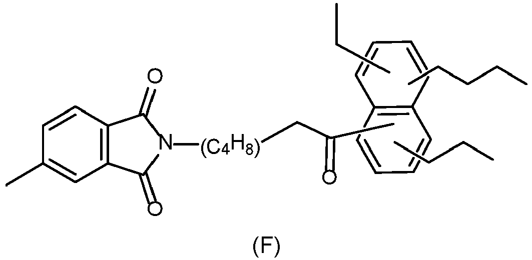

### Stufe 6a: 4-Methyl-N-(5-carboxypentyl)phthalimid

345,0 g (2,63 mol) 6-Aminohexansäure und 426,4 g (2,63 mol) 4-Methylphthalsäureanhydrid wurden in 657,0 g Essigsäure vorgelegt. Die Mischung wurde für acht Stunden unter Rückfluss gerührt. Während der Synthese wurde ein leichter Stickstoffstrom durch die Apparatur geleitet. Das abgekühlte Reaktionsgemisch wurde in 3,0 L Eiswasser gefällt und der Feststoff wurde viermal mit 3,0 L Wasser gewaschen und anschließend bei 100°C unter Vakuum (0,8 mbar) getrocknet. Es wurde ein schwach gelblicher Feststoff mit einer Ausbeute von 709,6 g (98%) erhalten.
¹H-NMR (CDCl₃): δ = 1,32-1,54 (m, 2 H), 1,60-1,82 (m, 4 H), 2,35 (t, 2 H), 2,51 (s, 3 H), 3,67 (t, 2 H), 7,50 (d, 1 H), 7,64 (s, 1 H), 7,72 (d, 1 H) ppm.

### Stufe 6b: Säurechlorid von 4-Methyl-N-(5-carboxypentyl)phthalimid

330,8 g (1,20 mol) der Verbindung aus Stufe 6a, 574,2 g (4,83 mol) Thionylchlorid und 12 Tropfen N,N-Dimethylformamid wurden vorgelegt und das Gemisch unter Stickstoff für 6,7 Stunden unter Rückfluss gerührt. Die erhaltene Lösung wurde abgekühlt, Thionylchlorid unter Membranpumpenvakuum (140 mbar) abdestilliert und abschließend bei 60°C unter Vakuum (0,2 mbar) getrocknet. Es wurde ein hellgelber Feststoff mit einer Ausbeute von 345,5 g (98%) erhalten.
¹H-NMR (CDCl₃): δ = 1,32-1,50 (m, 2 H), 1,62-1,83 (m, 4 H), 2,51 (s, 3 H), 2,90 (t, 2 H), 3,67 (t, 2 H), 7,51 (d, 1 H), 7,64 (s, 1 H), 7,72 (d, 1 H) ppm.

### Stufe 6c: Verbindung F)

290,5 g (2,18 mol) wasserfreies Aluminiumchlorid wurde in 1661,0 g wasserfreiem Dichlormethan vorgelegt. Die Innentemperatur der Mischung wurde auf 5°C erniedrigt. Anschließend wurde eine Lösung aus 200,0 g (0,68 mol) WTA2047 in 288,0 g wasserfreiem Dichlormethan über einen Zeitraum von 12 Minuten bei einer maximalen Innentemperatur von 11°C zugegeben. Nach weiteren fünf Minuten wurde eine Lösung aus 600,0 g NA-LUBE KR-015 in 400,0 g wasserfreiem Dichlormethan über einen Zeitraum von 7 Minuten bei einer maximalen Innentemperatur von 10°C zugegeben. Das Gemisch wurde für 53 Minuten bei 5°C gerührt, innerhalb von 50 Minuten auf Raumtemperatur erwärmt und dann für 22 Stunden refluxiert. Während der Synthese wurde ein leichter Stickstoffstrom durch die Apparatur geleitet.

Das abgekühlte Reaktionsgemisch wurde in 3,0 L Eiswasser gefällt und für eine Stunde gerührt. Die org. Phase wurde abgetrennt und dreimal mit 0,8 L wässr. Na₂CO₃-Lösung (5%) gewaschen. Zum Abschluss wurde die org. Phase zweimal mit 0,8 L Wasser gewaschen. Die org. Phase wurde über Na₂SO₄ getrocknet, abgesaugt, einrotiert und bei 120°C unter Vakuum (0,5 mbar) getrocknet. Es wurde ein viskoses, braunes Öl mit einer Ausbeute von 605,1 g erhalten.

### Analytik:

DSC (10 K/min.): T_{g} = -44°C

Dynamische Viskosität (29,0 s⁻¹ @ 100°C): 45 mPas

### Beispiel 7: Synthese von Verbindung G)

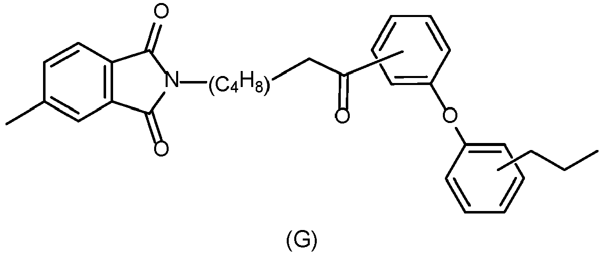

290,5 g (2,18 mol) wasserfreies Aluminiumchlorid wurde in 1661,0 g wasserfreiem Dichlormethan vorgelegt. Die Innentemperatur der Mischung wurde dann auf 5°C erniedrigt. Anschließend wurde eine Lösung aus 200,0 g (0,68 mol) der Verbindung aus Schritt 6b in 287,0 g wasserfreiem Dichlormethan über einen Zeitraum von 21 Minuten bei einer maximalen Innentemperatur von 11°C zugegeben. Nach weiteren fünf Minuten wurde eine Mischung aus 510,2 g alkylierten Diphenylethern (HILUBE LB-15 , dynamische Viskosität 15,8 mm²/s bei 40°C, Fa. Moresco, JP) in 404,0 g wasserfreiem Dichlormethan über einen Zeitraum von 16 Minuten bei einer maximalen Innentemperatur von 10°C zugegeben. Das Gemisch wurde für 66 Minuten bei 5°C gerührt, innerhalb von 40 Minuten auf Raumtemperatur erwärmt und dann für 20 Stunden refluxiert. Während der Synthese wurde ein leichter Stickstoffstrom durch die Apparatur geleitet. Das abgekühlte Reaktionsgemisch wurde in 3,0 L Eiswasser gefällt und für eine Stunde gerührt. Die organische Phase wurde abgetrennt und dreimal mit 0,6 L wässriger Na₂CO₃-Lösung (5%) gewaschen. Nach dem ersten Waschschritt wurde zur organischen Phase 0,5 L Ethylacetat gegeben. Zum Abschluss wurde die organische Phase zweimal mit 0,6 L Wasser gewaschen, über Na₂SO₄ getrocknet, abgesaugt und das Lösungsmittel wurde am Rotationsverdampfer entfernt. Das überschüssige HILUBE LB-15 wurde bei 220°C unter Vakuum (0,2 mbar) entfernt. Es wurde ein viskoses, dunkelbraunes Öl mit einer Ausbeute von 375,1 g erhalten.
DSC (10 K/min.): T_{g} = -33°C
Dynamische Viskosität (90,6 s⁻¹ @ 100°C): 72 mPas

### II.) Anwendungstechnische Beispiele

### Messmethoden

### Bestimmung der kinematischen Viskosität

Die kinematische Viskosität bei 40°C (KV40) und bei 100°C (KV100) wird gemäß ASTM D7042, Ausgabe Juli 2016 bestimmt. Die Viskositätsmessung wird mittels eines Stabinger Viskosimeters SVM 3000 (Anton Paar GmbH, DE) durchgeführt.

### Bestimmung der Scherviskosität

Die Scherviskosität wird mit einem Rheometer MCR 300 (Anton Paar GmbH, DE) gemäß DIN 53019-1:2008-09 bei einer Prüftemperatur von 25°C und einer Scherrate von 300 s⁻¹ bestimmt. Die nach einer Messdauer von 90 s ermittelten Werte entsprechen der Scherviskosität.

### Viskositätsindex

Die Berechnung des Viskostitätsindex (VI) kann aus der kinematischen Viskosität erfolgen, wie dies z.B. in der DIN ISO 2909:2004-08 angegeben ist.

### Dichte

Die Bestimmung der Dichten bei 20°C (p20), 40°C (p40), 100°C (p100) erfolgt gemäß DIN 51757:2011-01 mit einem Stabinger Rheometer.

### Bestimmung der Oxidationsstabilität:

Die Bestimmung der Oxidationsstabilität erfolgte gemäß DIN 51830-1, 2022-10 mit einem RapidOxy-Messgerät (Anton Paar GmbH, DE). Gemessen wird die Zeit, bis ausgehend von einem Anfangsdruck von 700 kPa Sauerstoff bei Raumtemperatur ein Druckverlust von 30 % des Anfangsdruckes bei der Messtemperatur auftritt.

### Pour point

Die Bestimmung des Pour points erfolgte gemäß DIN ISO 3016-2017-11.

### Verdampfungsverlust Aluschälchentest

1,50 g des zu prüfenden Öles werden in ein rundes Aluschälchen mit 2,3 cm Durchmesser und 1 cm Tiefe eingewogen. Das Gewicht des Schälchens mit Öl wird notiert. Ein Umluft Trockenschrank (Fa. Binder, Type FD 56) wird auf 200°C geheizt. Sobald die Prüftemperatur für 30 min stabil vorherrscht, wird das Ölgefüllte Schälchen in den Prüfraum des Trockenschrankes gegeben. Das Schälchen wird nach 24 bzw. 100 h aus dem Trockenschrank entnommen und das Schälchen ausgewogen. Aus der Differenz des Gewichtes vor der Prüfung und nach der Prüfung ergibt sich nach Division mit der Einwaage an Öl der Verdampfungsverlust.

### ¹H-NMR Spektroskopie

Verwendet wurde ein 300 MHz-Gerät des Typs Mercury 300 (Hersteller Varian).

### Glasübergangstemperaturen:

Die Bestimmung der Glasübergangstemperaturen erfolgte durch DSC (differential scanning calorimetry) mit einem DSC-Messgerät der Firma Netzsch-Gerätebau, DE, Typ DSC 214 Polyma. Die Probeneinwaage betrug 15 mg, die Aufheizrate 1K/min, erste Temperaturrampe von -150°C bis 100°C zur thermischen Kalibrierung, Abkühlen der Probe auf -150°C, neuerliche Temperaturrampe von -150°C bis 100°C. Ausgewertet wird der zweite Temperaturdurchlauf.

### II.1) Bestimmung der Oxidationsstabilität verschiedener Muster

Die Proben wurden bei 140°C gemessen, die Einwaage betrug 4,0 g.

| Beispiel Nr. | Verbindung | t [min] |
|---|---|---|
| 1 | D) | 6357 |
| 2 | G) | 3390 |
| 3 (Vergleich) | Alkylierter Diphenylether (Hilube PEC100, Fa. Moresco, JP, kinematische Viskosität bei 40°C ca. 100 mm²/s) | 326 |
| 4 (Vergleich) | Alkyliertes Napthalin | 1674 |
| | (NA-LUBE KR 015, King Industries, kinematische Viskosität bei 40°C ca. 100 mm²/s) | |
| 5 (Vergleich) | Dipentaerytrithester (Reaktionsprodukt von Dipentaerythrit mit C7, C8, i-C9 und C10 Säuren, kinematische Viskosität bei 40°C ca. 173 mm²/s) | 537 |

### II.2) Bestimmung des Viskositäts-Temperaturverhaltens, des Pour Points und der Oxidationsbeständigkeit verschiedener Muster

Die Bestimmung der Oxidationsbeständigkeit erfolgte mit einer Einwaage an Schmieröl von 5,0 ml bei einer Messtemperatur von 160°C.

| | | Verbindung | | | |
|---|---|---|---|---|---|
| Beispiel Nr. | Messwert | D) | E) | F) | G) |
| 6 | KV40 [mm²/s] | 79.79 | 4239.0 | 1408.0 | 4803.9 |
| 7 | KV100 [mm²/s] | 13.7 | 93.5 | 48.9 | 70.5 |
| 8 | VI | 177 | 70.6 | 68.1 | 4.8 |
| 9 | Pour Point [°C] | - 54 | - 9 | -15 | +6 |
| 10 | Scherviskosität bei 100°C [mPas] | 14 | 90 | 45 | 72 |
| 11 | Oxidationsbeständigkeit mit Rapid Oxy Tester, Muster mit 1 % p,p'-Dinonyldiphenylamin, [min] | 4334 | 438 | 220 | 265 |
| 12 | Glasübergangstemperatur [°C] | -88 | -46 | -35 | -46 |

### II.3) Bestimmung der kinematischen Viskosität, des Viskositätsindexes und der Dichte verschiedener Muster

Die Messungen erfolgten an Abmischungen verschiedener erfindungsgemäßer Verbindungen mit Trimellitsäureester A (TMA = Reaktionsprodukt der Trimellitsäure mit einem 1:1 molaren Gemisch von n-Octanol und n-Decanol) in einem Gewichtsmengenverhältnis von 1:1. Die Mischungen wurden durch Rühren mit einem Magnetrührer bei 80°C für 2 h hergestellt.

| Bsp. Nr. | Messwert | Verbindung D)/TMA | Verbindung E)/TMA | Verbindung F)/TMA | Verbindung F)/TMA |
|---|---|---|---|---|---|
| 13 | KV40 [mm²/s] | 61.27 | 260.77 | 203.46 | 281.88 |
| 14 | KV100 [mm²/s] | 10.1 | 20.8 | 17.7 | 19.4 |
| 15 | VI | 152 | 95 | 94 | 75 |
| 16 | ρ20 [g/ml] | 1.006 | 0.978 | 0.969 | 1.013 |
| 17 | ρ40 [g/ml] | 0.989 | 0.964 | 0.952 | 0.999 |
| 18 | ρ100 [g/ml] | 0.938 | 0.923 | 0.899 | 0.958 |

### II.4) Bestimmung der kinematischen Viskosität, des Viskositätsindexes und der Dichte verschiedener Muster

Die Messungen erfolgten an Abmischungen verschiedener erfindungsgemäßer Verbindungen mit Trimellitsäureester A (TMA = Reaktionsprodukt der Trimellitsäure mit einem 1:1 molaren Gemisch von n-Octanol und n-Decanol), wobei die Gewichtsmengenverhältnisse variiert wurden. Die Mischungen wurden durch Rühren mit einem Magnetrührer bei 80°C für 2 h hergestellt.

| Bsp. Nr. | Messwert | Verbindung E)/ TMA, 65:35 | Verbindung F)/ TMA, 75:25 | Verbindung G)/ TMA, 60:40 |
|---|---|---|---|---|
| 19 | KV40 [mm2/s] | 507.39 | 482.10 | 437.33 |
| 20 | KV100 [mm2/s] | 30.5 | 27.9 | 24.1 |
| 21 | VI | 89 | 81 | 66 |
| 22 | ρ20 [g/ml] | 0.979 | 0.972 | 1.021 |
| 23 | ρ40 [g/ml] | 0.965 | 0.959 | 1.007 |
| 24 | ρ100 [g/ml] | 0.925 | 0.920 | 0.966 |

### II.5) Versuche zur Additivierbarkeit von Verbindung D) im Vergleich zu einem Standard-Dimethylsilikonöl

Als Vergleichssilikonöl (Si Ref) wird ein Dimethylsilikonöl mit einer KV40 von 75 mm²/s verwendet.

Verbindung D) bzw. Si Ref werden mit je 1 Gew.-% eines Schmierstoffadditivs gemäß der folgenden Tabelle versetzt und die Mischung bei 60°C für 30 min gerührt (Magnetrührer, Becherglas, Rührstäbchen). Nach Erkalten werden die Proben begutachtet. Mischungen die Niederschläge oder Emulsionen bilden, werden als nicht mischbar beurteilt.

| | | Additiv | | |
|---|---|---|---|---|
| Bsp. Nr. | Verbindung | 1% Zinkdialkyldithiophosphat | 1 % Thiadiazol | 1 % Aminphosphat |
| 25 (Vergleich) | Si Ref | nicht mischbar | nicht mischbar | nicht mischbar |
| 26 | Verbindung D) | mischbar | mischbar | mischbar |

### II.6) Effekt von Antioxidantien auf die oxidative Stabilität von Verbindung D)

Verbindung D) wird mit 1 Gew.-% bzw. 3 Gew.-% des jeweiligen Antioxidants versetzt und bei 60°C für 30 min gerührt (Magnetrührer, Becherglas, Rührstäbchen). Nach Erkalten liegen klare Lösungen vor. Geprüft wird die Oxidationsstabilität mit dem RapidOxy Test wie unter Messmethoden beschrieben.

| Beispiel Nummer | 27 | 28 | 29 | 30 |
|---|---|---|---|---|
| Muster | D) ohne Additiv | D) mit 1% aminischem Antioxidant | D) mit 1% phenolischem Antioxidant | D) mit 3 % Ionischer Flüssigkeit |
| Oxidationsstabilität [min] | 1403 | 4334 | 3639 | 2155 |
| Verdampfungsverlust Aluschälchentest (200°C, 24h) | 20,0% | 11,5% | 9,3% | 9,2% |
| Verdampfungsverlust-Aluschälchentest (200°C, 168h) | 31,9% | 29,3% | 30,2% | 86,5% |

### Aminischer Antioxidant: p,p'-Dinonyldiphenylamin

Phenolischer Antioxidant: Tetrakis(methylen(3,5-di-tert-butyl-4-hydroxyhydrocinnamat))methan
lonische Flüssigkeit: Trihexyltetradecylphosphonium bis(trifluormethylsulfonyl)imid

### II.7) Mischungsversuche Verbindung D) mit Verbindung E) im Vergleich zu Si Ref mit Verbindung E)

Die Mischungen werden in den Gewichtsmengenverhältnissen wie in den nachfolgenden Tabellen angegeben, durch Rühren mit einem Magnetrührer bei 80°C für 2 h hergestellt. Mischungen, die nach Erkalten zwei Phasen aufweisen, werden als nicht mischbar gekennzeichnet.

**Vergleichsmischungen**

| Beispiel Nr. | 31 | 32 | 33 | 34 |
|---|---|---|---|---|
| SI Ref [Gew.-%] | 20 | 40 | 60 | 80 |
| Verbindung E) [Gew.-%] | 80 | 60 | 40 | 20 |
| Mischbarkeit | nicht mischbar | nicht mischbar | nicht mischbar | nicht mischbar |

**erfindungsgemäße Mischungen**

| Beispiel Nr. | 35 | 36 | 37 | 38 |
|---|---|---|---|---|
| Verbindung D) [Gew.-%] | 80 | 60 | 40 | 20 |
| Verbindung E) [Gew.-%] | 20 | 40 | 60 | 80 |
| Mischbarkeit | mischbar | mischbar | mischbar | mischbar |
| KV40 [mm²/sec] | 148,9 | 295,7 | 651,0 | 1522,7 |
| KV100 [mm2/sec] | 18,9 | 26,5 | 39.1 | 58,3 |
| VI | 144 | 117 | 98 | 85 |
| p40 [g/ml] | 1,019 | 1,006 | 0,994 | 0,982 |
| p100 [g/ml] | 0,974 | 0,963 | 0,952 | 0,941 |

## Patentansprüche

1. Schmierstoffzusammensetzung, enthaltend
a) ein Basisöl, enthaltend oder bestehend aus wenigstens eine(r) Phthalimidverbindung der allgemeinen Formel (I) 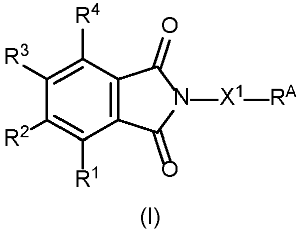 worin
R¹, R², R³ und R⁴ unabhängig voneinander ausgewählt sind unter Wasserstoff, geradkettigem oder verzweigtem Alkyl, geradkettigem oder verzweigtem Alkoxy, geradkettigem oder verzweigtem Alkanoyl, geradkettigem oder verzweigtem Alkyloxycarbonyl,
unsubstituiertem oder substituiertem Aryl, unsubstituiertem oder substituiertem Arylalkylen, unsubstituiertem oder substituiertem Aryloxy, unsubstituiertem oder substituiertem Arylalkylenoxy, unsubstituiertem oder substituiertem Arylthio, unsubstituiertem oder substituiertem Arylalkylenthio, unsubstituiertem oder substituiertem Aroyl, unsubstituiertem oder substituiertem Aryloxycarbonyl, unsubstituiertem oder substituiertem Arylcarbonyloxy,
Monoalkylaminocarbonyl, Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Dialkylaminocarbonyl, Diarylaminocarbonyl, Di(arylalkylen)aminocarbonyl, Monoalkanoylamino, Monoaroylamino, Monoalkanoylmonoaroylamino, Dialkanoylamino, Diaroylamino, wobei die Arylreste von Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Diarylaminocarbonyl, Di(arylalkylen)aminocarbonyl, Monoaroylamino, Monoalkanoylmonoaroylamino und Diaroylamino jeweils unsubstituiert oder substituiert sind,
unsubstituierten oder substituierten aromatischen Kohlenwasserstoffen mit 2, 3, 4 oder mehr aromatischen Ringen, wobei jeweils 2 der Ringe durch eine Einfachbindung oder eine divalente Gruppe, ausgewählt unter -CH₂-, -O-, -S- oder -N(H)- miteinander verbunden sind, und wobei die unsubstituierten oder substituierten aromatischen Kohlenwasserstoffe mit 2, 3, 4 oder mehr aromatischen Ringen über eine Einfachbindung oder eine divalente Gruppe, ausgewählt unter -CH₂-, -O-, -S-, -N(R^{a})-, -O-C(=O)- oder -N(R^{b})-C(=O)- an den Benzolring der Phthalimidgruppe gebunden sind, wobei R^{a} und R^{b} unabhängig voneinander ausgewählt sind unter Wasserstoff und C₁-C₄-Alkyl,
Carboxyl, Hydroxy, Nitro, NH₂, Alkylamino und Dialkylamino,
wobei die Reste R¹ und R² oder R² und R³ oder R³ und R⁴ auch gemeinsam für eine Gruppe der Formel (I.1) stehen können 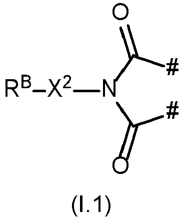 wobei
# jeweils die Bindungsstelle zum Rest des Moleküls bezeichnet,
wobei einer der Reste R¹, R², R³ oder R⁴ auch gemeinsam mit einem weiteren Rest R¹, R², R³ oder R⁴, der an eine weitere Gruppe der Formel (I) gebunden ist, für eine zweiwertige verbrückende Gruppe Y stehen kann, die die zwei Gruppen der Formel (I) miteinander verbindet,
X¹ und, falls vorhanden, X² unabhängig voneinander für geradkettiges oder verzweigtes C₁-C₃₀-Alkylen steht, wobei die Alkylenkette durch 1 bis 15 nicht benachbarte Heteroatome, ausgewählt unter O und S, unterbrochen sein kann, oder eine Gruppe der Formel #-(CR⁵R⁶)ₙ-SiR⁷R⁸(O-SiR⁷R⁸)ₘ-(CR⁹R¹⁰)ₚ-# steht, worin
# jeweils die Bindungsstelle zum Rest des Moleküls bezeichnet,
R⁵, R⁶, R⁹ und R¹⁰ unabhängig voneinander für Wasserstoff oder C₁-C₄-Alkyl stehen,
R⁷ und R⁸ unabhängig voneinander für geradkettiges oder verzweigtes Alkyl oder für unsubstituiertes oder jeweils mit 1, 2 oder 3 C₁-C₄-Alkylgruppen substituiertes Phenyl stehen,
n für eine ganze Zahl von 1 bis 30 steht,
m für eine ganze Zahl von 1 bis 30 steht,
p für eine ganze Zahl von 1 bis 30 steht,
oder eine Gruppe der Formel #-(CR¹¹R¹²)_{q}-NR¹³-(CR¹⁴R¹⁵)ᵣ-# steht, worin
# jeweils die Bindungsstelle zum Rest des Moleküls bezeichnet,
R¹¹, R¹², R¹⁴ und R¹⁵ unabhängig voneinander für Wasserstoff oder C₁-C₄-Alkyl stehen,
R¹³ für Wasserstoff oder C₁-C₄-Alkyl steht,
q für eine ganze Zahl von 1 bis 30 steht,
r für eine ganze Zahl von 1 bis 30 steht,
oder eine Gruppe der Formel (I.2) 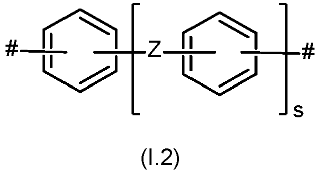 steht, worin
# jeweils die Bindungsstelle zum Rest des Moleküls bezeichnet,
Z für O oder S steht,
s für eine ganze Zahl von 1 bis 30 steht,
Y für eine Gruppe der Formel -(O)_{0,1}-(C₁-C₃₀-Alkylen)-(O)_{0,1}- oder -(S)_{0,1}-(C₁-C₃₀-Alkylen)-(S)_{0,1}- oder -(C=O)-(O)_{0,1}-(C₁-C₃₀-Alkylen)-(O)_{0,1}-(C=O)- steht,
wobei Alkylen geradkettig oder verzweigt ist und durch 1 bis 15 nicht benachbarte Heteroatome, ausgewählt unter O und S, unterbrochen sein kann,
oder Y für eine Gruppe der Formel
#-(O)_{0,1}-(CR⁵R⁶)ₙ-SiR⁷R⁸(O-SiR⁷R⁸)ₘ-(CR⁹R¹⁰)ₚ-(O)_{0,1}-# oder
#-(S)_{0,1}-(CR⁵R⁶)ₙ-SiR⁷R⁸(O-SiR⁷R⁸)ₘ-(CR⁹R¹⁰)ₚ-(S)_{0,1}-#
steht, worin
# jeweils die Bindungsstelle zum Rest des Moleküls bezeichnet,
R⁵, R⁶, R⁹ und R¹⁰ unabhängig voneinander für Wasserstoff oder C₁-C₄-Alkyl stehen,
R⁷ und R⁸ unabhängig voneinander für geradkettiges oder verzweigtes Alkyl oder für unsubstituiertes oder jeweils mit 1, 2 oder 3 C₁-C₄-Alkylgruppen substituiertes Phenyl stehen,
n für eine ganze Zahl von 1 bis 30 steht,
m für eine ganze Zahl von 1 bis 30 steht,
p für eine ganze Zahl von 1 bis 30 steht,
oder Y für eine Gruppe der Formel
#-(O)_{0,1}-(CR¹¹R¹²)_{q}-NR¹³-(CR¹⁴R¹⁵)ᵣ-(O)_{0,1}-# oder
#-(S)_{0,1}-(CR¹¹R¹²)_{q}-NR¹³-(CR¹⁴R¹⁵)ᵣ-(S)_{0,1}-#
steht, worin
# jeweils die Bindungsstelle zum Rest des Moleküls bezeichnet,
R¹¹, R¹², R¹⁴ und R¹⁵ unabhängig voneinander für Wasserstoff oder C₁-C₄-Alkyl stehen,
R¹³ für Wasserstoff oder C₁-C₄-Alkyl steht,
q für eine ganze Zahl von 1 bis 30 steht,
r für eine ganze Zahl von 1 bis 30 steht,
oder Y für eine Gruppe der Formel (I.3) 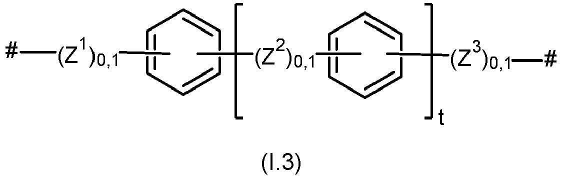 steht, worin
# jeweils die Bindungsstelle zum Rest des Moleküls bezeichnet,
Z¹, Z² und Z³ unabhängig voneinander für O oder S stehen,
t für 0 oder eine ganze Zahl von 1 bis 30 steht,
R^{A} und, falls vorhanden, R^{B} unabhängig voneinander für Wasserstoff, unsubstituiertes oder substituiertes Aryl, unsubstituiertes oder substituiertes Aroyl, Monoalkylaminocarbonyl, Monoarylaminocarbonyl, Mono(arylalkylen)aminocarbonyl, Monoalkylmonoarylaminocarbonyl, Monoalkyl(monoarylalkylen)aminocarbonyl, Monoarylmono(arylalkylen)aminocarbonyl, Dialkylaminocarbonyl, Diarylaminocarbonyl, Di(arylalkylen)aminocarbonyl, Monoalkanoylamino, Monoaroylamino, Monoalkanoylmonoaroylamino, Dialkanoylamino, Diaroylamino, wobei die Arylreste von Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Diarylaminocarbonyl, Di(arylalkylen)aminocarbonyl, Monoaroylamino, Monoalkanoylmonoaroylamino und Diaroylamino jeweils unsubstituiert oder substituiert sind, oder eine Phthalimidgruppe der Formel (I.4) 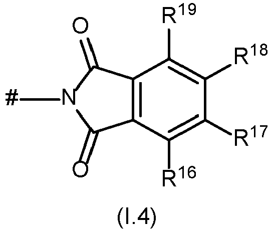
steht, worin
R¹⁶, R¹⁷, R¹⁸ und R¹⁹ unabhängig voneinander die zuvor für R¹, R², R³ und R⁴ angegebene Bedeutung haben,
und
b) wenigstens ein davon verschiedenes Additiv.

2. Schmierstoffzusammensetzung nach einem der vorhergehenden Ansprüche, wobei in den Verbindungen der Formel (I) wenigstens einer der Reste R¹, R², R³ und R⁴ und/oder, falls vorhanden, wenigstens einer der Reste R¹⁶, R¹⁷, R¹⁸ und R¹⁹ für eine Gruppe steht, ausgewählt unter den Gruppen (AR-I) bis (AR-XXIV) 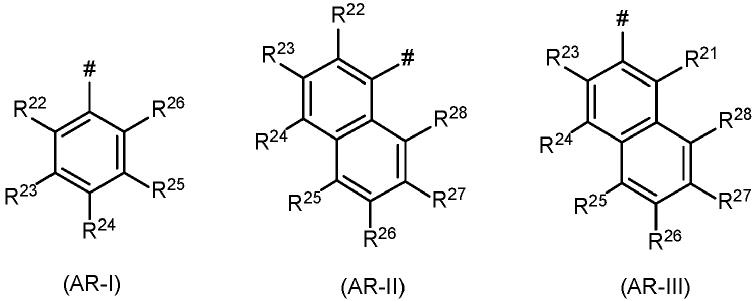 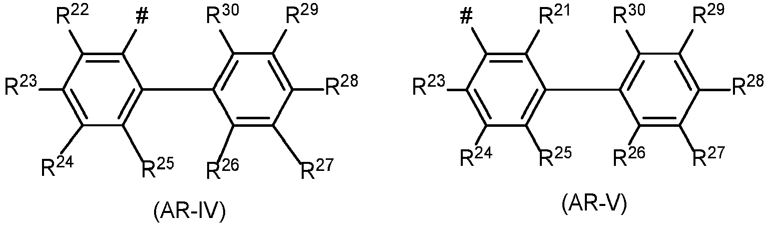 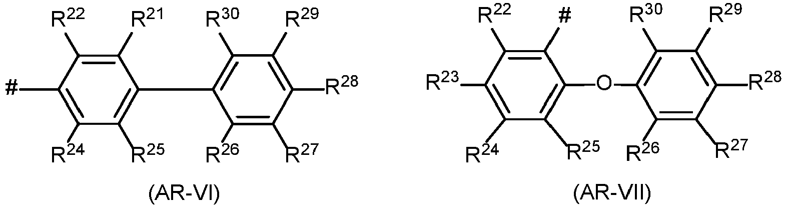 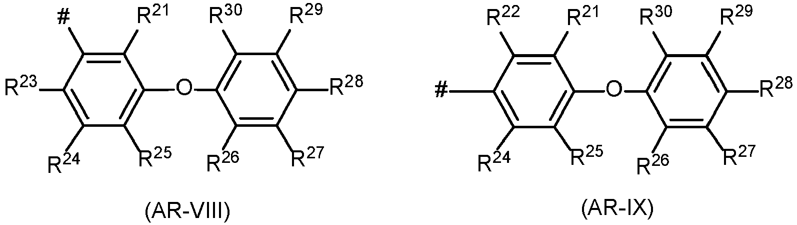 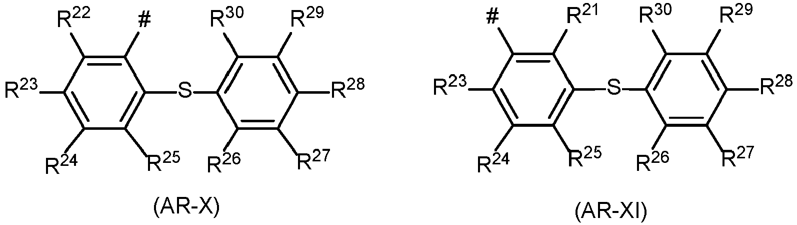 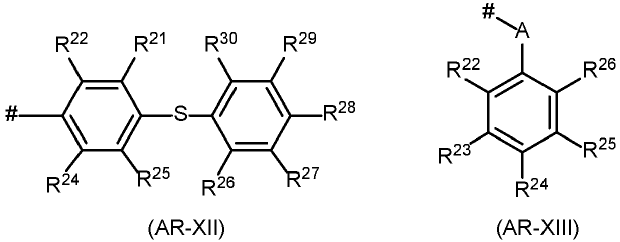 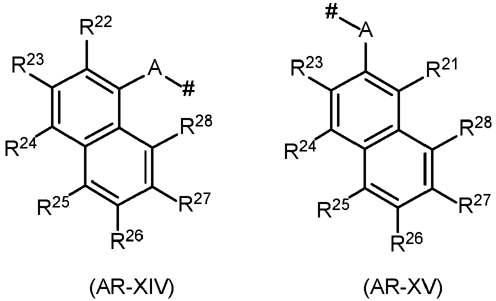 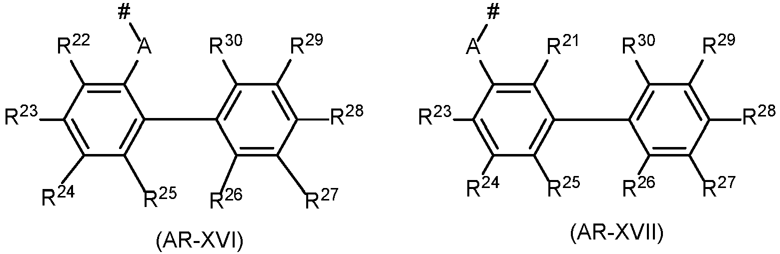 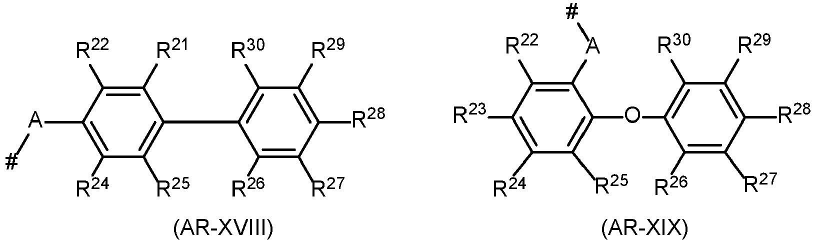 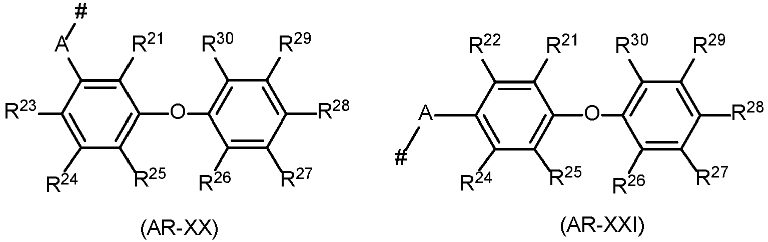 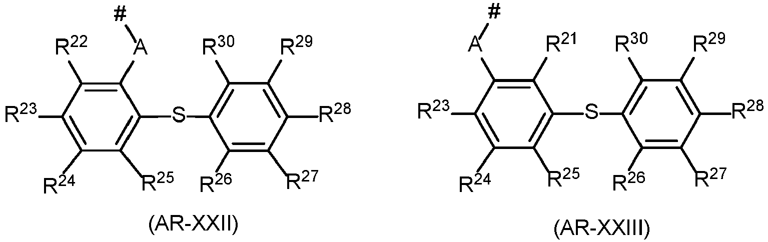 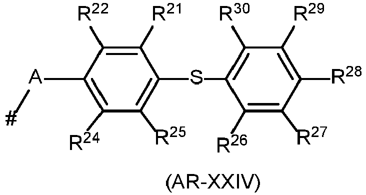 worin
# jeweils die Bindungsstelle zum Rest des Moleküls bezeichnet,
A ausgewählt ist unter den Gruppen 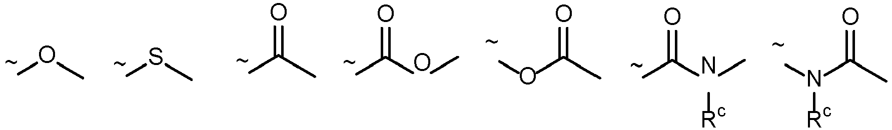
worin
~ jeweils die Bindungsstelle der Gruppe A zum Benzolring bezeichnet,
R^{c} für Wasserstoff oder C₁-C₄-Alkyl steht,
R²¹, R²², R²³, R²⁴, R²⁵, R²⁶, R²⁷, R²⁸, R²⁹ und R³⁰, falls vorhanden, unabhängig voneinander ausgewählt sind unter Wasserstoff, geradkettigem oder verzweigtem C₁-C₂₀-Alkyl und geradkettigem oder verzweigtem C₁-C₄-Alkoxy.

3. Schmierstoffzusammensetzung nach Anspruch 1 oder 2, wobei
in den Verbindungen der Formel (I) wenigstens einer der Reste R¹, R², R³ und R⁴ für Wasserstoff steht, bevorzugt wenigstens zwei der Reste R¹, R², R³ und R⁴ für Wasserstoff stehen, bevorzugt wenigstens drei der Reste R¹, R², R³ und R⁴ für Wasserstoff stehen, insbesondere die Reste R¹, R³ und R⁴ für Wasserstoff stehen oder die Reste R², R³ und R⁴ für Wasserstoff stehen oder die Reste R¹, R², R³ und R⁴ für Wasserstoff stehen,
und/oder
falls vorhanden, in den Gruppen der Formel (I.4) wenigstens einer der Reste R¹⁶, R¹⁷, R¹⁸ und R¹⁹ für Wasserstoff steht, bevorzugt wenigstens zwei der Reste R¹⁶, R¹⁷, R¹⁸ und R¹⁹ für Wasserstoff stehen, bevorzugt wenigstens drei der Reste R¹⁶, R¹⁷, R¹⁸ und R¹⁹ für Wasserstoff stehen, insbesondere die Reste R¹⁶, R¹⁸ und R¹⁹ für Wasserstoff stehen oder die Reste R¹⁷, R¹⁸ und R¹⁹ für Wasserstoff stehen oder die Reste R¹⁶, R¹⁷, R¹⁸ und R¹⁹ für Wasserstoff stehen.

4. Schmierstoffzusammensetzung nach einem der vorhergehenden Ansprüche, wobei in den Verbindungen der Formel (I)
X¹ und, falls vorhanden, X² unabhängig voneinander für
geradkettiges oder verzweigtes C₁-C₂₀-Alkylen steht, oder
eine Gruppe der Formel #-(CH₂CH₂O)₁₋₁₀-(CH₂CH₂)-# steht, oder
eine Gruppe der Formel #-(CH₂)₁₋₅-Si(CH₃)₂-(O-(Si(CH₃)₂))₁₋₅-(CH₂)₁₋₅-# steht, oder
eine Gruppe der Formel #-(CH₂)₁₋₅-NR¹³-(CH₂)₁₋₅-# steht, worin R¹³ für Wasserstoff oder C₁-C₄-Alkyl steht, oder
oder eine Gruppe der Formel (I.2a) 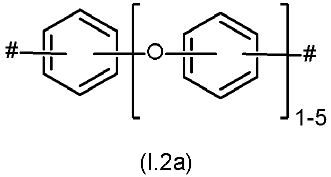 steht, worin
# jeweils die Bindungsstelle zum Rest des Moleküls bezeichnet.

5. Schmierstoffzusammensetzung nach einem der vorhergehenden Ansprüche, wobei in den Verbindungen der Formel (I)
Y für -(O)_{0,1}-(C₁-C₂₀-Alkylen)-(O)_{0,1}- oder -(S)_{0,1}-(C₁-C₂₀-Alkylen)-(S)_{0,1}- oder -(C=O)-(O)_{0,1}-(C₁-C₃₀-Alkylen)-(O)_{0,1}-(C=O)- steht, wobei Alkylen geradkettig oder verzweigt ist, oder
eine Gruppe der Formel #-(O)_{0,1}-(CH₂CH₂O)₁₋₁₀-(CH₂CH₂)-(O)_{0,1}-# steht, oder
eine Gruppe der Formel #-(O)_{0,1}-(CH2)₁₋₅-Si(CH₃)₂-(O-(Si(CH₃)₂))₁₋₅-(CH₂)₁₋₅-(O)_{0,1}-# steht, oder
eine Gruppe der Formel #-(O)_{0,1}-(CH₂)₁₋₅-NR¹³-(CH₂)₁₋₅-(O)_{0,1}-# steht, worin R¹³ für Wasserstoff oder C₁-C₄-Alkyl steht, oder
oder eine Gruppe der Formel (I.3a) 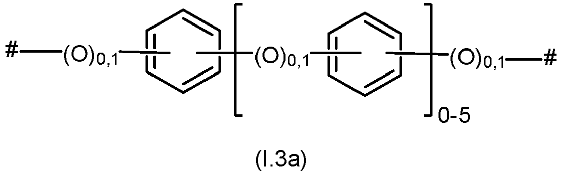 steht, worin
# jeweils die Bindungsstelle zum Rest des Moleküls bezeichnet.

6. Schmierstoffzusammensetzung nach einem der vorhergehenden Ansprüche, wobei in den Verbindungen der Formel (I) wenigstens einer der Reste R^{A} und, falls vorhanden, R^{B} für eine Gruppe (AR-I) bis (AR-XXIV), wie in Anspruch 2 definiert, stehen.

7. Schmierstoffzusammensetzung nach einem der vorhergehenden der Ansprüche, wobei in den Verbindungen der Formel (I) wenigstens einer der Reste R^{A} und, falls vorhanden, R^{B} für eine Phthalimidgruppe der Formel (I.4) stehen.

8. Schmierstoffzusammensetzung nach einem der vorhergehenden der Ansprüche, umfassend eine Verbindung der Formel (I), ausgewählt unter Verbindungen (1) bis (116) und den durch Reduktion der nicht-amidischen Carbonylgruppe(n) der Verbindungen (1) - (9), (22) - (27) und (71) - (90) erhältlichen Verbindungen 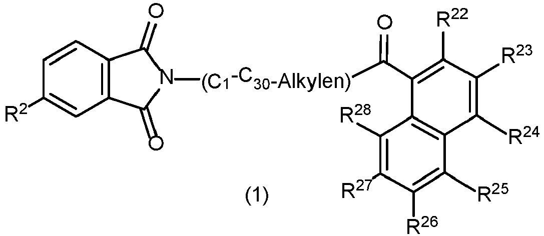 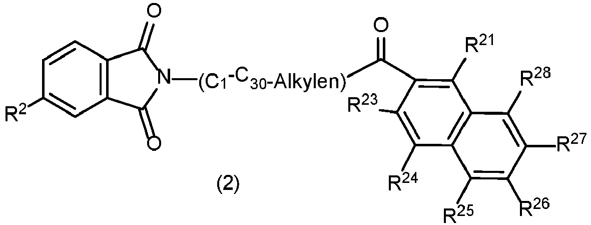 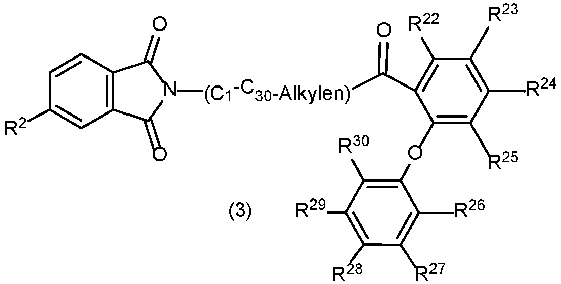 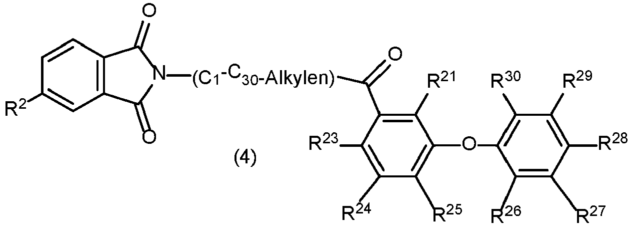 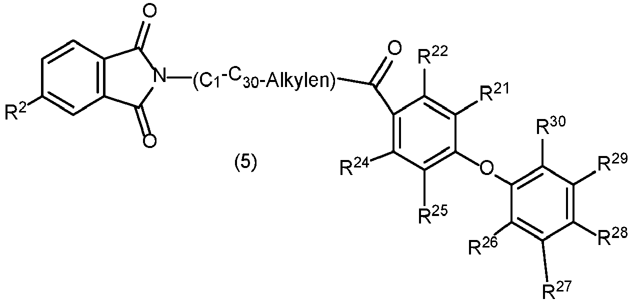 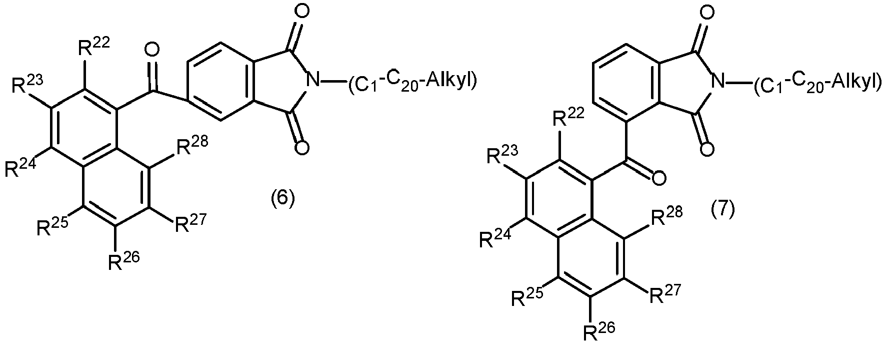 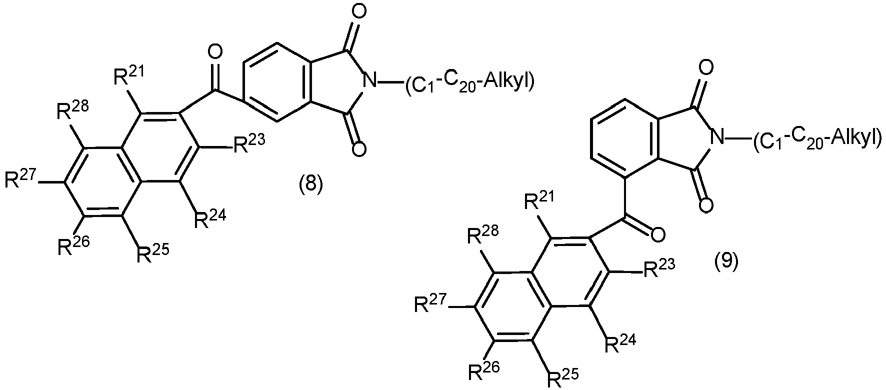 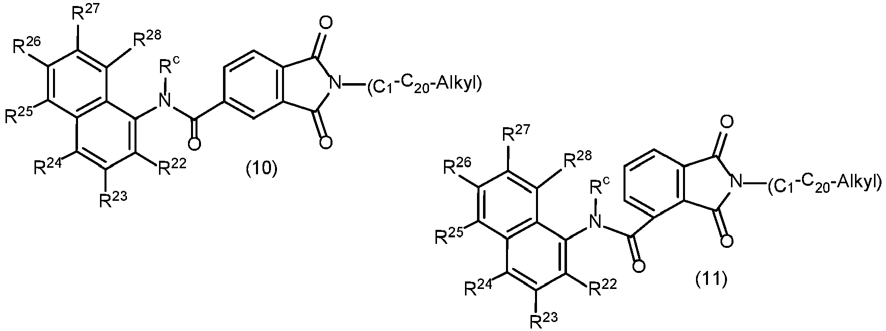 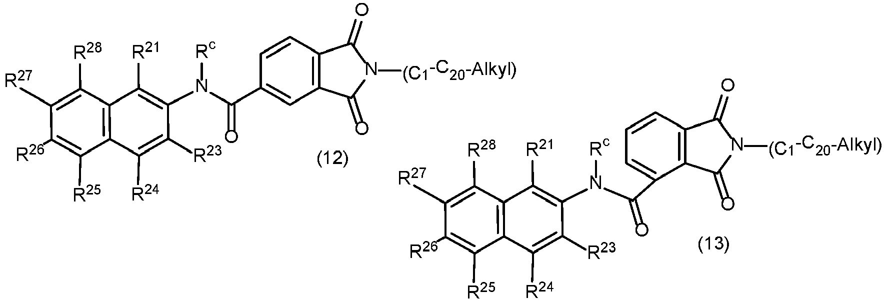 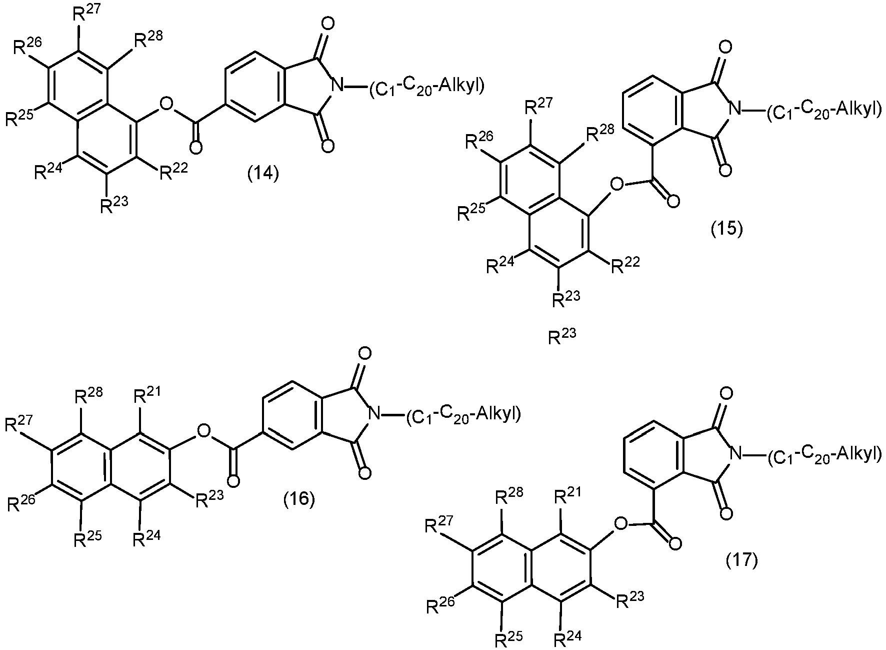 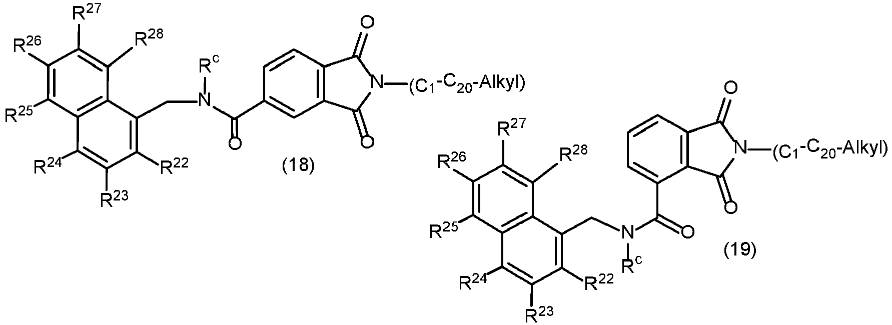 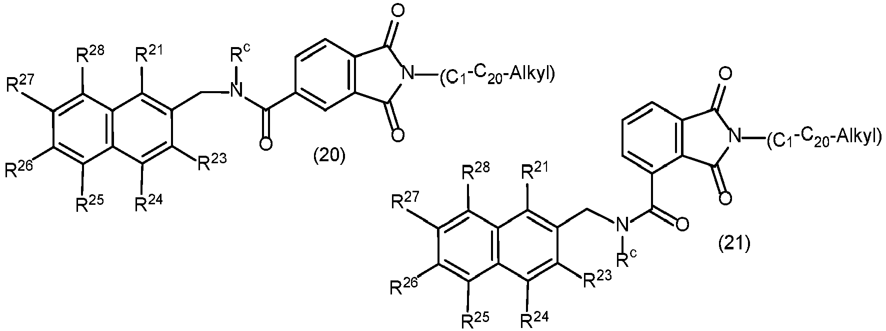 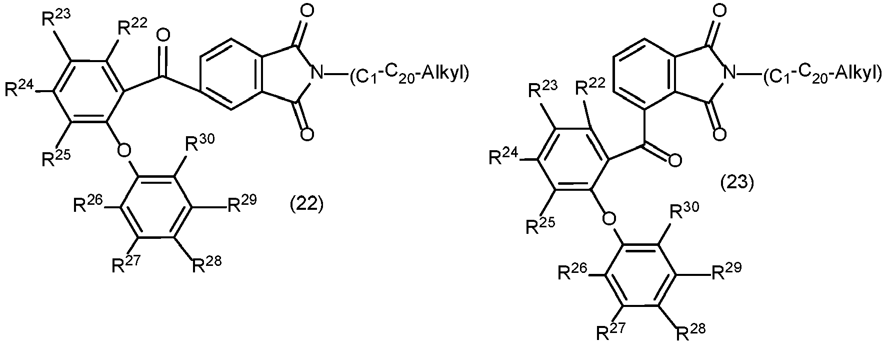 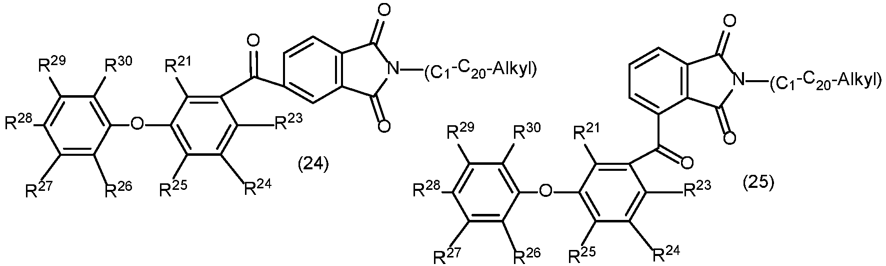 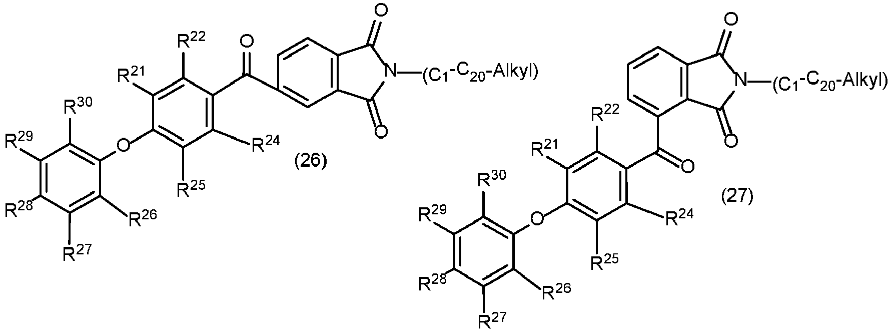 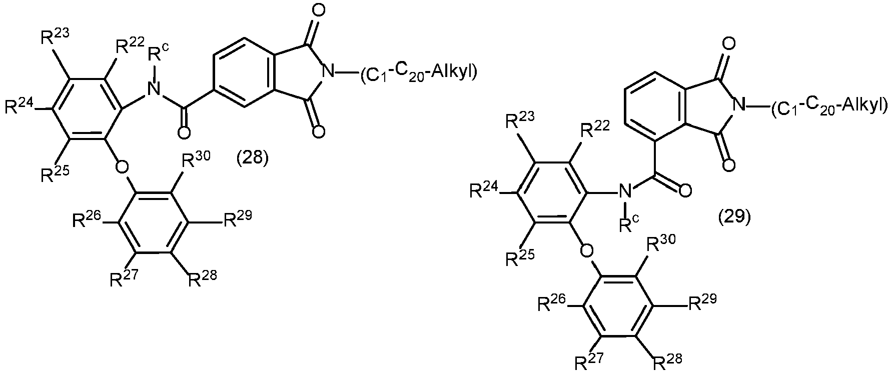 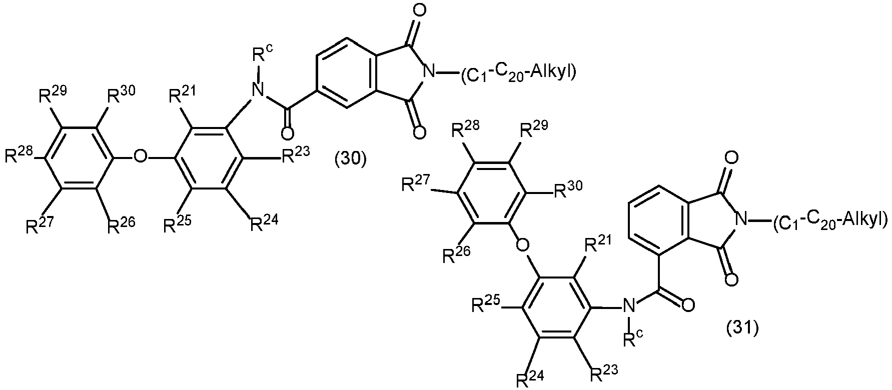 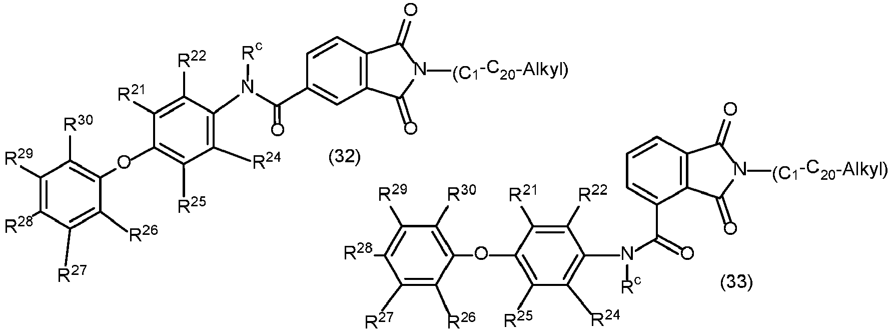 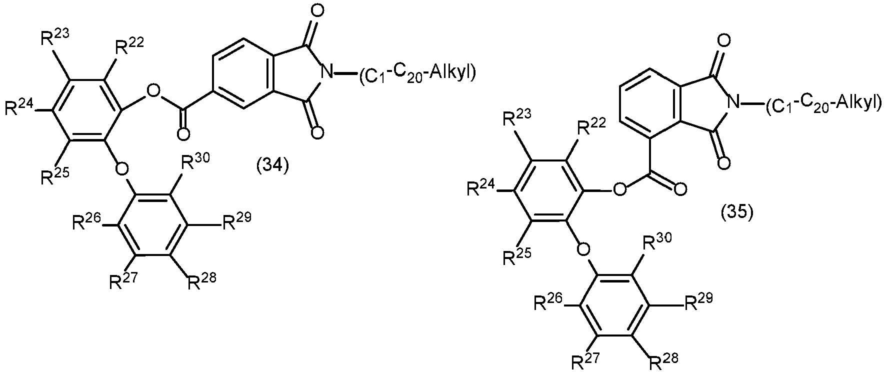 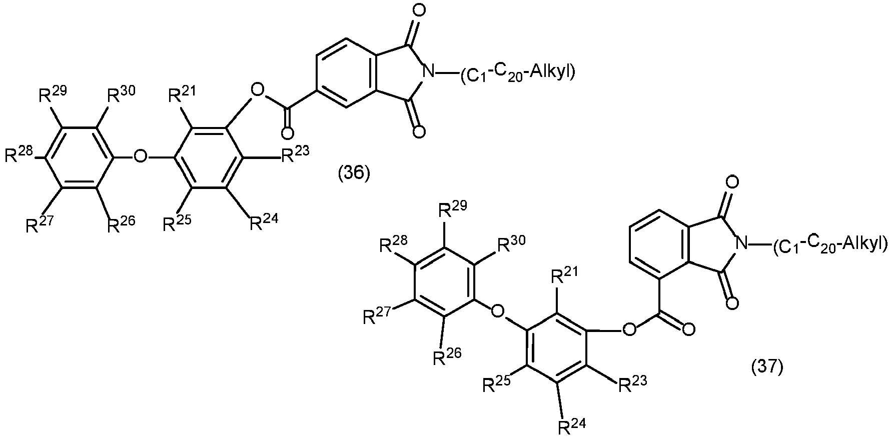 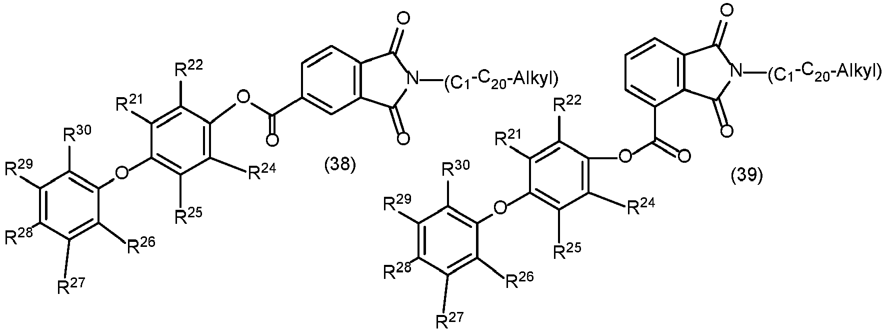 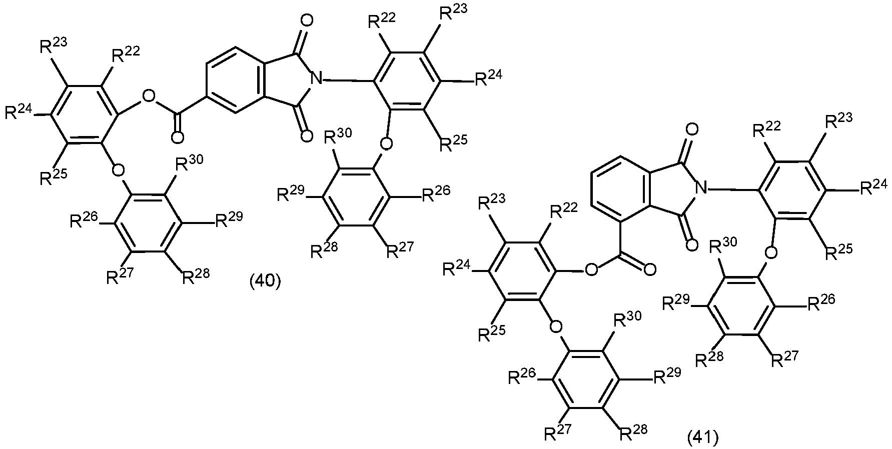 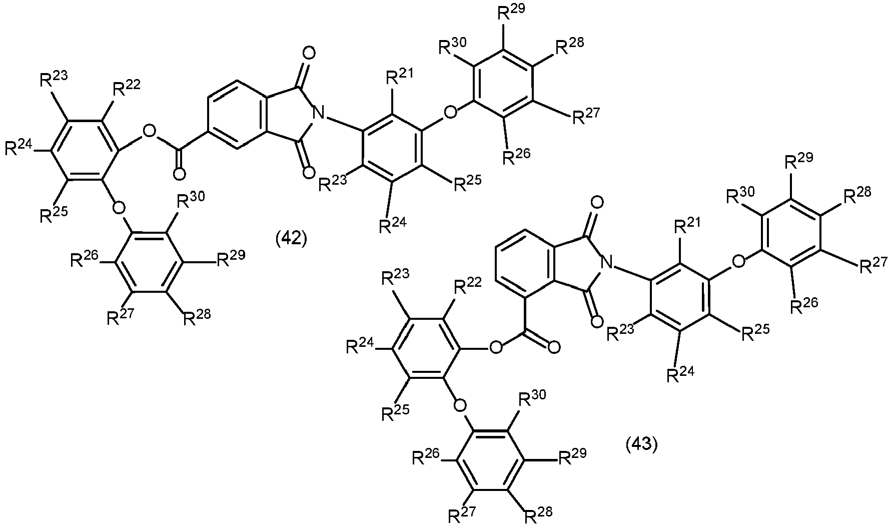 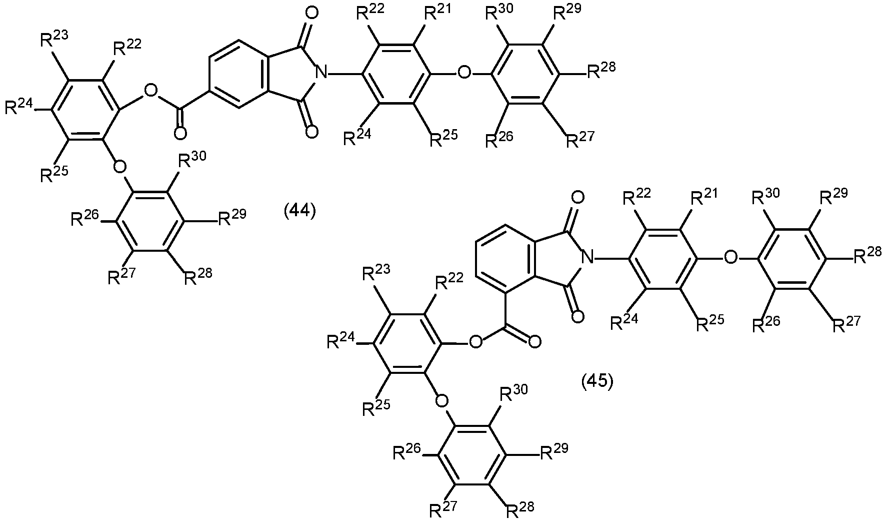 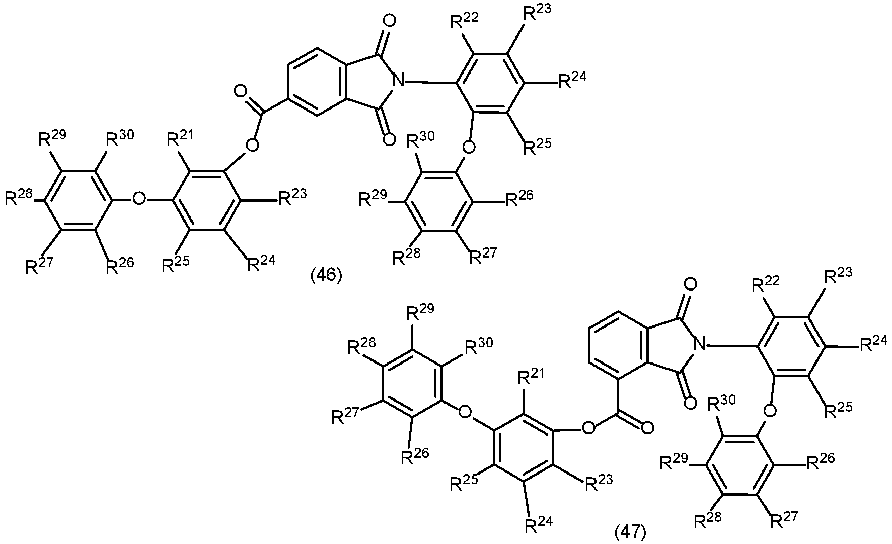 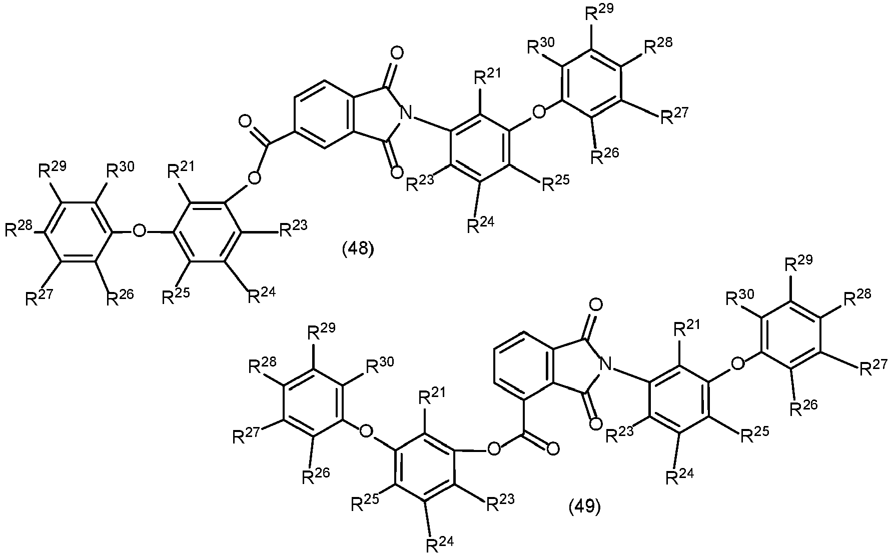 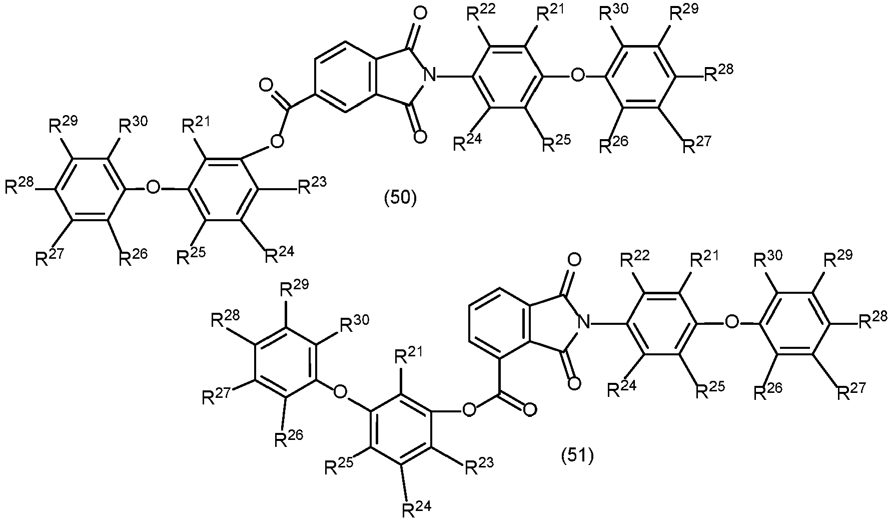 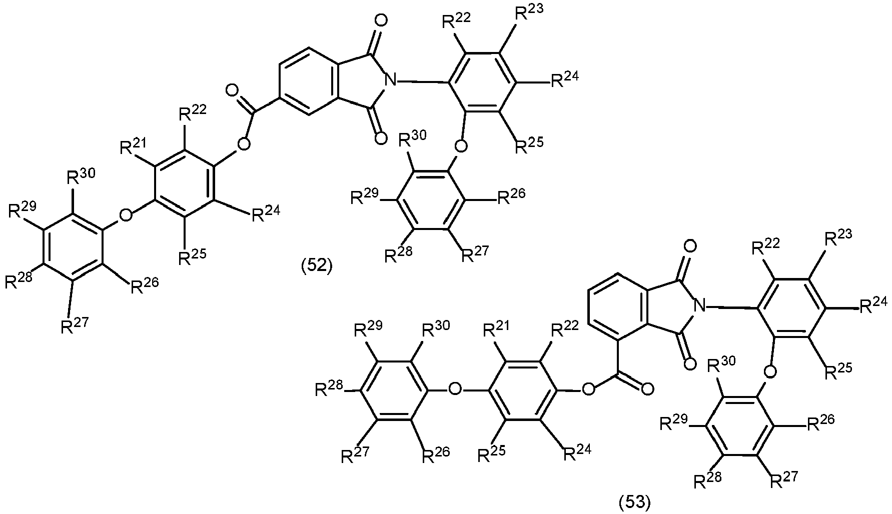 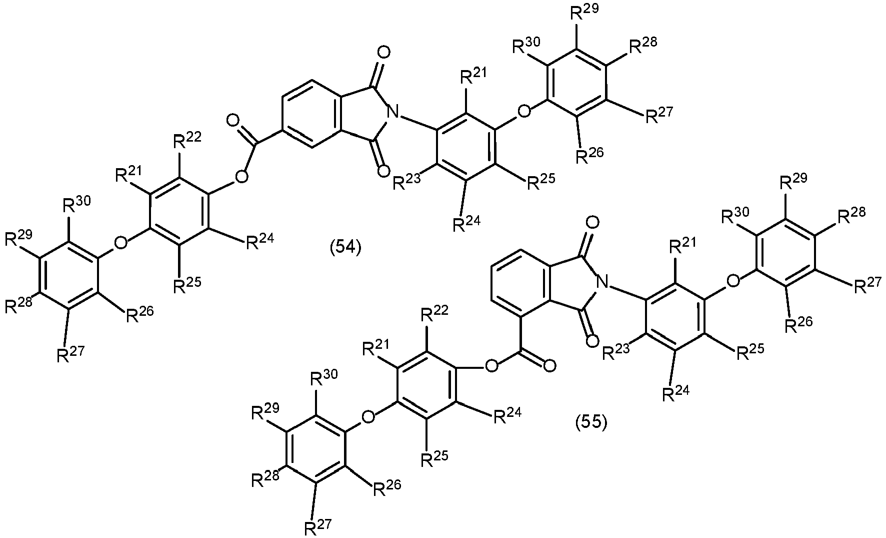 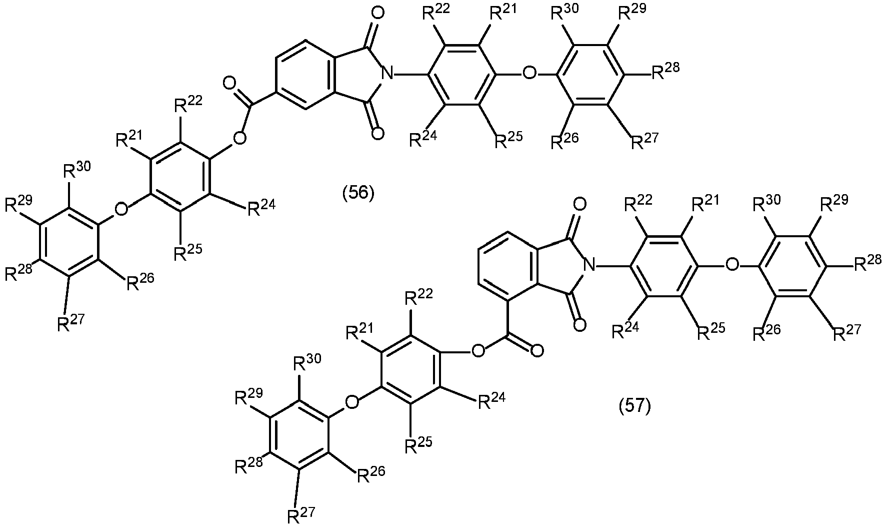 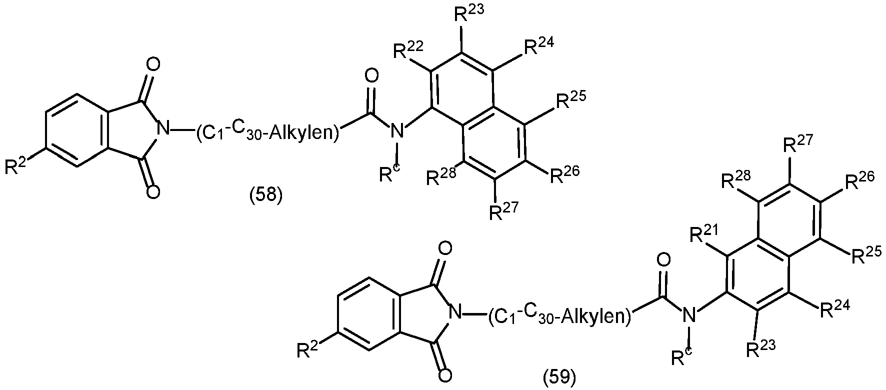 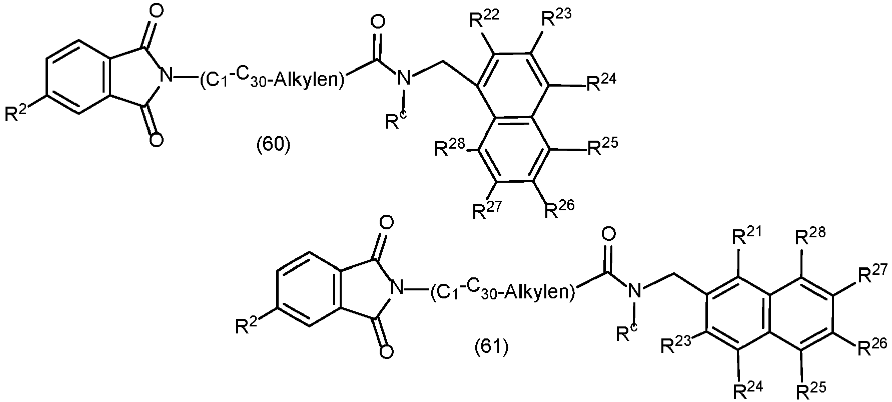 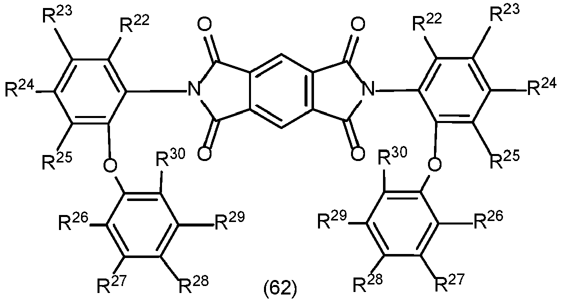 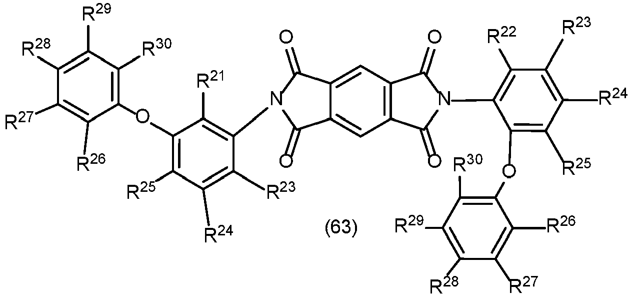 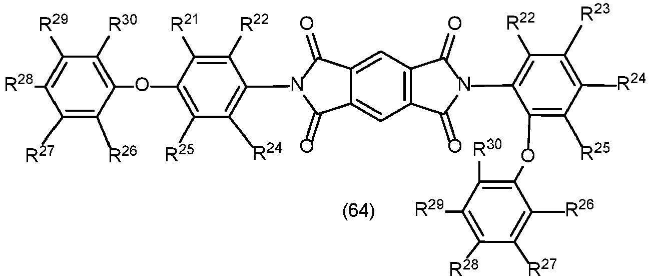 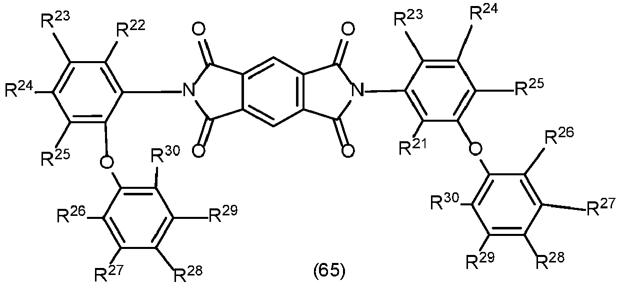 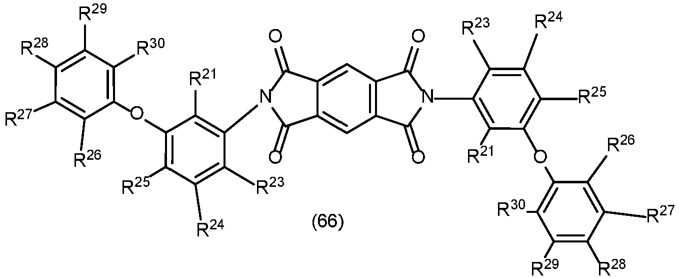 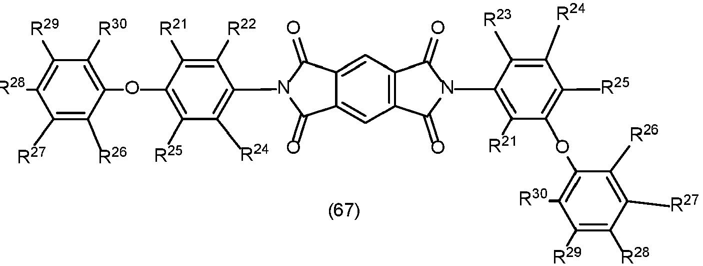 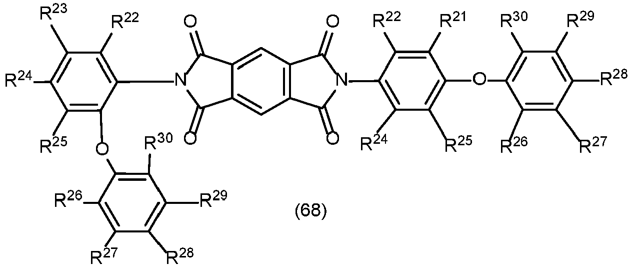 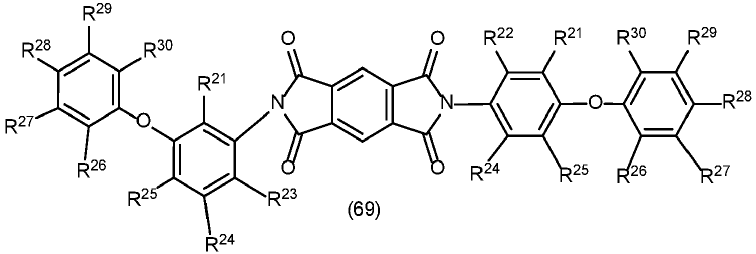 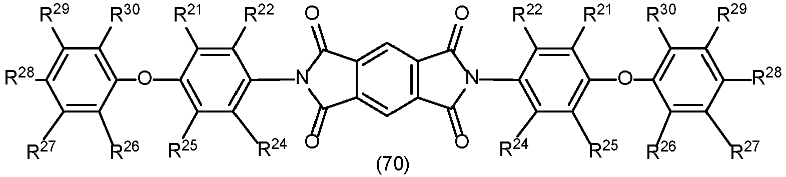 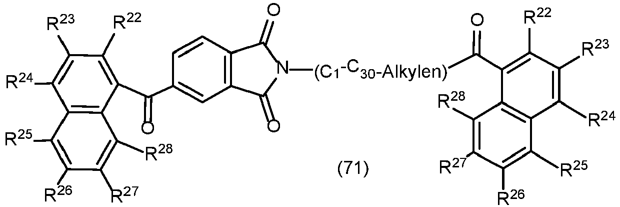 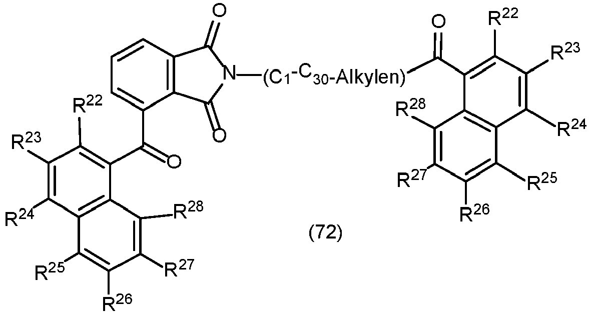 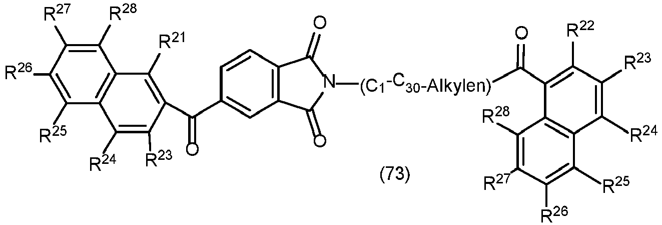 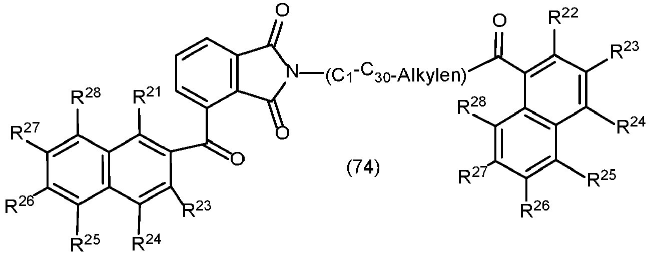 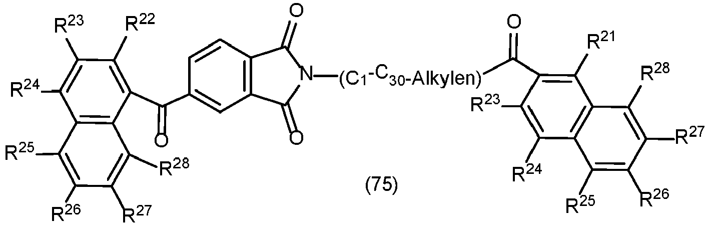 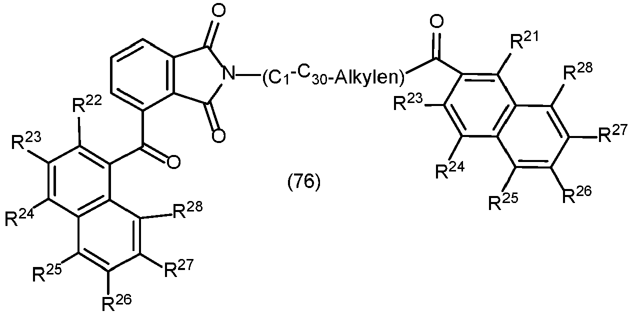 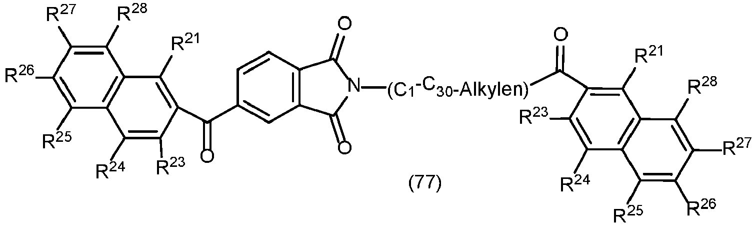 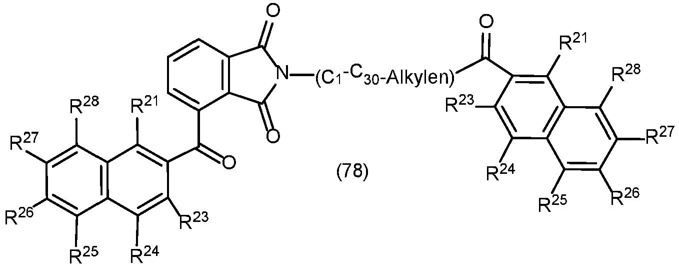 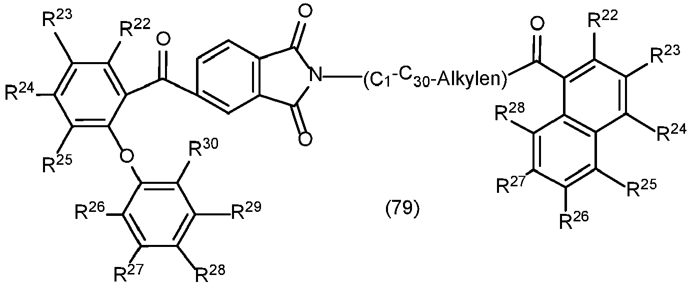 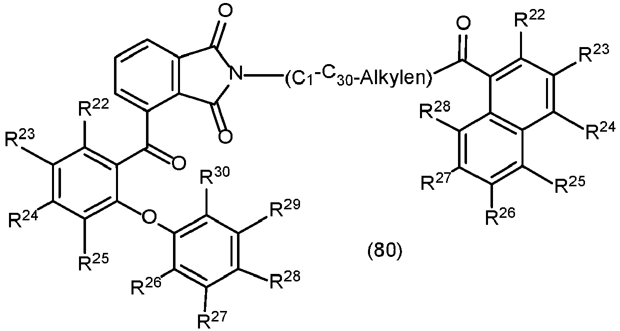 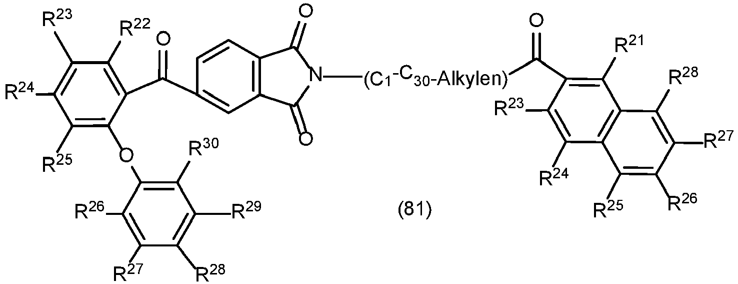 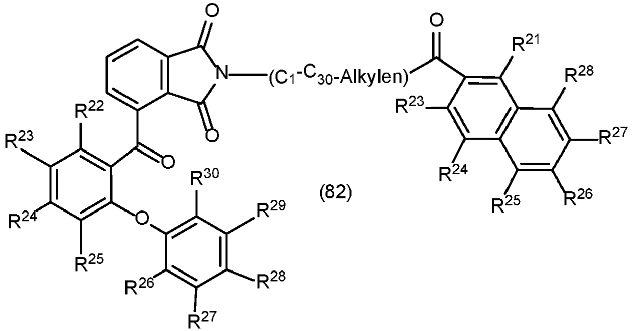 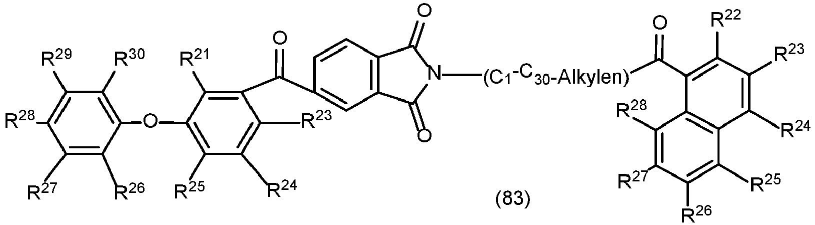 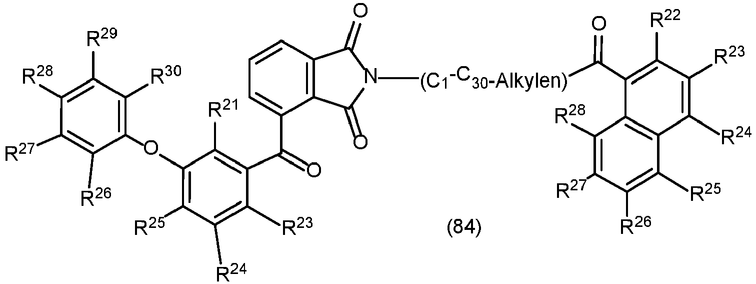 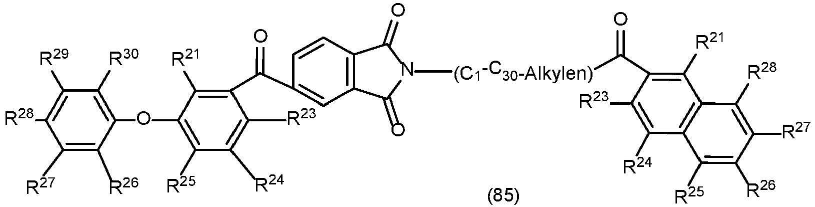 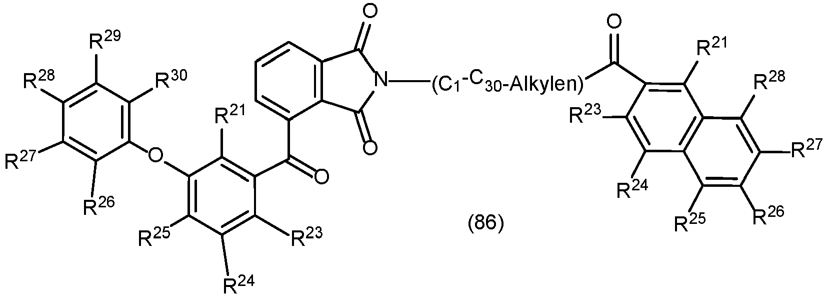 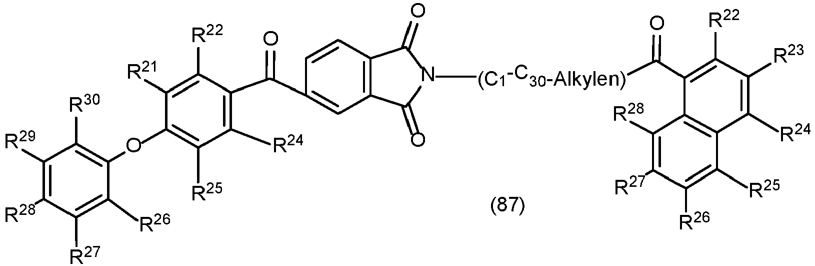 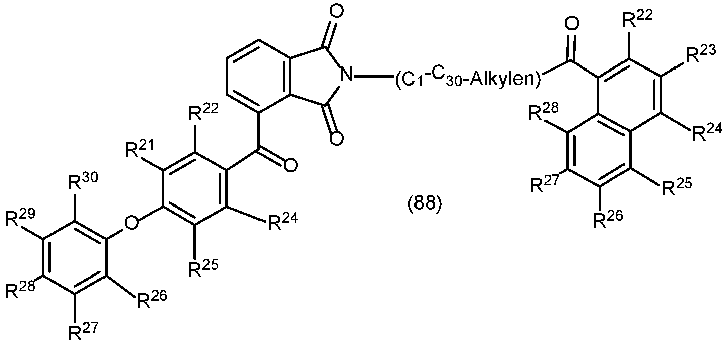 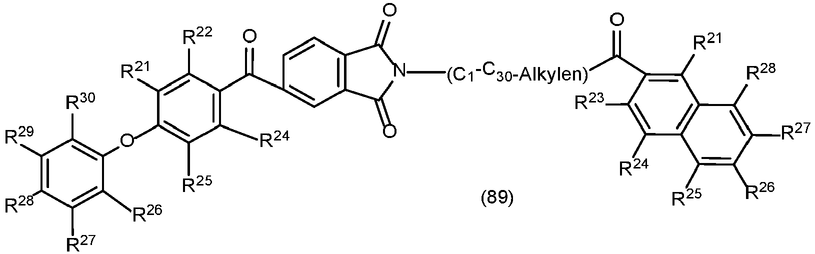 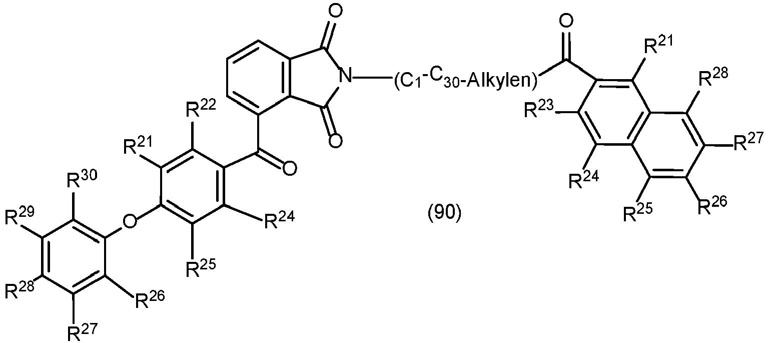 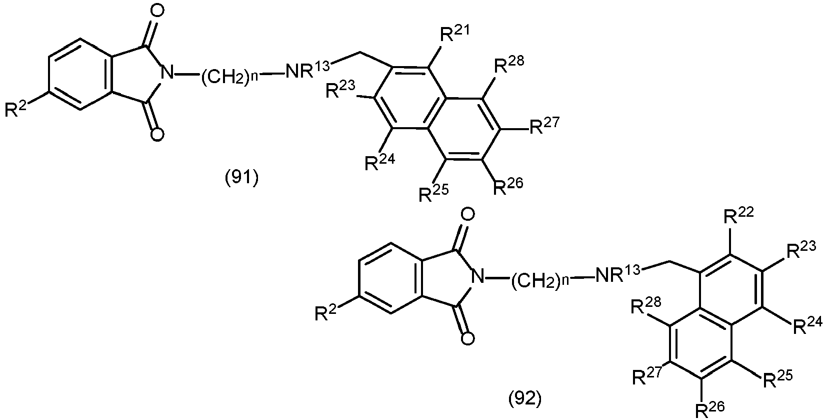 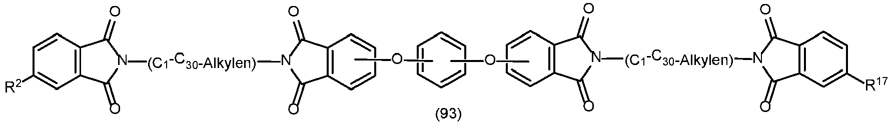 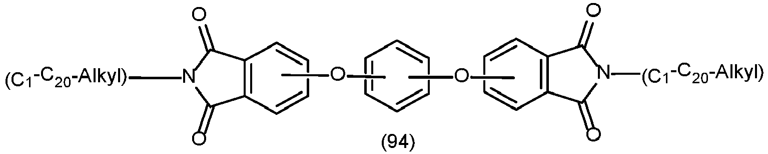 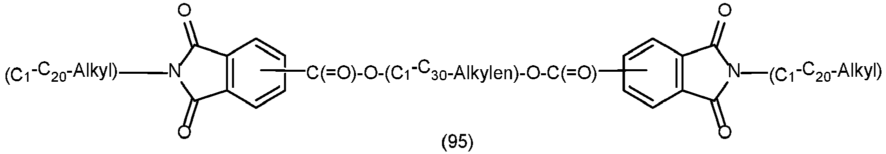 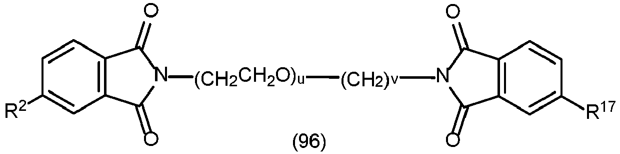 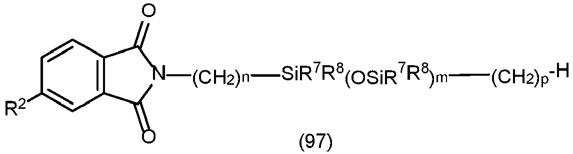 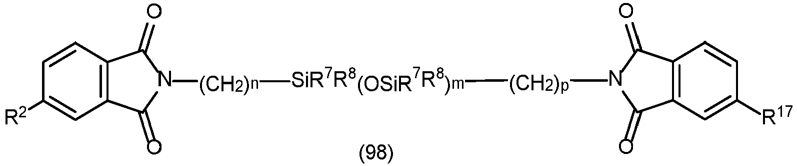 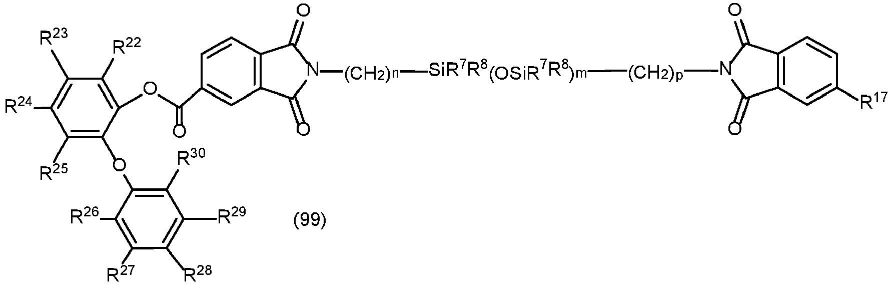 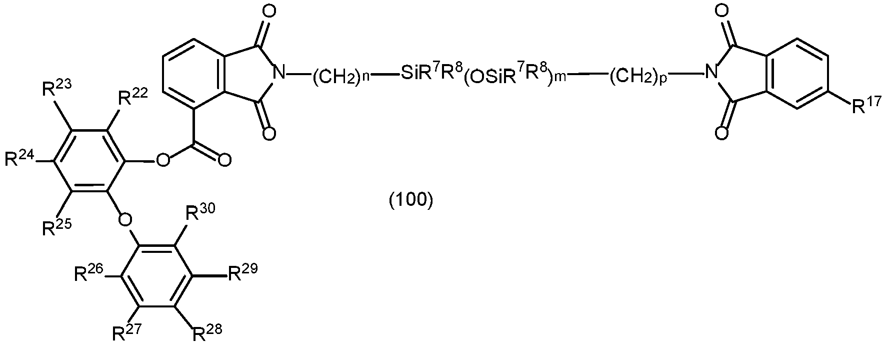 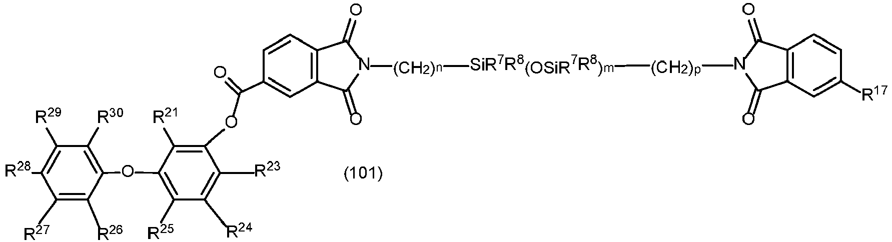 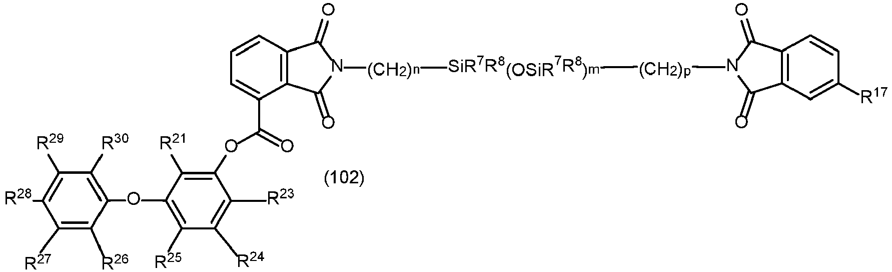 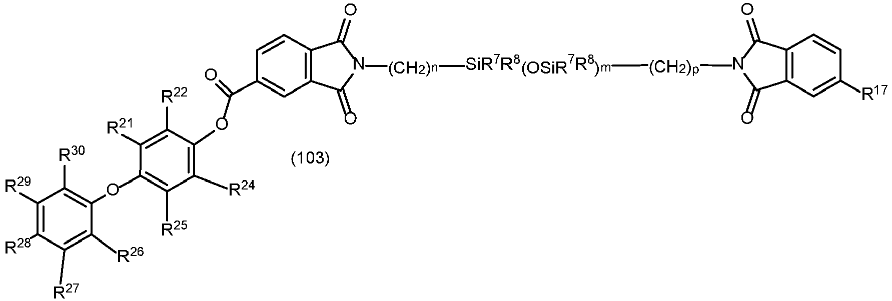 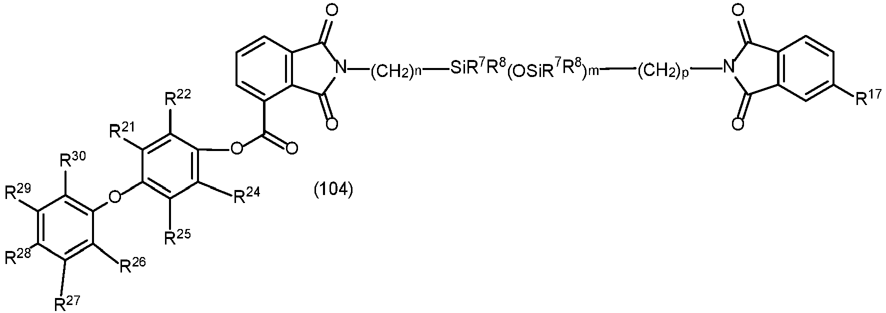 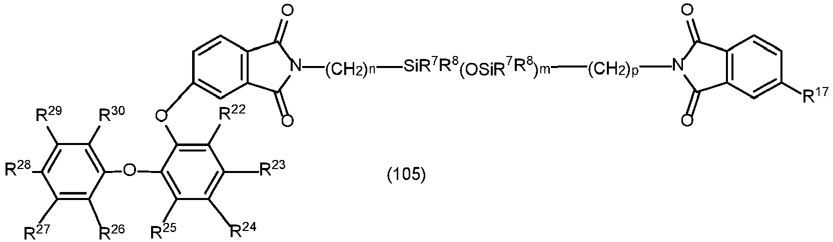 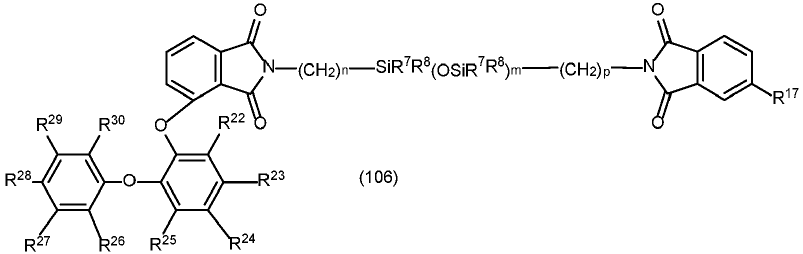 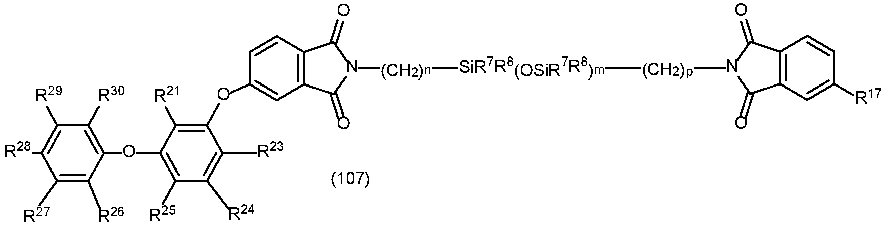 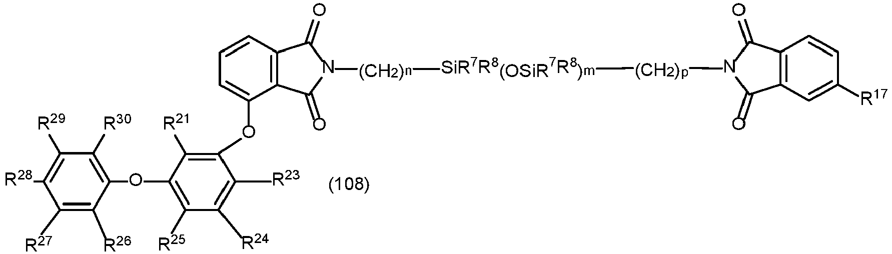 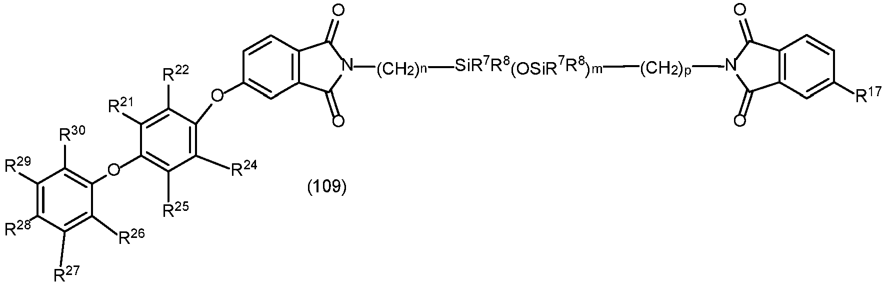 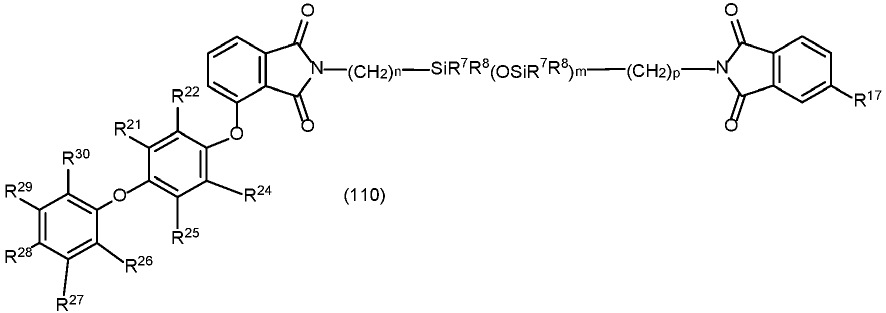 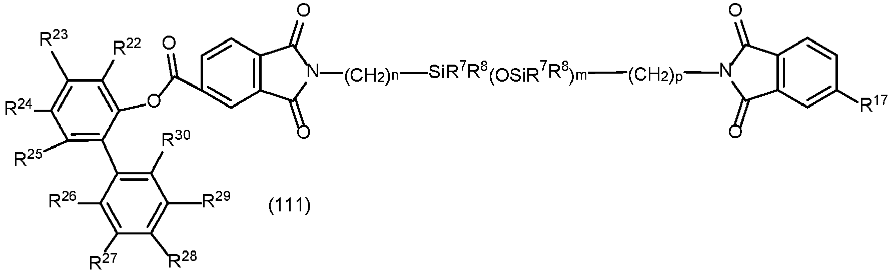 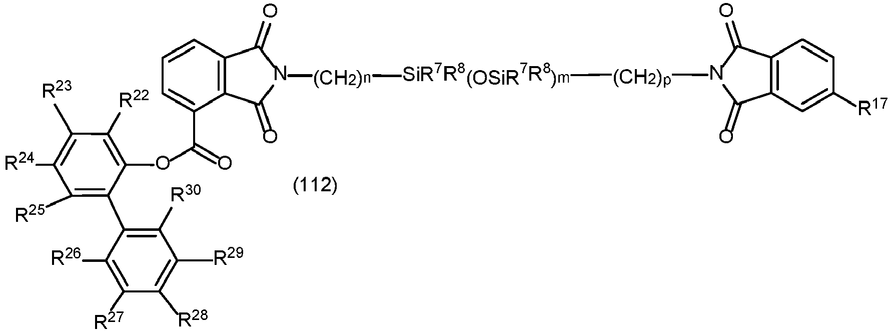 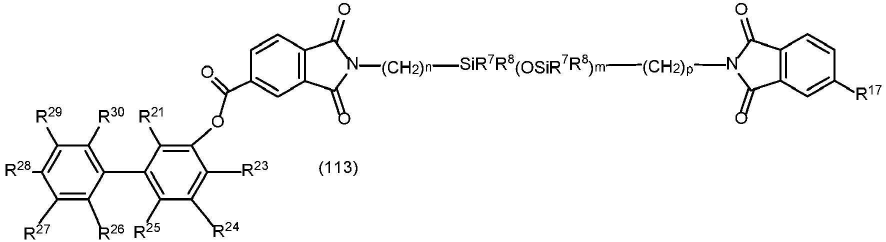 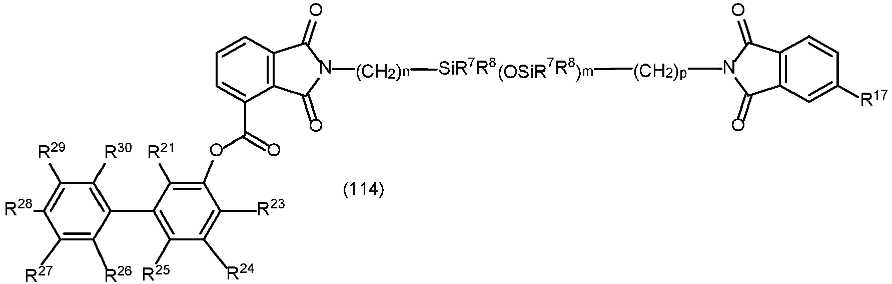 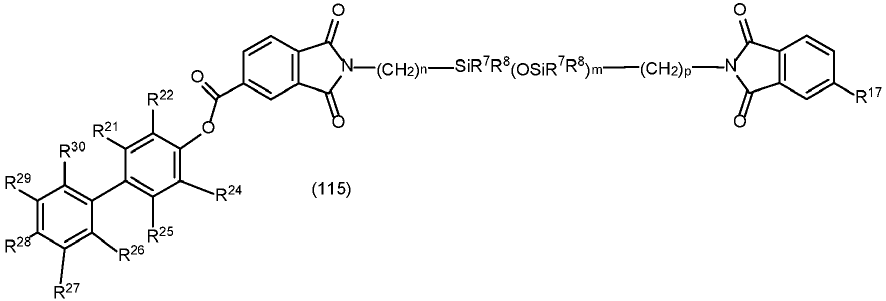 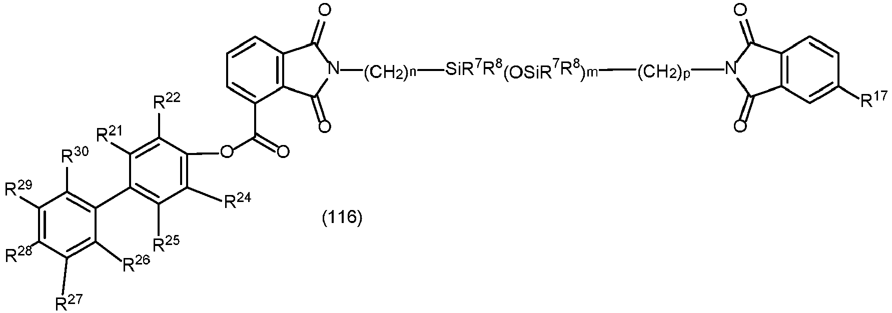 worin
n für eine ganze Zahl von 1 bis 30 steht,
m für eine ganze Zahl von 1 bis 30 steht,
p für eine ganze Zahl von 1 bis 30 steht,
u für eine ganze Zahl von 1 bis 15 steht,
v für 1 oder 2 steht,
R² und R¹⁷ unabhängig voneinander ausgewählt sind unter Wasserstoff, geradkettigem oder verzweigtem Alkyl, geradkettigem oder verzweigtem Alkoxy, geradkettigem oder verzweigtem Alkanoyl, geradkettigem oder verzweigtem Alkyloxycarbonyl,
unsubstituiertem oder substituiertem Aryl, unsubstituiertem oder substituiertem Aryloxy, unsubstituiertem oder substituiertem Arylalkylenoxy, unsubstituiertem oder substituiertem Arylthio, unsubstituiertem oder substituiertem Arylalkylenthio, unsubstituiertem oder substituiertem Aroyl, unsubstituiertem oder substituiertem Aryloxycarbonyl, unsubstituiertem oder substituiertem Arylcarbonyloxy,
Monoalkylaminocarbonyl, Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Dialkylaminocarbonyl, Diarylaminocarbonyl, Di(arylalkylen)aminocarbonyl, Monoalkanoylamino, Monoaroylamino, Monoalkanoylmonoaroylamino, Dialkanoylamino, Diaroylamino, wobei die Arylreste von Monoarylaminocarbonyl, Mono(arylalkylen)aminocarbonyl, Monoalkylmonoarylaminocarbonyl, Monoalkylmono(arylalkylen)aminocarbonyl, Monoarylmono(arylalkylen)aminocarbonyl, Diarylaminocarbonyl, Di(arylalkylen)aminocarbonyl, Monoaroylamino, Monoalkanoylmonoaroylamino und Diaroylamino jeweils unsubstituiert oder substituiert sind,
R⁷ und R⁸ unabhängig voneinander für geradkettiges oder verzweigtes C₁-C₄-Alkyl stehen,
R¹³ für Wasserstoff oder C₁-C₄-Alkyl steht,
R^{c} für Wasserstoff oder C₁-C₄-Alkyl steht,
R²¹, R²², R²³, R²⁴, R²⁵, R²⁶, R²⁷, R²⁸, R²⁹ und R³⁰, falls vorhanden, unabhängig voneinander ausgewählt sind unter Wasserstoff, geradkettigem oder verzweigtem C₁-C₂₀-Alkyl und geradkettigem oder verzweigtem C₁-C₄-Alkoxy.

9. Schmierstoffzusammensetzung, enthaltend
a1) wenigstens eine Verbindung der allgemeinen Formel (I) als Basisöl,
a2) gegebenenfalls wenigstens ein von a1) verschiedenes weiteres Basisöl,
b) wenigstens ein Additiv, vorzugsweise ausgewählt unter Verdickern, Korrosionsinhibitoren, Antioxidantien, Verschleißschutzadditiven, Radikalfängern, UV-Stabilisatoren, anorganischen oder organischen Festschmierstoffen, Pourpoint-Verbesserern, Reibwertveränderern, Additiven zur Verbesserung der Hochdruckeigenschaften, Viskositätsindex-Verbesserern, Haftfähigkeitsverbesserern, weiteren oberflächenaktiven Verbindungen und Mischungen davon.

10. Schmierstoffzusammensetzung nach einem der vorhergehenden Ansprüche, enthaltend wenigstens eine Phthalimidverbindung der allgemeinen Formel (I) in einer Menge von 10,0 Gew.-% bis 99,9 Gew.-%, bevorzugt von 20,0 Gew.-% bis 99,0 Gew.-%, noch bevorzugter von 25,0 Gew.-% bis 95,0 Gew.-% und insbesondere von 30,0 Gew.-% bis 90,0 Gew.-%, jeweils bezogen auf das Gesamtgewicht der Schmierstoffzusammensetzung.

11. Schmierstoffzusammensetzung nach einem der vorhergehenden Ansprüche, enthaltend wenigstens ein Basisöl a2) ausgewählt unter Estern, bevorzugt Dipentaerythritester, Trimellitsäureester, Hemimellitsäureester, Pyromellitsäureester, Estoliden, Pentaerythritester, Dimersäureester, Trimersäureester, Trimethylolpropanester (TMP-Ester), Neopentylglycolester, Dicarbonsäureester; Ethern, bevorzugt Polyphenylether, Diarylether, Triarylether, Polyglycolen, bevorzugt Homo- und/oder Copolymere von Ethylenoxid, Propylenoxid, 1,2-Butylenoxid und/oder Tetrahydrofuran(THF), vorzugsweise gestartet mit Monoalkoholen, Dialkoholen, Trialkoholen und höherwertigen Alkoholen mit 4, 5 oder mehr alkoholischen OH-Gruppen, linearen oder verzweigten Perfluoropolyetherölen (PFPE-Ölen); synthetischen Kohlenwasserstoffen, bevorzugt alkylierte Naphthaline, Polyalphaolefine (PAOs), metallocene Polyalphaolefine (mPAOs); Mineralöle, unbehandelte und chemisch modifizierte pflanzliche Öle, Gruppe III Ölen; Gas to Liquid (GTL) Ölen; Reraffinate und Recyclate, bevorzugt gewonnen von Mineralölen, Gruppe III Ölen, GTL Ölen und/oder PAO, Dimethylsilikonölen; arylierten Silikonölen, Alkylarylsilikonöle, Polyisobutenen und Mischungen davon.

12. Schmierstoffzusammensetzung in Form eines Schmieröls nach einem der Ansprüche 1 bis 11, enthaltend
a) 80 bis 99,9 Gew.-%, bevorzugt 90 bis 99,8 Gew.-%, bezogen auf das Gesamtgewicht der Schmierstoffzusammensetzung wenigstens eine Verbindung der allgemeinen Formel (I),
b) 0,1 bis 20 Gew.-%, bevorzugt 0,2 bis 10 Gew.-%, bezogen auf das Gesamtgewicht der Schmierstoffzusammensetzung, wenigstens ein Additiv.

13. Schmierstoffzusammensetzung in Form eines Schmieröls nach Anspruch 11, enthaltend wenigstens ein Basisöl a2) in einer Menge von bis zu 50 Gew.-%, bevorzugt von bis zu 30 Gew.-%, insbesondere von bis zu 15 Gew.-%, bezogen auf das Gesamtgewicht der Schmierstoffzusammensetzung.

14. Schmierstoffzusammensetzung nach einem der Ansprüche 1 bis 11 in Form eines Schmierfetts, enthaltend
a1) wenigstens eine Verbindung der allgemeinen Formel (I) als Basisöl,
a2) gegebenenfalls wenigstens ein von a1) verschiedenes weiteres Basisöl,
b1) wenigstens einen Verdicker,
b2) gegebenenfalls wenigstens ein weiteres Additiv.

15. Schmierstoffzusammensetzung in Form eines Schmierfetts nach Anspruch 14, enthaltend wenigstens ein Basisöl, ausgewählt unter Basisiölen a1) und a2), in einer Menge von 40,0 bis 95,0 Gew.-%, bevorzugt von 50,0 bis 90 Gew.-%, bezogen auf das Gesamtgewicht der Schmierstoffzusammensetzung.

16. Schmierstoffzusammensetzung in Form eines Schmierfetts nach Anspruch 14 oder 15, enthaltend wenigstens einen Verdicker b1), in einer Menge von 0,2 bis bis 60,0 Gew.-%, bevorzugt von 3,0 bis 55 Gew.-%, insbesondere von 5,0 bis 50 Gew.-%, bezogen auf das Gesamtgewicht der Schmierstoffzusammensetzung.

17. Verwendung von Phthalimidverbindungen der allgemeinen Formel (I), wie in einem der Ansprüche 1 bis 8 definiert, als Basisöl einer Schmierstoffzusammensetzung zum Schmieren von tribologischen Systemen, insbesondere von tribologischen Systemen in Anwendungen, bei denen eine obere Gebrauchstemperatur, von wenigstens 160°C notwendig ist.

18. Verwendung von Phthalimidverbindungen der allgemeinen Formel (I), wie in einem der Ansprüche 1 bis 8 definiert, als Basisöl eines Hochtemperatur-schmierfetts zur Schmierung von Gleitlagern, insbesondere von Ketten, Wälzlagern und/oder zum Antrieb von Produktionsanlagen in der chemischen Industrie, wobei die Gleitlager, die Wälzlagern und/oder die Produktionsanlagen in der Chemischen Industrie, vorzugsweise zumindest zeitweise bei Temperaturen von wenigstens 160°C, betrieben werden.

19. Verwendung von Phthalimidverbindungen der allgemeinen Formel (I), wie in einem der Ansprüche 1 bis 8 definiert, als Basisöl eines Hochtemperaturschmierfetts zur Schmierung von Gleitlagern, insbesondere Gasarmaturen, Aktuatoren, Linearführungen, Regel- und Steuerklappen im Ansaugkrümmer, Kupplungen, Schrauben, Bolzen, Fittings, Ketten für die Lebensmittelindustrie, insbesondere in Brot- und Waffelbackautomaten; von Wälzlagern, insbesondere Wälzlagern für Holzpress- oder Wellpappanlagen und/oder zum Antrieb von Produktionsanlagen in der Chemischen Industrie.

20. Verwendung einer Phthalimidverbindung der Formel (I), wie in einem der Ansprüche 1 bis 8 definiert, als Komponente einer Schmierstoffzusammensetzung zum teilweisen oder vollständigen Ersatz einer fluorhaltigen Schmierstoffkomponente, insbesondere eines linearen oder verzweigten Perfluoropolyetheröls (PFPE-Öls).
